# EUROPEAN PATENT SPECIFICATION

(11) **EP 1 228 209 B1**
(45) Date of publication and mention of the grant of the patent: **14.12.2005**
(21) Application number: 00976787.2
(22) Date of filing: 01.11.2000
(51) Int. Cl.: C12N 15/12, C07K 14/47, C07K 16/18, G01N 33/53

(54) **"INSULIN-RESPONSIVE SEQUENCE DNA BINDING PROTEIN-1" (IRSDBP-1), GENE ENCODING IT AND USES THEREOF**
"INSULINABHÄNGIGES SEQUENZ DNA BINDENDES PROTEIN-1" (IRSDBP-1), DAFÜR KODIERENDES GEN UND IHRE VERWENDUNGEN
PROTEINE 1 DE LIAISON DE SEQUENCE D'ADN REAGISSANT A L'INSULINE ET PROCEDES DE REGULATION DE GENES REAGISSANT A L'INSULINE

(30) Priority: 01.11.1999 US 162687 P
(43) Date of publication of application: 07.08.2002
(73) Proprietor: EMORY UNIVERSITY, Atlanta, Georgia 30322 (US)
(72) Inventor: VILLAFUERTE, Betty, C., Atlanta, GA 30328 (US)
(74) Representative: Heusch, Christian
(86) International application number: PCT/US2000/030096
(87) International publication number: WO 2001/032873

(56) References cited:
- DATABASE EMBL/GENBANK/DDBJ [Online] EBI; 2 July 1998 (1998-07-02) "Homo sapiens BAC clone RP11-556H17 from 2" XP002164028
- VILLAFUERTE, B. C. ET AL.: "Identification of an Insulin-responsive Element in the Rat Insulin-like Growth Factor-binding Protein-3 Gene" J. BIOL. CHEM., vol. 272, no. 2, 21 February 1997 (1997-02-21), pages 5024-5030, XP002164027

## Description

### Reference to Related Application

This application claims priority from U.S. Provisional Application Serial No.: 60/162,687 filed November 1, 1999.

### Acknowledgment of Federal Research Support

This invention was made, at least in part, with funding from the National Institutes of Health (Grants NO. K08 DK022115 and RO1 DK52965). Accordingly, the United States Government has certain rights in this invention.

### Field of the Invention

The present invention relates generally to the identification of an *Insulin-Responsive Sequence DNA Binding Protein (IRSBP)* gene, specifically IRSBP-1, from mammals, and to corresponding proteins and coding sequences thereof. More specifically, the invention relates to a nucleotide sequence encoding the IRSBP-1 protein, to methods and compositions that employ this coding sequence, to the use of therapeutic agents that mimic or facilitate the action of IRSBP-1, and to nucleotide sequences useful in diagnostic methods and treatment of diabetes, obesity, insulin resistance syndrome and other pathological ailments.

### Background

It is estimated that there are more than 6.5 million people in the U.S. diagnosed as having diabetes mellitus (*Harris* (1993) Diabetes Care 16: 642-652). Of those diagnosed, more than 90% have Type II diabetes mellitus. Although β-cell dysfunction is detectable in all diabetic patients whose pancreas exhibits an inability to produce sufficient insulin to maintain glucose levels in the normal range (*Defronzo et al.* (1992) Diabetes Care 15: 318-368; *Taylor et al.* (1994) Diabetes 43: 735-774), the rapid increase in the prevalence of diabetes over the past several decades is apparently more likely to be due to insulin resistance (diminished insulin action on target tissues) (*Defronzo et al.* (1992) Diabetes Care 15: 318-368; *Taylor et al.* (1994) Diabetes 43: 735-774; *Muller-Wieland et al.* (1993) Exp. Clin. Endocrinol. 101: 17-*29; Defronzo & Ferrannini* (1991) Diabetes Care 14: 173-194). Thus, the current epidemic of Type II diabetes in the United States is usually attributed to the aging of the population, the increased prevalence of obesity and sedentary activity, and the enrichment of the population with ethnic groups that may have a genetically predisposed inability of the pancreas to meet the challenge of increased insulin resistance or pancreatic dysfunction (*Colditz et al.* (1995) Ann. Internal Med. 122: 481-486; *Helmrich et al.* (1991) N. Eng. J. Med. 325: 147-152). The high incidence of diabetes represents a significant economic burden, such that approximately $92 billion in health care expenditures in 1992 were diverted to the treatment of diabetes (*Rubin et al.* (1994) J. Clin. Endocrinol. 78: 809A-809F).

Insulin resistance is a key factor in the pathogenesis of Type II diabetes, and can precede abnormal insulin secretion and the onset of clinical diabetes by decades (*Kahn* (1994) Diabetes 43: 1066-1084; *Ericksson et al.* (1989) N. Engl. J. Med. 321: 337-343; *Lillioja et al.* (1988) N. Engl. J. Med. 318: 1217-1225). Resistance to insulin action involves all major target tissues, i.e., skeletal muscle, liver and fat (*Schanlin-Jantti et al.* (1992) Diabetes 41: 598-604; *Haring & Hehnert* (1993) Diabetol. 36: 176-182). Although insulin resistance appears to involve defects in insulin signaling at the post-receptor level, the mechanism of insulin resistance remains poorly understood.

The action of insulin is initiated by binding to cell surface receptors. Autophosphorylation and activation of the intrinsic tyrosine kinase of the insulin receptor β-subunit leads to phosphorylation of several proximal interacting proteins, including insulin receptor substrate-1 (IRS-1), IRS-2, and Shc (*Sun et al.* (1992) J. Biol. Chem. 267: 22662-22672; *Sun et al.* (1991) Nature 352: 73-77; *Kerouz et al.* (1997) J. Clin. Invest. 100: 3164-3172; *Zhou et al.* (1997) J. Biol. Chem. 272: 29829-29833; *Pellicci et al.* (1992) Cell 70: 93-104; *Kovacina & Roth* (1993) Biochem. Biophys. Res. Comm. 192: 1303-1311). IRS-1 interacts with several proteins that contain Src homology 2 (SH2) domains, including the p85 subunits of PI3'-kinase, GRB-2, Syp and Nck (*Myers et al.* (1992) Proc. Natl. Acad. Sci. 89: 10350-10354; *Skolnik et al.* (1991) Cell 65: 83-90; *Backer et al.* (1992); *Lowenstein et al.* (1992) Cell 70: 431-442). Activation of these proteins and the subsequent cascade activation of other intracellular signaling molecules, such as p21^{ras}, raf-1, MAP kinases, and S6 kinase, account for many of insulin's pleiotropic effects (*Medina et al.* (1993) Mol. Cell Biol. 13: 155-162; *Medema & Box* (1993) Crit. Reviews in Oncogenesis 4: 615-661; *Wood et al.* (1992) Cell 68: 1041-1050; *Kyriakis et al.* (1992) Nature 358: 417-*421; Sturgill et al.* (1988) Nature 33.4: 715-718). Each of these cytoplasmic substrates and the activating regulatory loop involved represents a potential linkage to the development of insulin resistance.

The substantial number of signaling circuits involved, including interacting, bypassing and overlapping pathways, the involvement of numerous serine/threonine kinases and phosphatases (*Reik et al.* (1994) Mol. Endocrinol. 8: 490-497; *Reusch et al.* (1995) Endocrinol. 136: 2464-2469; *McGuire et al.* (1991) Diabetes 40: 939-942; *Kusari et al.* (1992) Diabetes 41: 184A), and still uncharacterized links, characterize the complexity of the signaling from the insulin signal at the cell surface receptor to targets within the cell. One approach to the study of insulin interactions with cells is to select a physiological action of insulin and then trace back toward the receptor, an approach known as the target backward approach. This target backward approach has yielded information concerning the mechanism of insulin regulation by focusing on the genetic regulation of the insulin-regulated gene insulin-like growth factor binding protein-3 (IGFBP-3).

Genetic factors also contribute to the development of non-insulin dependent Type II diabetes mellitus (NIDDM). The concordance rate for NIDDM in identical twins approaches 100% (*Barnett et al.,* (1981); *Newman et al.,* (1987)), while the risk to other siblings of a diabetic proband is between 30 and 40% (*Kobberling & Tillil* (1982)). Despite considerable investigative efforts, the genetic heterogeneity of diabetes and the contribution of environmental factors in the development of the phenotype make the identification of specific diabetes-related genes difficult. Methods used in the study of the genetics of NIDDM include association of case control studies, positional searches, parametric linkage, and molecular screening using single-strand conformation polymorphism analysis (*Elbein et al.* (1994); *Cox & Bell* (1989); *Cox et al.* (1988); *Cook et al.* (1993); *Orita et al.* (1989)). In addition, cloned genes, including genes important for both insulin secretion and insulin action, have been examined for sequence abnormalities. Specific mutations associated with insulin resistance and the development of diabetes have been identified for the α- and β-subunits of the insulin receptor (*Sesti et al.* (1991); *van der Vorm & Maassen* (1994); *Benecke et al*. (1992); *Hansen et al.* (1992)), the glucose transporter GLUT4 (*Kusari et al*. (1991); *Choi et al*. (1991)), Rad (Ras-associated with diabetes) (*Reynet & Kahn* (1993)), and the glucokinase gene implicated in MODY (maturity onset diabetes of the young), as well as HNF-1 and HNF-4 (*Vionnet et al.* (1992); *Lesage et al.* (1995); *Bell et al.* (1991); *Kaisaki et al.* (1997); *Moller et al.* (1997)). Such mutations, however, appear to account for less than 5% of patients with Type II diabetes.

A series of adapter proteins or substrates link the receptor tyrosine kinases to gene transcription, and determine the response to insulin in a given cell or tissue. Each of the proteins in the signaling cascade is a potential candidate for an acquired or genetic defect contributing to insulin resistance. Thus, characterization of the insulin-responsive binding proteins (IRBPs) that may bind to gene transcriptional regulatory sequences essential for insulin-regulated expression of target genes, and delineation of the pattern of signal transduction to the IRBPs constitutes an important strategy to identify genes important in mediating insulin resistance.

Insulin-like growth factors I and II (IGF-I and -II) are proteins that have insulin-like metabolic and trophic effects and mediate some of the peripheral actions of growth hormone (*Daughaday & Reeder* (1966)). IGFs also have a role in wound healing by stimulating fibroblasts to produce collagen and induce hematopoiesis through an erythropoietin-like activity. Studies have also shown that certain cancer cells, such as from breast and kidney, produce IGFs. IGF production in cancer cells auto-regulates cell proliferation and the production of a vascular system required for growth of the tumor mass (*LeRoth et al.* (1995) Ann. Intern. Med. 122: 154-159). IGFs have also been implicated in diabetic retinopathy by stimulating endothelial and fibroblast proliferation (*Pfeiffer et al.* (1997) Diabetes 46: S26-S30; *Paques et al.* (1997) Diabetes and Metabolism 23: 125-130; *Flyvbjerg* (1997) Kidney Int. (Suppl.) 60:S12-S19).

The actions of IGFs are modulated by a family of six IGF-binding proteins (IGFBPs) that have different tissue distribution and production sites (*Albiston & Herington* (1992) Endocrinol. 130: 497-502; *Shimasaki et al.* (1991) J. Biol. Chem. 266: 10646-10653). One binding protein, IGFBP-1, has a molecular weight of approximately 30-40 kd in the human and the rat. Most of the circulating plasma IGF-I and IGF-II, however, are associated with IGFBP-3 and an acid-labile subunit thereof that serve as reservoirs for IGFs (*Martin & Baxter* (1986) J. Biol. Chem. 261: 8754-8760). Diabetes mellitus in humans and animal models is associated with decreased levels of serum IGFBP-3 (*Zapf et al.* (1986) J. Clin. Invest. 77: 1768-1775; *Graubert et al.* (1991) Diabetes, 40: 959-965). Hepatic expression of IGFBP-3 is correlated with circulating IGFBP-3 levels in streptozotocin-diabetic and BB/W rats (*Luo & Murphy* (1992) J. Mol. Endocrinol. 8: 155-163; *Binoux et al.* (1984) J. Clin. Endocrinol. Metab. 59: 453-462; *Umezawa et al.* (1991) Brit. J. Nutrition. 66: 105-116). Thus, hepatic expression of IGFBP-3 appears to determine systemic IGFBP-3 levels; and the study of the mechanisms by which insulin stimulates hepatic synthesis of IGFBP-3 is critical for understanding the regulation of systemic IGFBP-3.

Most evidence indicates that IGFBP-3 is inhibitory to IGF action (*Blat et al.* (1989) J. Biol. Chem. 264: 12449-12454; *Valentinis et al.* (1995) Mol. Endocrinol. 9:361-367; *DeMellow & Baxter* (1988) Biochem. Biophys. Res. Commun. 156: 199-204). Furthermore, IGFBP-3 can: (a) mediate the growth inhibitory actions of transforming growth factor-β (TGF-β), retinoic acid, anti-estrogens and fibroblast growth factor (*Oh et al.* (1995), J. Biol. Chem. 270: 13589-13592; *Gucev et al.* (1996) Cancer Res. 56: 1545-1550; *Huynh et al.* (1996) J. Biol. Chem. 271: 1016-1021; *Zadeh & Binoux* (1997) Endocrinol. 138: 3069-3072), (b) mediate the induction of apoptosis by the tumor suppressor gene p53 (*Buckbinder et al.* (1995) Nature 377: 646-649; *Rajah et al.* (1997) J. Biol. Chem. 272: 12181-12188), and (c) travel to the cell nucleus, potentially directly regulating the transcription of critical growth inhibitory genes independent of IGF-I (*Jaques et al.* (1997) Endocrinol. 134: 1767-1770, *Li et al.* (1997) Endocrinol. 138: 1763-1766).

The levels of IGFBP-3 in serum and liver mRNA are highest during puberty and adult life (*Romanus et al.* (1986) Endocrinol. 118: 1743-1758). Unlike other IGFBPs, IGFBP-3 levels increase in the presence of anabolic hormones such as insulin and growth hormone (*Unterman et al.* (1991) Endocrinol. 128: 2693-2701; *Binoux et al.* (1984) J. Clin. Endocrinol. Metab. 59: 453-462). Dependence on growth hormone (GH) has been inferred from the deceased levels of IGFBP-3 in hypopituitary subjects and GH-deficient children and increased levels in acromegalic patients (*Baxer & Martin* (1986) J. Clin. Invest. 78: 1504-1512; *Blum et al.* (1990) J. Clin. Endocrinol. Metab. 70: 1292-1298). Additionally, IGFBP-3 production is inhibited at the level of gene expression by glucocorticoids (*Villafuerte et al.* (1995) Endocrinol. 136: 1928-1933).

The mechanisms by which IGFBP-3 is regulated are complex. IGFBP-3 may undergo post-translational processing to yield various proteolytically cleaved, phosphorylated, and glycosylated products (*Blum et al.* (1989) Endocrinol. 125: 766-*772; Busby et al.* (1989) Endocrinol. 125: 773-777; *Hoeck & Mukku* (1994) J. Cell Biochem. 56: 262-273; *Hossenlopp et al.* (1990) J. Clin. Endocrinol. & Metab. 71: 797-805; *Grimes & Hammond* (1994) Endocrinol. 134: 337-343; *Lassaree et al.* (1994) Growth Reg. 4: 48-55; *Young & Rechler* (1995) Endocrinol. 136: 668-678). These processes have been shown to alter the binding of IGFBP-3 to the acid-labile subunit, cell surfaces and to affect the affinity of IGFBP-3 for IGFs (*Coverly & Baxter* (1995) Endocrinol. 136: 5778-5781). IGFBP-3 can also associate with the cell surface and extracellular matrix; dissociation of cell-associated IGFBP-3 is one mechanism by which IGF-1 promotes release of IGFBP-3 into conditioned medium by fibroblasts and breast cancer cells (*Martin et al.* (1992) Endocrinol. 131: 1703-1710; *Oh et al.* (1992) Endocrinol. 131: 3123-3125).

Insulin increases IGFBP-3 expression by stimulating the rate of gene transcription rather than by stabilization of mRNA transcripts (*Villafuerte et al.* (1996) Mol. Endocrinol. 10: 622-630). This enhancement is mediated through a cis-regulatory insulin-responsive element (IRE) localized to the -1150 to -1124 bp region of the gene encoding IGFBP-3 (*Villafuerte et al.* (1997) J. Biol. Chem. 272: 5024-5030). The IGFBP-3 IRE comprises the nucleotide dyad ACC(A/G)A which has a strong resemblance to the recognition sequence of ETS-related transcription factors, namely AGGAA, which is within the IRE of both the prolactin and somatostatin genes (*Jacob et al.* (1995) J. Biol. Chem. 270: 27773-27779). The 10-bp core sequence of the IGFBP-3 IRE that is most critical for insulin responses (base positions-1148 to -1139) had no significant consensus sequence similarity to previously identified transcription factor binding sites. What was not known, however, was any protein or other factor that would mediate a cellular response to insulin and which directly binds to such insulin-response elements like the IRE of IGFBP-3.

### Summary of the Invention

Briefly described, the present invention relates to a novel protein called Insulin-Responsive Sequence DNA Binding Protein-1 (IRSBP-1) and nucleotide sequences that encode it. IRSBP-1 is capable of binding to nucleic acid regions associated with genes that respond when cells are exposed to insulin or insulin-like factors. IRSBP-1 regulates genes important in mediating the insulin response in humans and animals and in regulating pathological conditions such as diabetes, obesity, insulin-resistant syndrome and cell proliferative disorders.

One aspect of the present invention relates to isolated or non-naturally occurring nucleic acid molecules that encode at least a portion of a human or animal IRSBP-1 protein or a variant thereof.

The present invention provides isolated molecules that can hybridize to nucleic acid sequences of the genome of a human or animal and which encode an IRSBP-1 protein or variants thereof.

The present invention further provides nucleic acids that are fragments or derivatives of the cDNA molecules comprising at least in part a region of the IRSBP-1 coding region and or an untranslated region of the cDNA, wherein the fragments may be used as probes specific for hybridizing and detecting nucleic acid molecules that encode at least in part a region of the IRSBP-1 protein.

Also within the scope of the present invention are recombinant cells, tissues and animals containing non-naturally occurring recombinant nucleic acid molecules encoding IRSBP-1, including expression vectors for the expression of IRSBP-1, antibodies to the IRSBP-1 proteins, assays utilizing the IRSBP-1 polypeptide, and methods relating to all of the foregoing. Also within the scope of the present invention is the development of therapeutic and diagnostic agents that mimic, facilitate or inhibit the action of IRSBP-1, and/or are based on relationships to the structure and action of IRSBP-1.

The invention further provides non-naturally occurring recombinant nucleic acid molecules encoding IRSBP-1 that can be in a cell or an organism. The recombinant nucleic acid may comprise IRSBP-1-related sequences, functional derivatives thereof, and a vector or a promoter effective to initiate transcription in a host cell. The recombinant nucleic acid molecule can alternatively contain transcription regulatory sequences functional in a particular cell, a sequence complementary to a mRNA sequence encoding an IRSBP-1 polypeptide and transcriptional control sequences functional in that cell.

The present invention still further provides oligopeptides having amino acid sequences derived from the amino acid sequence of a human or animal IRSBP-1 protein that may be used to induce the formation of polyclonal or monoclonal antibodies that specifically bind to at least one region of the IRSBP-1 protein from human or animal. The antibodies may be used for, but are not limited to, the detection and assay of IRSBP-1 in biological samples, or the purification of the IRSBP-1 protein. Diagnostic kits for the detection of IRSBP-1 in biological samples are also within the scope of the present invention.

The invention also provides a recombinant cell or tissue containing non-naturally occurring recombinant nucleic acid molecules coding for an IRSBP-1 polypeptide or a portion thereof. In such cells, the IRSBP-1 coding sequence may be expressed under the control of its genomic regulatory elements, or may be under the control of exogenous regulatory elements including an exogenous promoter. The present invention further provides for the production of animals that have modified nucleic acids encoding at least a portion of the IRSBP-1 protein, or have the *IRSBP-1* gene inactivated. The present invention further provides for methods of gene therapy and pharmaceutical compositions including antisense and sense nucleic acids that will modulate, in the human or animal, the activity of the *IRSBP-1* gene or the IRSBP-1 protein encoded therein.

The invention features methods for identifying mammalian cells containing an IRSBP-1 polypeptide, or a related sequence. Such methods comprise identifying the IRSBP-1 polypeptide in mammalian cells using techniques that are routine and standard in the art, for example, PCR amplification, and Northern, Western, Southern and Southwestern blotting using oligonucleotides and derivatives thereof, or antibodies specific to the IRSBP-1 protein.

The present invention also relates to methods of detecting proliferating cells, and the cells of humans or animals having diabetic disorders. The present invention further relates to methods of activating or inhibiting the expression of the gene in humans or animals that encode an IRSBP-1 protein, wherein the proliferation of cells may be modified. The present invention further contemplates that modulation of the activity of the IRSBP-1 protein or the expression thereof may be used to relieve the symptomatic effects of diabetes, particularly type II diabetes.

Additional objects and aspects of the present invention will become more apparent upon review of the detailed description set forth below when taken in conjunction with the accompanying figures, which are briefly described as follows.

### Brief Description of the Figures

Fig. 1 shows the nucleotide sequence of the rat clone 52 cDNA (SEQ ID NO: 2) isolated by the yeast one-hybrid screening procedure with the translated protein sequence (SEQ ID NO: 3) therefrom depicted in Fig. 2.
Fig. 2 shows the protein sequence of the rat clone 52 (SEQ ID NO: 3) translated from the cDNA nucleic acid sequence (SEQ ID NO: 2) depicted in Fig. 1.
Fig. 3 shows the nucleotide sequence of a nucleic acid probe (SEQ ID NO: 4) derived from the rat clone 52 cDNA (SEQ ID NO: 2) depicted in Fig. 1 that was subcloned into a transcription plasmid vector, transcribed and used as a riboprobe.
Figs. 4A-4B show the nucleotide sequence of the rat cDNA (SEQ ID NO: 5) encoding a rat IRSBP-1 protein having the translated protein sequence (SEQ ID NO: 11 as depicted in Fig. 8), wherein Fig. 4A is the rat IRSBP-1 coding region (SEQ ID NO: 9) with the corresponding 3-letter amino acid designation listed below its respective nucleotide triplet, and Fig. 4B is the untranslated region that is 3' of the coding region in Fig. 4A.
Figs. 5A-5B show the nucleotide sequence of a truncated rat cDNA (SEQ ID NO: 6) wherein Fig. 5A is a partial region of a rat IRSBP-1 coding region with the corresponding 3-letter amino acid designation listed below its respective nucleotide triplet, and Fig. 5B is the untranslated region that is 3' of the coding region in Fig. 5A.
Figs. 6A-6D show the nucleotide sequence of the human cDNA (SEQ ID NO: 7) encoding a human IRSBP-1 protein having the translated protein sequence (SEQ ID NO: 12) (as shown in Fig. 9) wherein Fig. 6A shows a region 5' to the coding region, Fig. 6B is a human IRSBP-1 coding region (SEQ ID NO: 9), with the corresponding 3-letter amino acid designation listed below each respective nucleotide triplet, Fig. 6C is the untranslated region that is 3' of the coding region shown in Fig. 6B, and Fig. 6D shows the exons (SEQ ID NOS: 16-41) that comprise SEQ ID NO: 7.
Figs. 7A-7B show the nucleotide sequence of a variant human cDNA (SEQ ID NO: 10), wherein Fig. 7A is a partial region of a human variant IRSBP-1 coding region with the corresponding 3-letter amino acid designation listed below its respective nucleotide triplet, and Fig. 7B is the untranslated region that is 3' of the coding region in Fig. 7A.
Fig. 8 shows the amino acid sequence (SEQ ID NO: 11) of the rat IRSBP-1 protein translated from the coding region of the rat IRSBP-1 cDNA, the sequence (SEQ ID NO: 8) of which is depicted in Fig. 4A.
Fig. 9 shows the amino acid sequence (SEQ ID NO: 12) of the human IRSBP-1 protein translated from the coding region of the human IRSBP-1 cDNA, the sequence (SEQ ID NO: 9) of which is depicted in Fig. 6B.

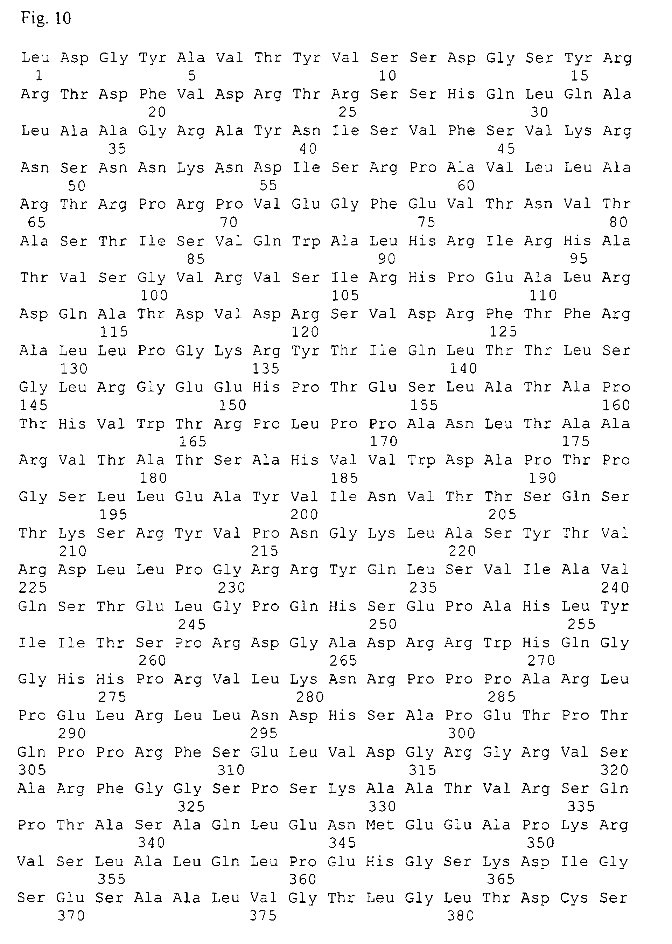

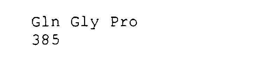

Fig. 10 shows the amino acid sequence (SEQ ID NO: 13) of the variant human IRSBP-1 protein translated from the coding region of the human IRSBP-1 cDNA, the sequence (SEQ ID NO: 10) of which is depicted in Fig. 7A.
Figs. 11A-11B show the nucleotide sequence of the truncated rat cDNA (SEQ ID NO: 14), wherein Fig. 11A is a partial region of the rat IRSBP-1 coding region with the corresponding 3-letter amino acid designation listed below its respective nucleotide triplet, and Fig. 11B is the untranslated region that is 3' of the coding region in Fig. 11A.
Fig. 12 illustrates gel mobility shift of the -1150/-1117 bp IRE fragment of IGFBP-3 (SEQ ID NO: 1) by polypeptides derived from cDNA clones isolated using the yeast one-hybrid system.
Fig. 13 illustrates gel mobility shift analysis of the -1150/-1117 IRE fragment of IGFBP-3 (SEQ ID NO: 1) by cDNAs expressed as thioredoxin fusion proteins.
Fig. 14 illustrates a competition-binding assay of the polypeptide encoded by clone 52 binding to the -1150/-1117 bp IRE fragment of IGFBP-3 (SEQ ID NO: 1).
Fig. 15 illustrates IREs derived from other genes competing with the IRE of IGFBP-3 (SEQ ID NO: 1) for binding to the protein product of clone 52.
Fig. 16 illustrates IGFBP-1 IRE reporter activity in CHO cells transfected with a vector containing HBP1 encoding sequences, a rat IRSBP-1 encoding sequence (SEQ ID NO: 2), or both IRSBP-1 and HBP1.
Fig. 17 illustrates the effect of insulin on IGFBP-3 IRE-regulated reporter gene activity in response to the addition of insulin to cultured cells containing either expression vector or expression vector with clone 52 (SEQ ID NO: 14).
Fig. 18A illustrates the effect of insulin on the expression of the *IRSBP-1* gene, and Fig 18B illustrates the effect of an IRSBP-1 expressing clone (SEQ ID NO: 14) on insulin regulation of glucose uptake in myoblasts.
Fig. 19 illustrates the effect of IRSBP-1 (SEQ ID NO: 14) on the glycogen content of cultured cells.
Fig. 20 illustrates the tissue distribution of clone 52 mRNA.
Fig. 21 illustrates ribonuclease protection assays of the expression of IRSBP-1 in tissues that utilize glucose as a main energy source.
Fig. 22 illustrates the detection of IRSBP-1 mRNA within the hypothalamic portion of the brain by *in-situ* hybridization.
Fig. 23 illustrates the detection of IRSBP-1 mRNA in the lateral hypothalamus by *in-situ* hybridization.
Fig. 24 shows a comparison of IRSBP-1 mRNA in the lateral hypothalamic area of obese and lean rats by *in-situ* hybridization.
Fig. 25 illustrates IRSBP-1 expression in the solitary tract nuclei of lean and obese rats.
Fig. 26 illustrates an *in-situ* hybridization analysis of a rat brain section showing that IRSBP-1-specific mRNA is expressed in the pyramidal tract and decussations of the pyramidal tract in obese Zucker rats.
Fig. 27 illustrates an *in-situ* hybridization analysis showing the expression level of IRSBP-1 in the pyramidal area of the brain of a lean Zucker rat.
Fig. 28 illustrates IRSBP-1 expression in the olfactory bulb.
Fig. 29 illustrates IRSBP-1 expression in the amygdala.
Fig. 30 illustrates immunohistochemical staining with an anti-IRSBP-1 antibody, or with pre-immune serum, of a section of rat pancreas.
Fig. 31 illustrates immunohistochemical staining of rat kidney mesangial cells, using anti-IRSBP-1 antibody (right) or pre-immune serum (left).
Fig. 32 illustrates immunohistochemical staining of the endothelial lining of blood vessels of the kidney, using anti-IRSBP-1 antibody (right) or pre-immune serum (left).
Fig. 33 illustrates immunohistochemical staining of the rat brain, using anti-IRSBP-1 antibody.
Fig. 34 illustrates a Western blot analysis using anti-rat IRSBP-1 peptide polyclonal antibody and cell extracts from human vascular endothelial cells treated with and without insulin for 6 hours.

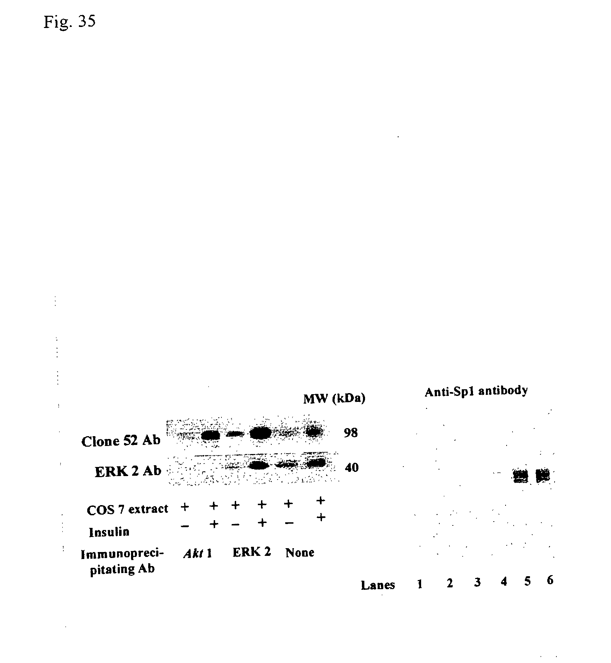

Fig. 35 illustrates a Western blot analysis showing IRSBP-1 in COS 7 cell extracts and co-immunoprecipitation of IRSBP-1 with both anti-Akt 1 (lanes 1, 2) and anti-Erk2 (lanes 3, 4) that is increased with the addition of insulin.
Fig. 36 illustrates the position of clone 52 in the compartment of the cell and the insulin signal transduction cascade.
Fig. 37 illustrates differential phosphorylation of IRSBP-1 in normal and streptozotocin-induced diabetic rats, and in Zucker lean and obese rats. Upper panel: western blot; lower panel: Erk2-phosphorylation of IRSBP-1.
Fig. 38A illustrates the effect of administered IRSBP-1 antisense (AS) or sense (S) oligonucleotides on the body weight of rats. Fig. 38B illustrates the loss in body weight of females rats receiving sense IRSBP-1 DNA.
Fig. 39 illustrates phase contrast micrographs of L6 cells stably transfected with control vector alone (left) or with vector containing the IRSBP-1 nucleic acid sequence (right) and grown in soft agar for 4 weeks.
Fig. 40 illustrates analysis by flow cytometry of the cell cycle compartments of L6 cells with (right panel) or without (left panel) over-expression of the IRSBP-1 nucleic acid sequence.

### Detailed Description of the Preferred Embodiments

Reference now will be made in detail to the presently preferred embodiments of the invention, one or more examples of which are illustrated in the accompanying drawings.

This description uses gene nomenclature accepted by the Cucurbit Genetics Cooperative as it appears in the *Cucurbit Genetics Cooperative Report* 18:85 (1995). Using this gene nomenclature, genes are symbolized by italicized Roman letters. If a mutant gene is recessive to the normal type, then the symbol and name of the mutant gene appear in italicized lower case letters.

For convenience, certain terms employed in the specification, examples, and appended claims are collected here.

The term "animal" is used herein to include all vertebrate animals, including humans. It also includes an individual animal in all stages of development, including embryonic and fetal stages. A "transgenic animal" is any animal containing one or more cells bearing genetic information altered or received, directly or indirectly, by deliberate genetic manipulation at a subcellular level, such as by targeted recombination or microinjection or by infection with recombinant virus. The term "transgenic animal" is not intended to encompass classical cross-breeding or in vitro fertilization, but rather is meant to encompass animals in which one or more cells are altered by, or receive, a recombinant nucleic acid molecule. This recombinant nucleic acid molecule may be specifically targeted to a defined genetic locus, may be randomly integrated within a chromosome, or it may be extrachromosomally replicating nucleic acid. The term "germ cell line transgenic animal" refers to a transgenic animal in which the genetic alteration or genetic information was introduced into a germ line cell, thereby conferring the ability to transfer the genetic information to offspring. If such offspring in fact possess some or all of that alteration or genetic information, they are transgenic animals as well.

The term "mammalian" as used herein refers to any species, subspecies or race of organism of the taxonomic class *mammalia,* such as, but not limited to, such organisms as mice, rats, rabbits, sheep, cattle, and primates, including humans.

As used herein, the term "IRSBP-1" refers to an Insulin-Responsive Sequence Binding Protein-1 capable of binding to at least one insulin responsive element associated with a gene or genes, and by so doing may regulate the expression of an insulin-responsive gene. The term "IRSBP-1" is also intended to apply to proteins, peptides or polypeptides capable of binding to at least one insulin-responsive element of any organism, including fungi or animals.

The term "nucleic acid" as used herein refers to any natural and synthetic linear and sequential arrays of nucleotides and nucleosides, for example cDNA, genomic DNA, mRNA, RNA, oligonucleotides, oligonucleosides and derivatives thereof. For ease of discussion, such nucleic acids may be collectively referred to herein as "constructs," "plasmids," or "vectors." Representative examples of the nucleic acids of the present invention include bacterial plasmid vectors including expression, cloning, cosmid and transformation vectors such as, but not limited to, pBR322, animal viral vectors such as, but not limited to, modified adenovirus, influenza virus, polio virus, pox virus, retrovirus, and the like, vectors derived from bacteriophage nucleic acid, and synthetic oligonucleotides like chemically synthesized DNA or RNA. The term "nucleic acid" further includes modified or derivatised nucleotides and nucleosides such as, but not limited to, halogenated nucleotides such as, but not only, 5-bromouracil, and derivatised nucleotides such as biotin-labeled nucleotides.

The term "isolated nucleic acid" as used herein refers to a nucleic acid with a structure (a) not identical to that of any naturally occurring nucleic acid or (b) not identical to that of any fragment of a naturally occurring genomic nucleic acid spanning more than three separate genes, and includes DNA, RNA, or derivatives or variants thereof. The term covers, for example, (a) a DNA which has the sequence of part of a naturally occurring genomic molecule but is not flanked by at least one of the coding sequences that flank that part of the molecule in the genome of the species in which it naturally occurs; (b) a nucleic acid incorporated into a vector or into the genomic nucleic acid of a prokaryote or eukaryote in a manner such that the resulting molecule is not identical to any vector or naturally occurring genomic DNA; (c) a separate molecule such as a cDNA, a genomic fragment, a fragment produced by polymerase chain reaction (PCR), ligase chain reaction (LCR) or chemical synthesis, or a restriction fragment; (d) a recombinant nucleotide sequence that is part of a hybrid gene, i.e., a gene encoding a fusion protein, and (e) a recombinant nucleotide sequence that is part of a hybrid sequence that is not naturally occurring. Isolated nucleic acid molecules of the present invention can include, for example, natural allelic variants as well as nucleic acid molecules modified by nucleotide deletions, insertions, inversions, or substitutions such that the resulting nucleic acid molecule still essentially encodes an IRSBP-1 protein or a variant thereof of the present invention.

By the use of the term "enriched" in reference to nucleic acid it is meant that the specific DNA or RNA sequence constitutes a significantly higher fraction of the total DNA or RNA present in the cells or solution of interest than in normal or diseased cells or in the cells from which the sequence was taken. Enriched does not imply that there are no other DNA or RNA sequences present, just that the relative amount of the sequence of interest has been significantly increased. The other DNA may, for example, be derived from a yeast or bacterial genome, or a cloning vector, such as a plasmid or a viral vector. The term significant as used herein is used to indicate that the level of increase is useful to the person making such an increase.

It is advantageous for some purposes that a nucleotide sequence is in purified form. The term "purified" in reference to nucleic acid represents that the sequence has increased purity relative to the natural environment.

As used herein the terms "polypeptide" and "protein" refer to a polymer of amino acids of three or more amino acids in a serial array, linked through peptide bonds. The term "polypeptide" includes proteins, protein fragments, protein analogues, oligopeptides and the like. The term "polypeptides" contemplates polypeptides as defined above that are encoded by nucleic acids, produced through recombinant technology, isolated from an appropriate source such as a mammal, or are synthesized. The term "polypeptides" further contemplates polypeptides as defined above that include chemically modified amino acids or amino acids covalently or noncovalently linked to labeling ligands.

The term "fragment" as used herein to refer to a nucleic acid (e.g., cDNA) refers to an isolated portion of the subject nucleic acid constructed artificially (e.g., by chemical synthesis) or by cleaving a natural product into multiple pieces, using restriction endonucleases or mechanical shearing, or a portion of a nucleic acid synthesized by PCR, DNA polymerase or any other polymerizing technique well known in the art, or expressed in a host cell by recombinant nucleic acid technology well known to one of skill in the art. The term "fragment" as used herein may also refer to an isolated portion of a polypeptide, wherein the portion of the polypeptide is cleaved from a naturally occurring polypeptide by proteolytic cleavage by at least one protease, or is a portion of the naturally occurring polypeptide synthesized by chemical methods well known to one of skill in the art.

The term "modulates" as used herein refers to the ability of a compound to alter the function of an IRE binding protein. A modulator preferably increases the binding or activating potential of an IRSBP-1. A modulator can alternatively decrease the binding or activating potential of IRSBP-1 polypeptide or fragments thereof.

The term "gene" or "genes" as used herein refers to nucleic acid sequences (including both RNA or DNA) that encode genetic information for the synthesis of a whole RNA, a whole protein, or any portion of such whole RNA or whole protein. Genes that are not naturally part of a particular organism's genome are referred to as "foreign genes", "heterologous genes" or "exogenous genes" and genes that are naturally a part of a particular organism's genome are referred to as "endogenous genes". The term "gene product" refers to RNAs or proteins that are encoded by the gene. "Foreign gene products" are RNA or proteins encoded by "foreign genes" and "endogenous gene products" are RNA or proteins encoded by endogenous genes. "Heterologous gene products" are RNAs or proteins encoded by "foreign, heterologous or exogenous genes" and which, therefore, are not naturally expressed in the cell.

The term "expressed" or "expression" as used herein refers to the transcription from a gene to give an RNA nucleic acid molecule at least complementary in part to a region of one of the two nucleic acid strands of the gene. The term "expressed" or "expression" as used herein also refers to the translation from said RNA nucleic acid molecule to give a protein or polypeptide or a portion thereof.

As used herein, the term "locus" or "loci" refers to the site of a gene on a chromosome. Pairs of genes control hereditary traits, each in the same position on a pair of chromosomes. These gene pairs, or alleles, may both be dominant or both be recessive in expression of that trait. In either case, the individual is said to be homozygous for the trait controlled by that gene pair. If the gene pair (alleles) consists of one dominant and one recessive trait, the individual is heterozygous for the trait controlled by the gene pair. Natural variation in genes or nucleic acid molecules caused by, for example, recombination events or resulting from mutation, gives rise to allelic variants with similar, but not identical, nucleotide sequences. Such allelic variants typically encode proteins with similar activity to that of the protein encoded by the gene to which they are compared, because natural selection typically selects against variations that alter function. Allelic variants can also comprise alterations in the untranslated regions of the gene as, for example, in the 3' or 5' untranslated regions or can involve alternate splicing of a nascent transcript, resulting in alternative exons being positioned adjacently.

As used herein "genomic sequence" refers to the total DNA in the genome of an organism, and includes non-coding regions like introns.

The term "transcription regulatory sequences" as used herein refers to nucleotide sequences that are associated with a gene nucleic acid sequence and which regulate the transcriptional expression of the gene. The "transcription regulatory sequences" may be isolated and incorporated into a vector nucleic acid to enable regulated transcription in appropriate cells of portions of the vector DNA. The "transcription regulatory sequence" may precede, but are not limited to, the region of a nucleic acid sequence that is in the region 5' of the end of a protein coding sequence that may be transcribed into mRNA. Transcriptional regulatory sequences may also be located within a protein coding region, in regions of a gene that are identified as "intron" regions, or may be in regions of nucleic acid sequence that are in the region of nucleic acid.

The term "coding region" as used herein refers to a continuous linear arrangement of nucleotides which may be translated into a protein. A full length coding region is translated into a full length protein; that is, a complete protein as would be translated in its natural state absent any post-translational modifications. A full length coding region may also include any leader protein sequence or any other region of the protein that may be excised naturally from the translated protein.

The term "complementary" as used herein refers to two nucleic acid molecules that can form specific interactions with one another. In the specific interactions, an adenine base within one strand of a nucleic acid can form two hydrogen bonds with thymine within a second nucleic acid strand when the two nucleic acid strands are in opposing polarities. Also in the specific interactions, a guanine base within one strand of a nucleic acid can form three hydrogen bonds with cytosine within a second nucleic acid strand when the two nucleic acid strands are in opposing polarities. Complementary nucleic acids as referred to herein, may further comprise modified bases wherein a modified adenine may form hydrogen bonds with a thymine or modified thymine, and a modified cytosine may form hydrogen bonds with a guanine or a modified guanine.

The term "probe" as used herein, when referring to a nucleic acid, refers to a nucleotide sequence that can be used to hybridize with and thereby identify the presence of a complementary sequence, or a complementary sequence differing from the probe sequence but not to a degree that prevents hybridization under the hybridization stringency conditions used. The probe may be modified with labels such as, but not only, radioactive groups, biotin, or any other label that is well known in the art.

The term "capable of hybridizing under stringent conditions" as used herein refers to annealing a first nucleic acid to a second nucleic acid under stringent conditions as defined below. Stringent hybridization conditions typically permit the hybridization of nucleic acid molecules having at least 70% nucleic acid sequence identity with the nucleic acid molecule being used as a probe in the hybridization reaction. For example, the first nucleic acid may be a test sample or probe, and the second nucleic acid may be the sense or antisense strand of a IRSBP-1 gene or a fragment thereof. Hybridization of the first and second nucleic acids may be conducted under stringent conditions, e.g., high temperature and/or low salt content that tend to disfavor hybridization of dissimilar nucleotide sequences. Alternatively, hybridization of the first and second nucleic acid may be conducted under reduced stringency conditions, e.g. low temperature and/or high salt content that tend to favor hybridization of dissimilar nucleotide sequences. Low stringency hybridization conditions may be followed by high stringency conditions to increase the selectivity of the binding of the first and second nucleic acids. The hybridization conditions may further include reagents such as, but not limited to, dimethyl sulfoxide (DMSO) or formamide to disfavor still further the hybridization of dissimilar nucleotide sequences. A suitable hybridization protocol may, for example, involve hybridization in 6X SSC (wherein 1X SSC comprises 0.015 M sodium citrate and 0.15 M sodium chloride), at 65° Celsius in aqueous solution, followed by washing with 1X SSC at 65° Celsius. Formulae to calculate appropriate hybridization and wash conditions to achieve hybridization permitting 30% or less mismatch between two nucleic acid molecules are disclosed, for example, in *Meinkoth et al.* (1984) Anal. Biochem. 138: 267-284. Protocols for hybridization techniques are well known to those of skill in the art and standard molecular biology manuals may be consulted to select a suitable hybridization protocol without undue experimentation. See, for example, *Sambrook et al* (1989) Molecular Cloning: A Laboratory Manual 2nd ed. Cold Spring Harbor Press.

The term "unique" nucleic acid region as used herein refers to a sequence present in a nucleic acid that is not present in any other nucleic acid sequence. The term "conserved nucleic acid region" as referred to herein is a nucleotide sequence present in two or more nucleic acid sequences, to which a particular nucleic acid sequence can hybridize under low, medium or high stringency conditions. The greater the degree of conservation between the conserved regions of two or more nucleic acid sequences, the higher the hybridization stringency that will allow hybridization between the conserved region and a particular nucleic acid sequence.

The terms "percent sequence identity" or "percent sequence similarity" as used herein refer to the degree of sequence identity between two nucleic acid sequences or two amino acid sequences as determined using the algorithm of *Karlin & Attschul* (1990) Proc. Natl. Acad. Sci. 87: 2264-2268, modified as in *Karlin & Attschul* (1993) Proc. Natl. Acad. Sci. 90: 5873-5877. Such an algorithm is incorporated into the NBLAST and XBLAST programs of *Attschul et al.* (1990) T. Mol. Biol. Q15: 403-410. BLAST nucleotide searches are performed with the NBLAST program, score = 100, wordlength = 12, to obtain nucleotide sequences homologous to a nucleic acid molecule of the invention. BLAST protein searches are performed with the XBLAST program, score = 50, wordlength = 3, to obtain amino acid sequences homologous to a reference polypeptide. To obtain gapped alignments for comparison purposes, Gapped BLAST is utilized as described in *Attschul et al.* (1997) Nuc. Acids Res. 25: 3389-3402. When utilizing BLAST and Gapped BLAST programs, the default parameters of the respective programs (e.g. XBLAST and NBLAST) are used. See http://www.ncbi.nlm.nih.gov.

Other algorithms, programs and default settings may also be suitable such as, but not only, the GCG-Sequence Analysis Package of the U.K. Human Genome Mapping Project Resource Centre that includes programs for nucleotide or amino acid sequence comparisons.

The term "sense strand" as used herein refers to a single stranded DNA molecule from a genomic DNA that may be transcribed into RNA and translated into the natural polypeptide product of the gene. The term "antisense strand" is used herein to mean the single strand DNA molecule of a genomic DNA that is complementary with the sense strand of the gene.

The term "antisense DNA" as used herein refers to a gene sequence DNA that has a nucleotide sequence complementary to the "sense strand" of a gene when read in reverse orientation, i.e., DNA read into RNA in a 3' to 5' direction rather than in the 5' to 3' direction. The term "antisense RNA" is used to mean an RNA nucleotide sequence (for example that encoded by an antisense DNA or synthesized complementary with the antisense DNA). Antisense RNA is capable of hybridizing under stringent conditions with an antisense DNA. The antisense RNA of the invention is useful for regulating expression of a "target gene" either at the transcriptional or translational level. For example, transcription of the subject nucleic acids may produce antisense transcripts that are capable of inhibiting transcription by inhibiting initiation of transcription or by competing for limiting transcription factors; or, the antisense transcripts may inhibit transport of the "target RNA", or, the antisense transcripts may inhibit translation of "target RNA".

The term "antisense therapy" as used herein refers to the administration or in situ generation of oligonucleotide probes or their derivatives that specifically hybridize (e.g. bind) under cellular conditions, with the cellular mRNA and/or genomic DNA encoding a IRSBP-1 protein so as to inhibit expression of that protein, e.g. by inhibiting transcription and/or translation. The binding may be by conventional base pair complementation, or, for example, in the case of binding to DNA duplexes, through specific interactions in the major groove of the double helix. In general, "antisense" therapy refers to the range of techniques generally employed in the art, and includes any therapy that relies on specific binding to oligonucleotide sequences.

The term "nucleic acid vector" as used herein refers to a natural or synthetic single or double stranded plasmid or viral nucleic acid molecule that can be transfected or transformed into cells and replicate independently of, or within, the host cell genome. A circular double stranded plasmid can be linearized by treatment with an appropriate restriction enzyme based on the nucleotide sequence of the plasmid vector. A nucleic acid can be inserted into a vector by cutting the vector with restriction enzymes and ligating the pieces together. The nucleic acid molecule can be RNA or DNA.

The term "expression vector" as used herein refers to a nucleic acid vector that may further include at least one regulatory sequence operably linked to the nucleotide sequence coding for the IRSBP-1 protein. Regulatory sequences are well recognized in the art and may be selected to ensure good expression of the linked nucleotide sequence without undue experimentation by those skilled in the art. As used herein, the term "regulatory sequences" includes promoters, enhancers, and other elements that may control expression. Standard molecular biology textbooks such as *Sambrook et al.* eds "Molecular Cloning: A Laboratory Manual" 2nd ed. Cold Spring Harbor Press (1989) may be consulted to design suitable expression vectors, promoters, and other expression control elements. It should be recognized, however, that the choice of a suitable expression vector depends upon multiple factors including the choice of the host cell to be transformed and/or the type of protein to be expressed.

The terms "transformation" and "transfection" as used herein refer to the process of inserting a nucleic acid into a host. Many techniques are well known to those skilled in the art to facilitate transformation or transfection of a nucleic acid into a prokaryotic or eukaryotic organism. These methods involve a variety of techniques, such as treating the cells with high concentrations of salt such as, but not only a calcium or magnesium salt, an electric field, detergent, or liposome mediated transfection, to render the host cell competent for the uptake of the nucleic acid molecules.

The term "recombinant nucleic acid" as used herein refers to combinations of at least two nucleic acid sequences that are not naturally found in a eukaryotic or prokaryotic cell. The nucleic acid sequences may include, but are not limited to nucleic acid vectors, gene expression regulatory elements, origins of replication, sequences that when expressed confer antibiotic resistance, and protein-encoding sequences. The term "recombinant polypeptide" it is meant to include a polypeptide produced by recombinant DNA techniques such that it is distinct from a naturally occurring polypeptide either in its location, purity or structure. Generally, such a recombinant polypeptide will be present in a cell in an amount different from that normally observed in nature.

The term "recombinant cell" refers to a cell that has a new combination of nucleic acid segments that are not covalently linked to each other in nature. A new combination of nucleic acid segments can be introduced into an organism using a wide array of nucleic acid manipulation techniques available to those skilled in the art. A recombinant cell can be a single eukaryotic cell, or a single prokaryotic cell, or a mammalian cell. The recombinant cell can harbor a vector that is extragenomic. An extragenomic nucleic acid vector does not insert into the cell's genome. A recombinant cell can further harbor a vector or a portion thereof that is intragenomic. The term intragenomic defines a nucleic acid construct incorporated within the recombinant cell's genome.

The term "antibody" as used herein refers to polyclonal and monoclonal antibodies and fragments thereof, and immunologic binding equivalents thereof that are capable of specifically binding to the IRSBP-1 polypeptides and fragments thereof, including epitopes thereof, or to polynucleotide sequences from the IRSBP-1 region, particularly from the IRSBP-1 locus or a portion thereof. The term "antibody" refers to a homogeneous molecular entity, or a mixture such as a serum product made up of a plurality of different molecular entities, and may further comprise any modified or derivatised variant thereof that retains the ability to specifically bind to IRSBP-1-related polypeptides.

Described herein are methods for the production of antibodies capable of specifically recognizing one or more differentially expressed or pathway gene epitopes. Such antibodies may include, but are not limited to polyclonal antibodies, monoclonal antibodies (mAbs), humanized or chimeric antibodies, single chain antibodies, Fab fragments, F(ab').sub.2 fragments, fragments produced by a FAb expression library, anti-idiotypic (anti-Id) antibodies, and epitope-binding fragments of any of the above. Such antibodies may be used, for example, in the detection of a fingerprint, target, or pathway gene in a biological sample, or, alternatively, as a method for the inhibition of abnormal target gene activity. Thus, such antibodies may be utilized as part of body weight disorder treatment methods, and/or may be used as part of diagnostic techniques whereby patients may be tested for abnormal levels of fingerprint, target, or pathway gene proteins, or for the presence of abnormal forms of the such proteins.

For the production of antibodies to a differentially expressed or pathway gene, various host animals may be immunized by injection with a differentially expressed or pathway gene protein, or a portion thereof. Such host animals may include but are not limited to rabbits, mice, and rats, to name but a few. Various adjuvants may be used to increase the immunologic response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and *Corynebacterium parvum.*

Polyclonal antibodies are heterogeneous populations of antibody molecules derived from the sera of animals immunized with an antigen, such as a target gene product, or an antigenic functional derivative thereof. For the production of polyclonal antibodies, host animals such as those described above, may be immunized by injection with differentially expressed or pathway gene product supplemented with adjuvants as also described above.

Monoclonal antibodies, which are homogeneous populations of antibodies to a particular antigen, may be obtained by any technique that provides for the production of antibody molecules by continuous cell lines in culture. These include, but are not limited to the hybridoma technique of *Kohler & Milstein* (1975) Nature 256: 495-497; and U.S. Patent No. 4,376,110), the human B-cell hybridoma technique (*Kosbor et al.* (1983) Immunology Today 4: 72; *Cole et al.* (1983) Proc. Natl. Acad. Sci. 80: 2026-2030), and the EBV-hybridoma technique (*Cole et al.* (1985) Monoclonal Antibodies And Cancer Therapy Alan R. Liss, Inc. pp. 77-96). Briefly, spleen cells are harvested from an immunized mouse and fused with immortalizing cells (i.e., myeloma cells) to yield antibody-producing hybridomas. Hybridomas can be screened immunochemically for production of monoclonal antibodies specifically reactive with the IRSBP-1 protein.

Protocols for producing, isolating and purifying conventional and monoclonal antibodies may be analogous to those described in *Cassone et al.* (1988) J. Med. Microbiol. 27: 233-238; *Hancock & Evan* Production and Characterization of Antibodies against Synthetic Peptides pp23-33 in Immunochemical Protocols ed. *M.M. Manson,* (1992) (Humana Press, Totowa, NJ); *Goding, J. W*., Monoclonal Antibodies: Principles and Practice, 2d ed., (1986) (Academic Press Ltd., London) and *Lam & Mutharia,* "Antigen-Antibody Reactions," pp104-132 in Methods for General and Molecular Bacteriology, ed. P. Gerhardt, (1994) (ASM Press, Washington, DC). Such antibodies may be of any immunoglobulin class including IgG, IgM, IgE, IgA, IgD and any subclass thereof. The hybridoma producing the mAb of this invention may be cultivated *in vitro* or *in vivo.* Production of high titers of mAbs *in vivo* makes this the presently preferred method of production.

In addition, techniques developed for the production of "chimeric antibodies" (*Morrison et al.* (1984) Proc. Natl. Acad. Sci. 81: 6851-6855; *Neuberger et al.* (1984) Nature 312: 604-608; *Takeda et al.* (1985) Nature 314: 452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region.

Alternatively, techniques described for the production of single chain antibodies such as, but not only U.S. Patent No. 4,946,778; *Bird* (1988) Science 242: 423-426; *Huston et al.* (1988) Proc. Natl. Acad. Sci. 85: 5879-5883; and *Ward et al.* (1989) Nature 334: 544-546 can be adapted to produce differentially expressed or pathway gene-single chain antibodies. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (*Huse et al.* (1989) Science 246: 1275-1281) to allow rapid and easy identification of monoclonal Fab fragments with the desired specificity.

Pharmaceutical compositions comprising agents that will modulate the physiological activity of the *IRSBP-1* gene product or the regulation of the expression of the *IRSBP-1* gene can be administered in dosages and by techniques well known to those skilled in the medical or veterinary arts, taking into consideration such factors as the age, sex, weight, species and condition of the particular patient, and the route of administration. The route of administration can be percutaneous, via mucosal administration (e.g., oral, nasal, anal, vaginal) or via a parenteral route (intradermal, intramuscular, subcutaneous, intravenous, or intraperitoneal). Pharmaceutical compositions can be administered alone, or can be co-administered or sequentially administered with other treatments or therapies. Forms of administration may include suspensions, syrups or elixirs, and preparations for parenteral, subcutaneous, intradermal, intramuscular or intravenous administration (e.g., injectable administration) such as sterile suspensions or emulsions. Pharmaceutical compositions may be administered in admixture with a suitable carrier, diluent, or excipient such as sterile water, physiological saline, glucose, or the like. The compositions can contain auxiliary substances such as wetting or emulsifying agents, pH buffering agents, adjuvants, gelling or viscosity enhancing additives, preservatives, flavoring agents, colors, and the like, depending upon the route of administration and the preparation desired. Standard pharmaceutical texts, such as "Remmington's Pharmaceutical Science," 17th edition, 1985 may be consulted to prepare suitable preparations, without undue experimentation. The effective dosage and route of administration are determined by the therapeutic range and nature of the compound, and by known factors, such as the age, weight, and condition of the host, as well as LD₅₀ and other screening procedures that are known and do not require undue experimentation. Dosages can generally range from a few hundred milligrams to a few grams.

As used herein, a "transgenic animal" is any animal, preferably a non-human mammal, in which one or more of the cells of the animal contain heterologous nucleic acid introduced by way of human intervention, such as by transgenic techniques well known in the art. The nucleic acid is introduced into the cell, directly or indirectly by introduction into a precursor of the cell, by way of deliberate genetic manipulation, such as by microinjection or by infection with a recombinant virus. The term genetic manipulation does not include classical cross-breeding, or in vitro fertilization, but rather is directed to the introduction of a recombinant DNA molecule. This molecule may be integrated within a chromosome, or it may be extrachromosomally replicating DNA. In the typical transgenic animals, the transgene causes cells to express a recombinant form of the subject IRSBP-1 protein, e.g. either agonistic or antagonistic forms, or in which the endogenous *IRSBP-1* gene has been disrupted. However, transgenic animals in which the recombinant *IRSBP-1* gene is silent are also contemplated, as for example, the FLP or CRE recombinase dependent constructs described below. The "non-human animals" of the invention include vertebrates such as rodents, non-human primates, sheep, dog, cow, birds, amphibians, reptiles, etc. Preferred non-human animals are selected from the rodent family including rat and mouse, most preferably mouse. The term "chimeric animal" is used herein to refer to animals in which the recombinant gene is found, or in which the recombinant is expressed in some but not all cells of the animal. The term "tissue-specific chimeric animal" indicates that the recombinant *IRSBP-1* gene is present and/or expressed in some tissues but not others.

As used herein, the term "transgene" means a nucleic acid sequence (encoding, e.g., a IRSBP-1 polypeptide) that is partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can include one or more transcriptional regulatory sequences and any other nucleic acid, such as introns, that may be necessary for optimal expression of a selected nucleic acid.

As used herein, an "IRSBP-1 nucleic acid molecule" includes nucleic acid sequences related to a natural IRSBP-1 gene and includes all regions such as regulatory regions that control production of an RNA nucleic acid encoding the IRSBP-1 protein or production of the IRSBP-1 protein encoded by the gene (such as, but not limited to, transcription, translation or post-translation regulatory sequences) as well as the coding region itself, and any introns or non-translated coding regions.

In the present context, an IRSBP-1 variant is an IRSBP-1 polypeptide that differs from an exemplified sequence in that one or more amino acids have been changed, added or deleted. An IRSBP-1 variant retains its useful function, i.e., for example, ability to bind IREs or activate or suppress insulin-regulating genes.

As used herein, the term "epitope" refers to a part of the protein that can specifically bind to an antibody by fitting into the antigen-binding site of the antibody.

The techniques used to isolate and characterize the nucleic acids and proteins of the present invention are well known to those of skill in the art and standard molecular biology and biochemical manuals may be consulted to select suitable protocols without undue experimentation. See, for example, *Sambrook et al* (1989) Molecular Cloning: A Laboratory Manual 2nd ed. Cold Spring Harbor Press.

Abbreviations used in the present specification include the following: aa, amino acid(s); bp, base pair(s); cDNA, DNA complementary to RNA; IGF, insulin-like growth factor; IGFBP, IGF-binding protein; IRE, insulin response element; PEPCK, phosphoenol pryuvate carboxykinase; GAPDH, glyceraldehyde-3-phosphate dehydrogenase enzyme; nt, nucleotide(s); SSC, sodium chloride-sodium citrate; DMSO, dimethyl sulfoxide.

The present invention provides isolated nucleic acids, derivatives and variants thereof that encode human and rat IRSBP-1 proteins, derivatives or variants thereof. The present invention further provides an isolated nucleic acid encoding a fragment of a rat IRSBP-1 protein isolated based on the ability of the expressed protein product thereof to bind to the nucleic acid Insulin Responsive Element (IRE), associated with the rat Insulin-like Growth Factor Binding Protein-3 (IGFBP-3) that has the nucleotide sequence 5'-AATTCAAGGGTATCCAGGAAAGTCTCC-3' (SEQ ID NO: 1). As used herein, IREs are regulatory nucleic acid sequences of insulin-regulated genes that are necessary to enable an insulin-dependent response. The nucleotide sequence of SEQ ID NO: 1 is localized between the -1150 and the -1124 bp positions of the promoter region of the IGFBP-3 encoding gene of the rat, as described in *Villafuerte et al.,* J. Biol. Chem. 272: 5024-5030 (1997).

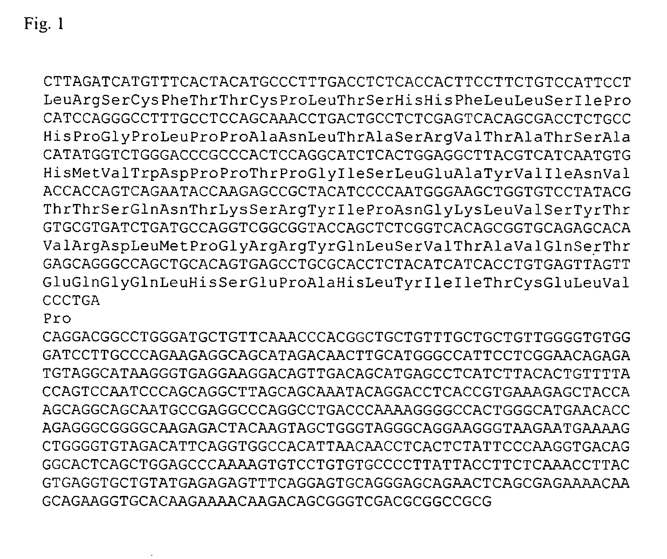

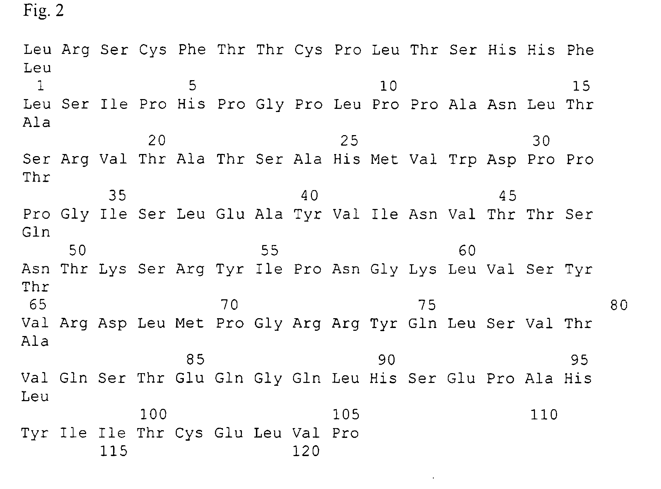

A rat liver cDNA library using the yeast one-hybrid system was screened using concatemerized IREs of rat IGFBP-3, as described by *Wang & Reed* (1993) Nature 364: 121-126, discussed in Example 1 below. The cDNA library screening provided a novel 952-bp cDNA (clone 52) encoding a portion of the Insulin-Responsive Sequence DNA Binding Protein-1 (IRSBP-1) that was identified and sequenced (SEQ ID NO: 2) as illustrated in Fig. 1. The nucleic acid sequence of clone 52 (SEQ ID NO: 2) encodes a polypeptide having the amino acid sequence of SEQ ID NO: 3, as shown in Fig. 2, that binds to the IRE region of the rat IGFBP-3 (SEQ ID NO: 1), as well as to other insulin-responsive genes. A clone 52-thioredoxin (Trx) fusion protein also binds to the IRE of rat IGFBP-3 (SEQ ID NO: 1). The amino acid sequence (SEQ ID NO: 3) deduced from the nucleotide sequence (SEQ ID NO: 2) of clone 52 contains a homeodomain motif typical of transcription factors. Binding by the polypeptide (SEQ ID NO: 3) encoded by clone 52 (SEQ ID NO: 2) to the IRE of IGFBP-3 (SEQ ID NO: 1) could be competed away by IGFBP-3 IRE nucleic acids but not by nucleic acids of sequences unrelated to the IRE. The interaction between the IRSBP-1-related polypeptide (SEQ ID NO: 3) and the IGFBP-3 IRE nucleic acid (SEQ ID NO: 1) was specific.

The IRSBP-1 polypeptide fragment (SEQ ID NO: 3) encoded by clone 52 also interacts with IREs associated with other insulin-responsive genes besides IGFBP-3. The polypeptide interacts with the IREs from insulin-responsive genes encoding IGF-1, IGFBP-1, phosphoenol pyruvate carboxykinase (PEPCK), amylase, and glyceraldehyde-3-phosphate dehydrogenase (GAPDH).

IGFBP-1 is a hepatic acute phase reactant protein that coordinates the level of IGF-1 in response to changes in insulin levels (*Lee et al.* (1993)). Amylase is important for intestinal hydrolysis of carbohydrates. GAPDH catalyzes the conversion of glyceraldehyde-3-phosphate to 1,3-diphosphoglycerate, a rate-limiting step in adipose tissue glycolysis. While not being bound by any theory, the naturally occurring IRSBP-1 protein is likely a transcription factor that coordinates the responses of several genes to insulin. The IRSBP-1 polypeptide (SEQ ID NO: 3) of clone 52 can regulate critical genes in target tissues implicated in insulin resistance and insulin secretion. It is believed to modulate the pleiotropic actions of insulin in the normal metabolism and storage of ingested carbohydrate and other fuels, in the modulation of intermediary metabolism, and in normal cellular growth and differentiation.

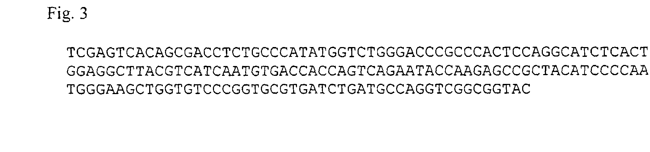

Ribonuclease protection assays (discussed in Example 8) using an antisense RNA probe obtained by transcribing a Kpn1-Xho1 fragment of clone 52 (SEQ ID NO: 4, as shown in Fig. 3) showed that at least one gene, encoding at least one nucleic acid with sequence similarity to a region of the clone 52 cDNA sequence (SEQ ID NO: 2) is expressed in at least liver, kidney, brain, small intestine, muscle, and fat pads.

The abundance of a rat RNA transcript capable of hybridizing to a probe having a nucleic acid sequence of the clone 52 (SEQ ID NO: 4) was increased with the addition of physiological concentrations of insulin (10⁻⁹ M) in cell culture. It was also decreased in the livers of diabetic rats.

The present invention provides for the use of the isolated cDNA clone 52 (SEQ ID NO: 2) as a probe to screen rat and human cDNA libraries to obtain isolated nucleic acids capable of hybridizing with clone 52, as discussed in Example 5. Nucleic acid regions extending the cDNA sequences in the 5' direction from the isolated human and rat partial cDNA clones were obtained by primer extension reactions and then sequenced.

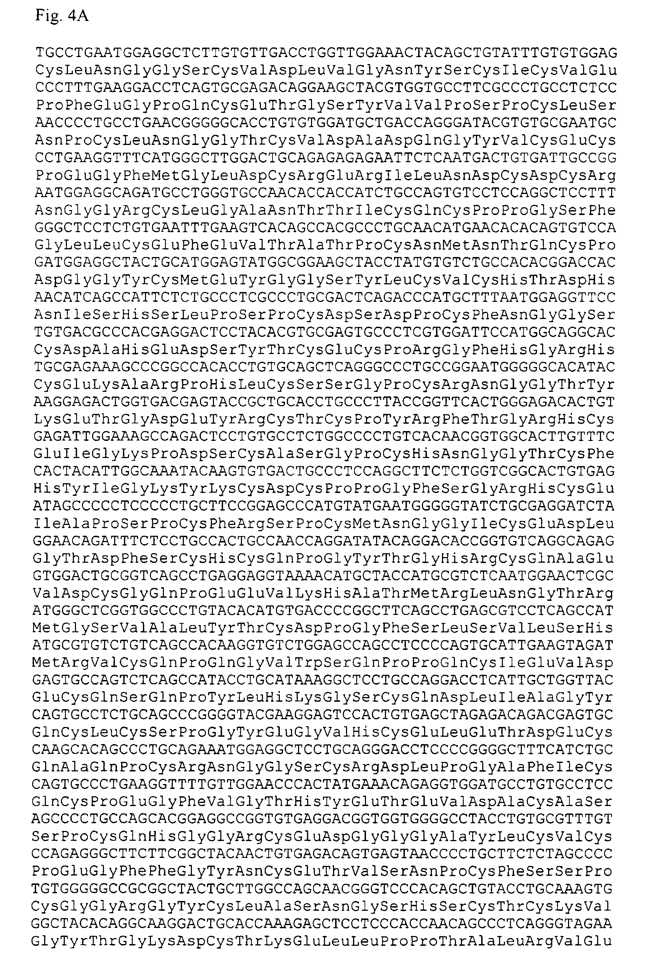

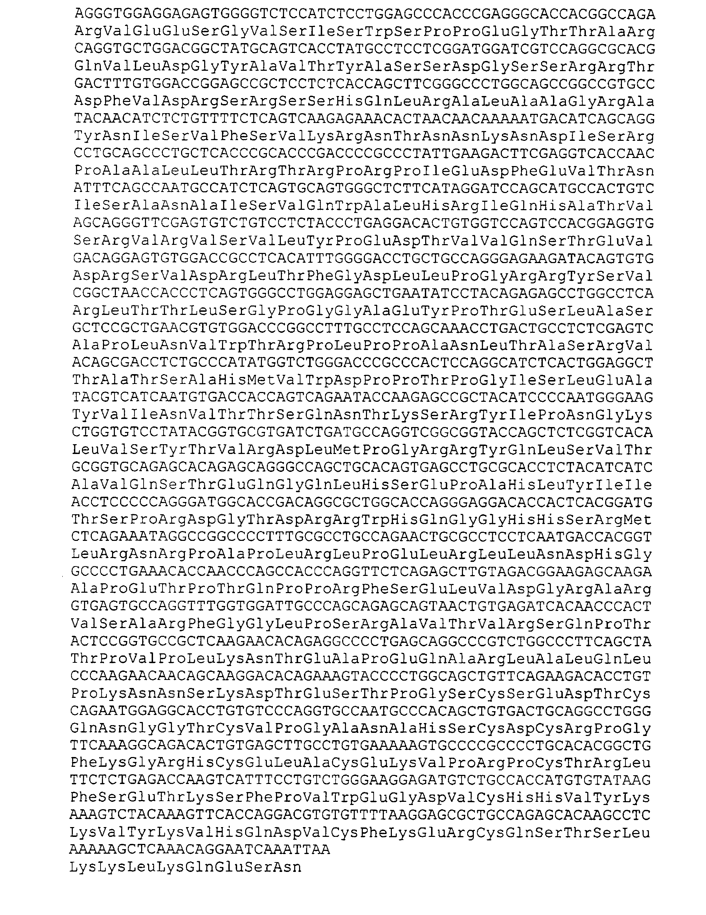

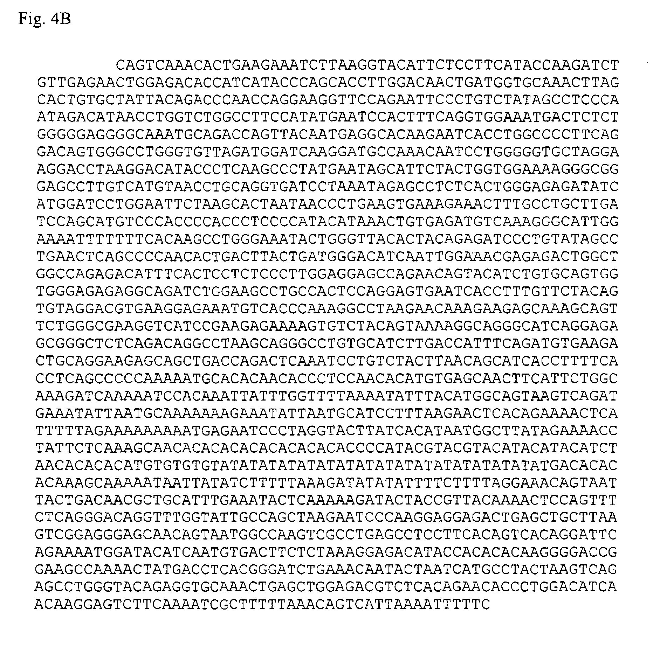

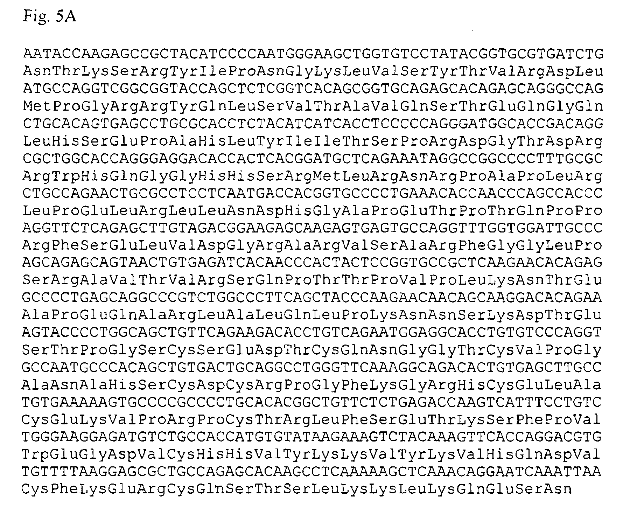

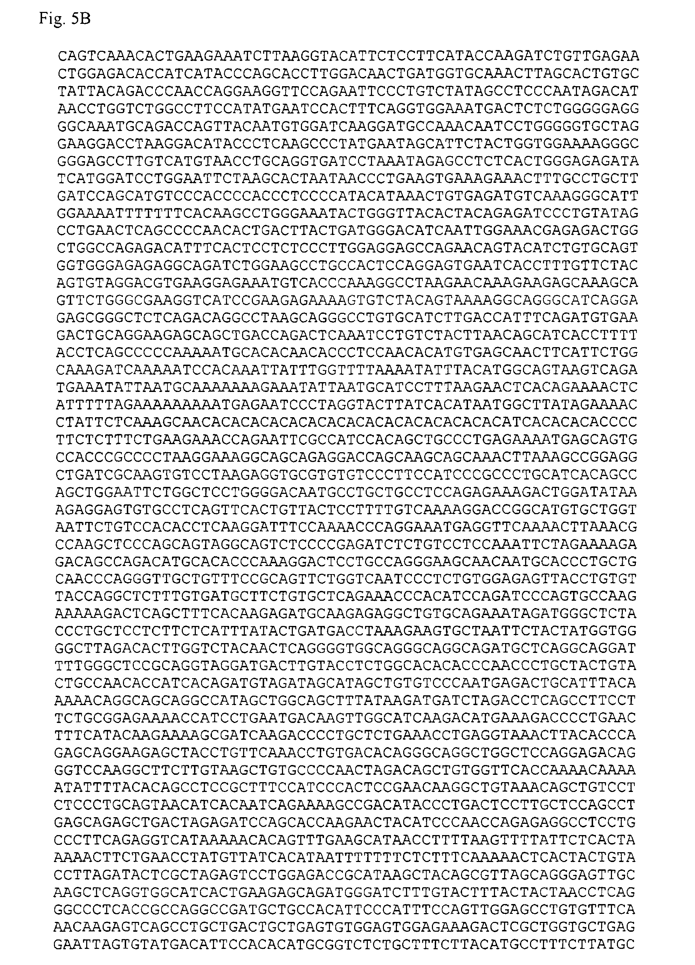

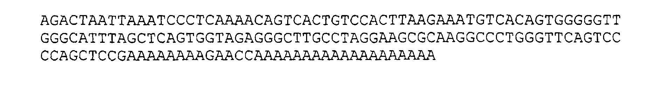

The present invention further provides two rat cDNA clones hybridizing to the clone 52 probe identified and sequenced as SEQ ID NOS: 5 and 6 and shown in Figs. 4A and 4B and 5A and 5B respectively. A first rat IRSBP-1 cDNA clone (SEQ ID NO 5; shown in Fig 4A and 4B) comprises about 4998 bp, and includes at least one open reading frame (ORF) as in Fig. 4A and which encodes a rat ISRBP-1 protein. The nucleotides at positions 68-349 of clone 52 (SEQ ID NO: 2) correspond to the nucleotide positions 2123-2404 of SEQ ID NO: 5 as shown in Fig. 4. A second rat cDNA clone (SEQ ID NO: 6, shown in Fig. 5A and 5B) is a partial cDNA comprising a partial open-reading frame (ORF) (Fig. 5A) having sequence similarity to a region of SEQ ID NO: 5 (Fig. 4A), and a 3'untranslated region (Fig. 5B) longer than that of SEQ ID NO: 5 (shown in Fig. 5B).

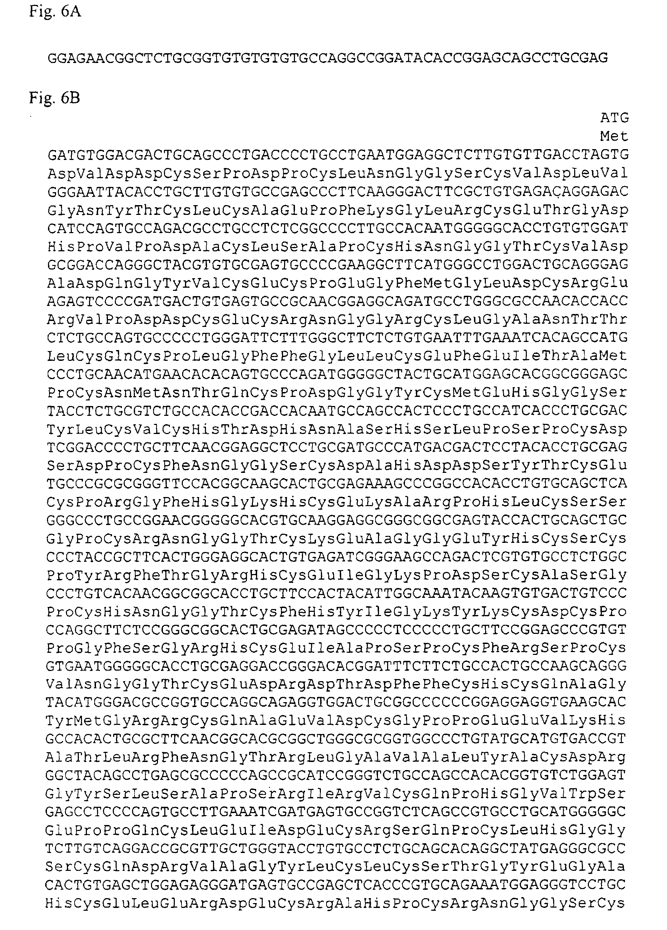

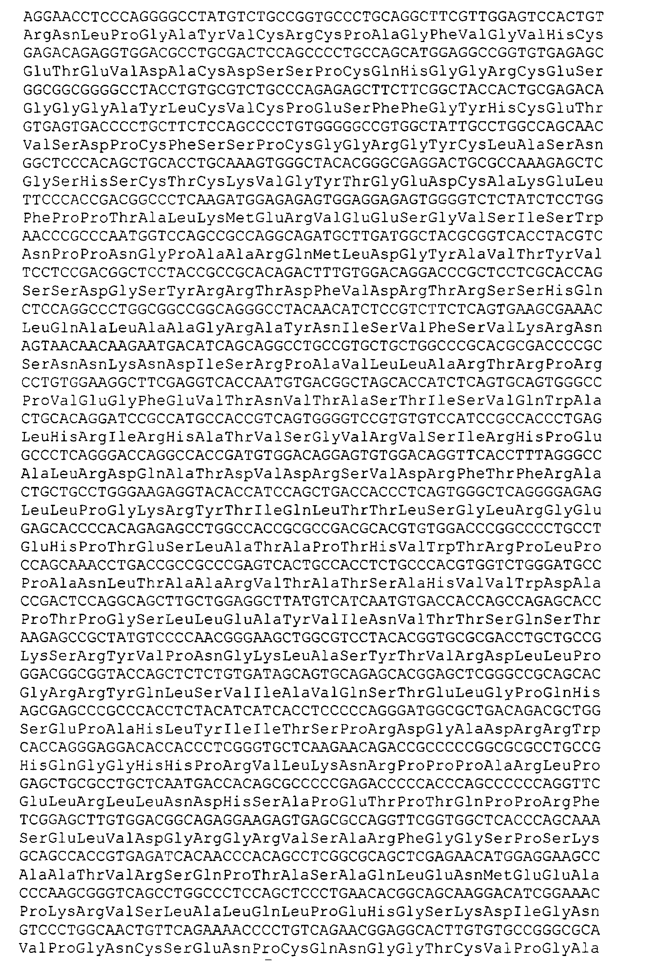

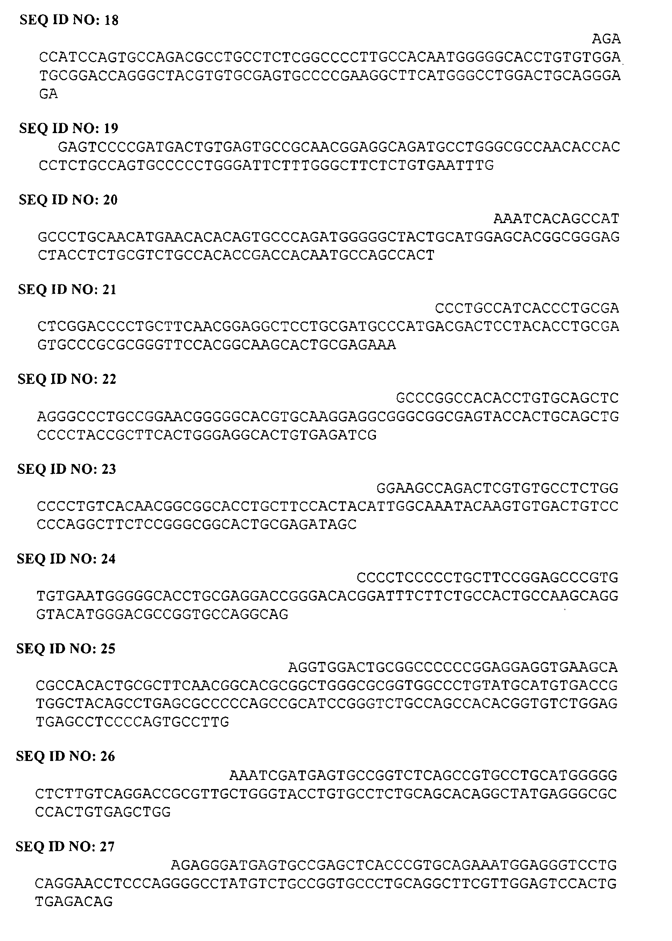

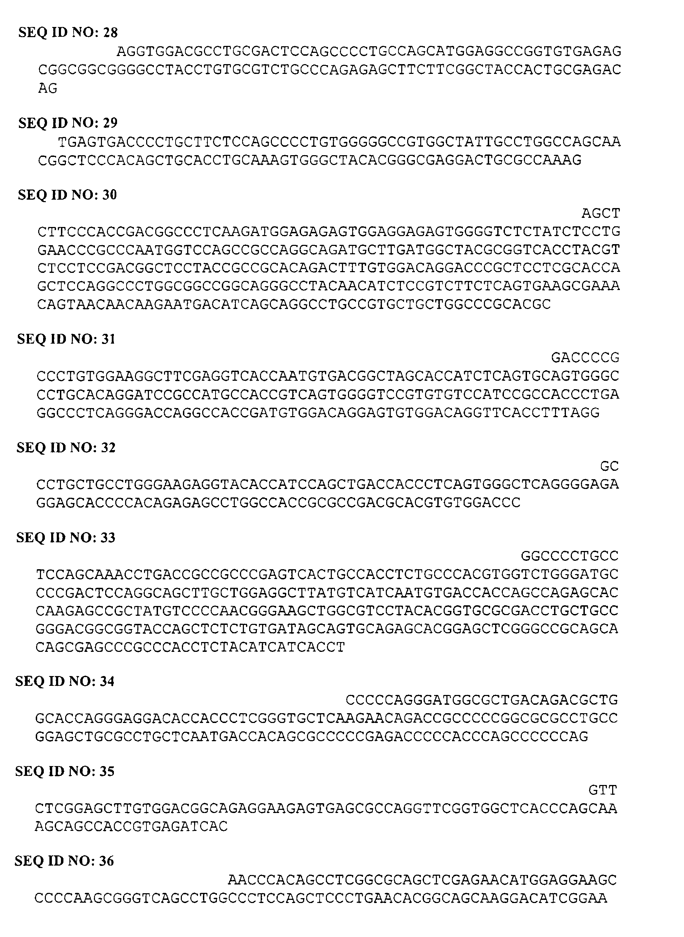

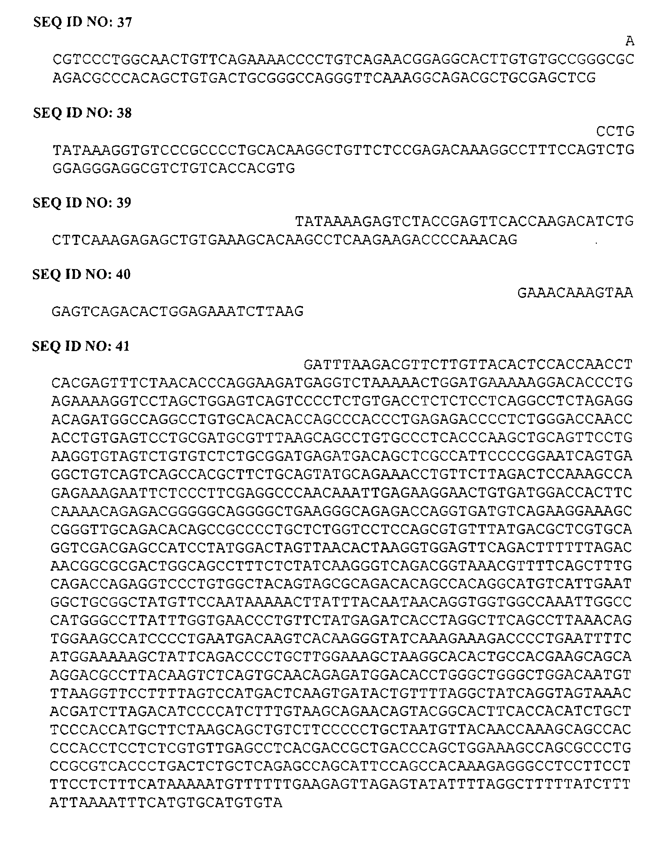

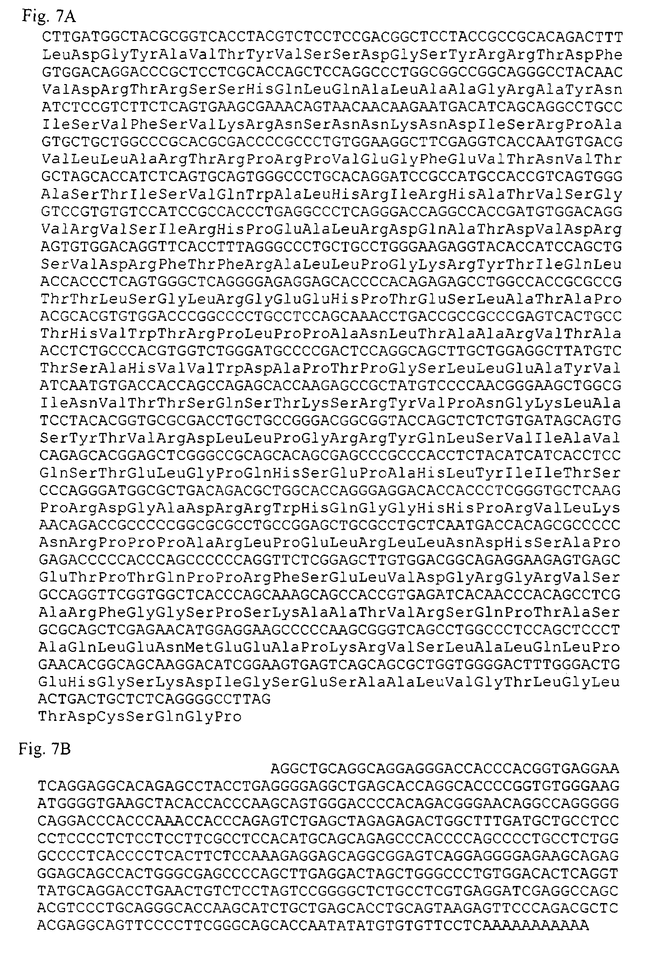

The present invention also provides for the human cDNA clones (SEQ ID NO: 7 as shown in Fig. 6A-6C and SEQ ID NO: 10 as shown in Fig. 7A and 7B) also identified by hybridization with the nucleic acid probe comprising the clone 52 nucleic acid sequence (SEQ ID NO: 2) during the screening of a human cDNA library.

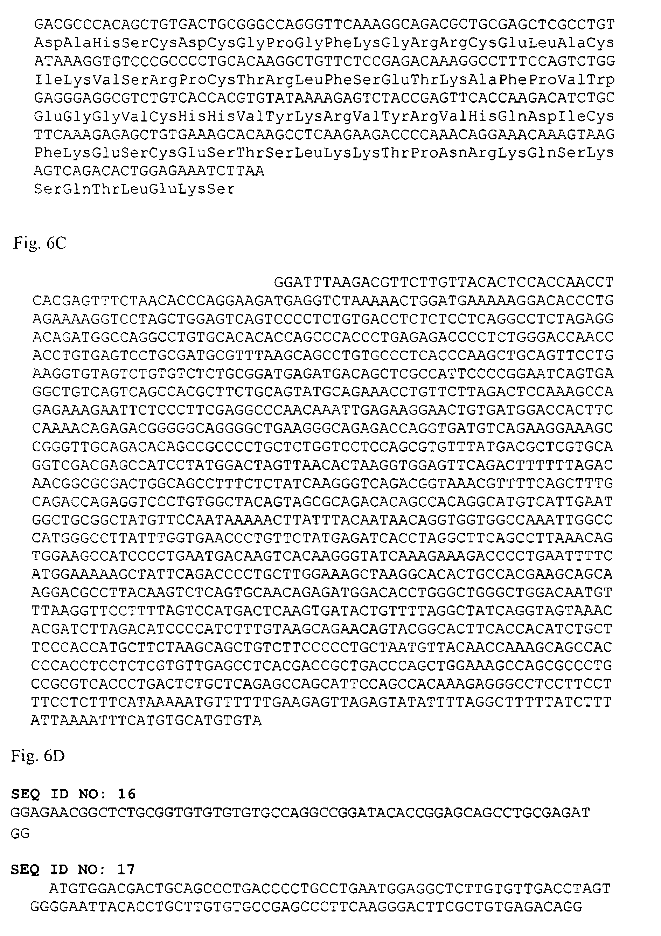

It is contemplated that any nucleic acid molecule of the present invention can comprise one or more regulatory regions, full-length or partial coding regions such as, but not limited to, fragments SEQ ID NOS: 16-41 as shown in Fig. 6D, or any combinations thereof. The minimal size of a nucleic acid molecule of the present invention is a size sufficient to allow the formation of a stable hybridization product with the complementary sequence of another nucleic acid molecule under selected stringency conditions.

Embodiments of the present invention may, therefore, include, but are not limited to, nucleic acid molecules such as: a) an IRSBP-1 cDNA molecule derived from the rat and comprising the protein coding region (SEQ ID NO: 8, shown in Fig. 4A) of SEQ ID NO: 5, and a 3' non-coding, or untranslated, region of SEQ ID NO: 5, shown in Fig. 4A and Fig. 4B respectively; b) an IRSBP-1 cDNA molecule derived from the rat nucleic acid SEQ ID NO: 5 and comprising the isolated coding region (SEQ ID NO: 8), the sequence of which is illustrated in Fig. 4A, or a substantial region thereof; or nucleic acid molecules representing degenerate variants, derivatives, modified sequences and truncated variants such as, but not limited to, SEQ ID NO: 6 shown in Figs. 5A and 5B, thereof; c) an IRSBP-1 cDNA molecule derived from the human comprising 5' and 3' non-coding regions and the protein coding region of the sequence SEQ ID NO: 7 as shown in Fig. 6A-C; d) a nucleic acid molecule derived from the human *IRSBP-1* cDNA sequence SEQ ID NO: 7 and comprising the human IRSBP-1 coding region alone (SEQ ID NO: 9), as depicted in Fig. 6B; and/or nucleic acid molecules representing degenerate variants, derivatives, alternatively spliced variants and modified variants thereof. A variant may be, but is not limited to, the sequence SEQ ID NO: 10 as shown in Fig. 7A and 7B. Such nucleic acid molecules can include nucleotides in addition to those included in SEQ ID NOS: 2, 5-10 such as, but not limited to, a full-length gene, a full-length coding region, or a nucleic acid molecule encoding a fusion protein. BLASTN algorithm searching of the Genbank database using the human IRSBP-1 nucleic acid sequence SEQ ID NO: 7 as the search target found that there was almost 100% identity with regions of the human genomic DNA sequence AC005237 from the human chromosome 1p31.31.3-32.2 and at least one human gene encoding the IRSBP-1 transcribed nucleic acid and protein derived therefrom is comprised of at least 26 exons as shown in Table 1, Example 5. The present invention, therefore, is intended also to provide isolated nucleic acids comprising at least one exon, or a fragment, variant or derivative thereof, capable of hybridizing with at least one region of the sequences SEQ ID NO:2, 5 - 10 under low, medium or high stringency conditions, wherein the hybridization is specific for an IRSBP-1-encoding nucleic acid, or a fragment, variant or derivative thereof.

One aspect of the invention therefore also provides nucleic acids that hybridize under high or low stringency conditions to a nucleic acid that encodes a peptide having all, a derivative of, or a portion of an amino acid sequence derived from the nucleic acid sequences SEQ ID NOS: 2, 5-10. Appropriate stringency conditions which promote DNA hybridization, for example, 6 x SSC at about 45°C., followed by a wash of 2 x SSC at 50°C., are well known to those skilled in the art or can be found, for example, in Current Protocols in Molecular Biology, John Wiley & Sons, N.Y. (1989), 6.3.1-6.3.6. For example, the salt concentration in the wash step can be selected from a low stringency of about 2 x SSC at 50°C. to a high stringency of about 0.2 x SSC at 50°C. In addition, the temperature in the wash step can be increased from low stringency conditions at room temperature, about 22°C., to high stringency conditions at about 65°C.

Isolated nucleic acids that differ in sequence from the nucleotide sequences represented in SEQ ID NOS: 2, 5-10 due to degeneracy in the genetic code are also within the scope of the invention. Such nucleic acids can encode functionally equivalent peptides (i.e., a polypeptide having a biological activity of a IRSBP-1 protein) but differ in sequence from the sequence shown in SEQ ID NO: 2, 5-10 due to degeneracy in the genetic code. Isolated nucleic acid sequence variants may also encode non-functional polypeptides, the sequences of which are substantially similar, but not identical, those of functional variants of IRSBP-1. These isolated nucleic acids may be used to generate variant animals with inactive or functionally modified IRSBP-1 polypeptides or fragments, variants or derivatives thereof.

For example, a number of amino acids are designated by more than one triplet. Codons that specify the same amino acid, or synonyms (for example, CAU and CAC are synonyms for histidine) may result in "silent" mutations which do not affect the amino acid sequence of the subject protein. However, it is expected that DNA sequence polymorphisms that do lead to changes in the amino acid sequences of the present IRSBP-1 protein of the present invention will exist from one human or animal subject to the next of the same species. One skilled in the art will appreciate that these variations in one or more nucleotides (up to about 3-4% of the nucleotides) of the nucleic acids encoding peptides having an activity of, for example, an IRSBP-1 protein may exist among individuals due to natural allelic variation. Any and all such nucleotide variations and resulting amino acid polymorphisms are within the scope of this invention.

Fragments of a nucleic acid encoding an active portion of one of the subject IRSBP-1 proteins are also within the scope of the invention. As used herein, a fragment of the nucleic acid encoding an active portion of a IRSBP-1 protein refers to a nucleotide sequence having fewer nucleotides than the nucleotide sequence encoding the entire amino acid sequence of the protein but which encodes a peptide that possesses agonistic or antagonistic activity relative to a naturally occurring form of the protein.

Nucleic acid fragments within the scope of the invention also include those capable of hybridizing under high or low stringency conditions with nucleic acids from other species for use in screening protocols to detect IRSBP-1 homologs. Comparison of the nucleic acid sequences of rat and human IRSBP-1 show that oligonucleotide primers can be generated that are suitable for detecting and isolating IRSBP-1 clones in other eukaryotes. For example, the cDNA clone 52 (SEQ ID NO: 1) can be used to detect IRSBP-1 homologs in other vertebrate species, such as, but not only, human, mice, rats, chickens.

One embodiment of the present invention provides a nucleic acid comprising a nucleic acid sequence substantially similar to the clone 52 cDNA sequence (SEQ ID NO: 2) encoding at least a region of a rat IRSBP-1 protein (SEQ ID NO: 3) as shown in Figs. 1 and 2 respectively, or any variants thereof. The nucleic acid molecules of the present invention can include an isolated deletion mutation corresponding to the IRSBP-1 phenotype, a natural *IRSBP-1* gene, an IRSBP-1 cDNA molecule, a degenerate variant, a truncated form thereof, a homolog thereof or any other modified versions.

In another embodiment of the present invention, a nucleic acid is provided comprising a nucleic acid sequence substantially similar to the cDNA sequence for a rat *IRSBP-1* (SEQ ID NO: 5) as shown in Figs. 4A and 4B, or any variant thereof. The nucleic acid molecules of the present invention can include an isolated deletion mutation corresponding to the IRSBP-1 phenotype, a natural *IRSBP-1* gene, an IRSBP-1 cDNA molecule, a degenerate variant thereof, a truncated variant thereof or a homolog thereof or any other variant thereof

In yet another embodiment of the present invention, a nucleic acid is provided comprising a nucleic acid sequence substantially similar to the cDNA sequence for a rat IRSBP-1 (SEQ ID NO: 6) as shown in Figs. 5A and 5B comprising a variant of SEQ ID NO: 5.

In yet another embodiment of the present invention, an isolated nucleic acid is provided that comprises the nucleic acid sequence corresponding to a human IRSBP-1 sequence (SEQ ID NO: 7) as shown in Figs. 6A - 6C.

In another embodiment of the present invention, an isolated nucleic acid is provided that comprises the nucleic acid sequence corresponding to a variant human IRSBP-1 (SEQ ID NO: 8) as shown in Figs. 7A and 7B.

In still another embodiment of the present invention, a mammalian *IRSBP-1* gene or nucleic acid molecule can be allelic variants of SEQ ID NOS: 5 - 10. An allelic variant is a gene that occurs essentially at the same locus or loci in the mammalian genome as the genes comprising SEQ ID NOS: 5 - 10, but which has similar, but not identical, sequences to that of SEQ ID NO: 5 - 10.

In one embodiment of the present invention, an isolated nucleic acid molecule of the present invention includes a nucleic acid that is at least about 75%, preferably at least about 80%, more preferably at least about 85%, even more preferably at least about 90%, and even more preferably at least about 95% identical to a rat-derived IRSBP-1-encoding nucleic acid molecule as depicted in SEQ ID NO: 5, and/or a variant thereof, such as, but not limited to, SEQ ID NO: 6.

In another embodiment of the present invention, an isolated nucleic acid molecule of the present invention includes a nucleic acid that is at least about 75%, preferably at least about 80%, more preferably at least about 85%, even more preferably at least about 90%, and even more preferably at least about 95% identical to a nucleic acid molecule as depicted in SEQ ID NO: 7, and/or a variant thereof, such as, but not limited to, SEQ ID NO: 8.

The nucleic acid sequences of a IRSBP-1 nucleic acid molecules (SEQ ID NOS: 2, 5 - 10) of the present invention allow one skilled in the art to, for example, (a) make copies of those nucleic acid molecules by procedures such as, but not limited to, insertion into a cell for replication by the cell, by chemical synthesis or by procedures such as PCR or LCR, (b) obtain nucleic acid molecules which include at least a portion of such nucleic acid molecules, including full-length genes, full-length coding regions, regulatory control sequences, truncated coding regions and the like, (c) obtain IRSBP-1 nucleic acid homologs in other mammalian species such as the dog, cat, cow, pig or primates other than human and, (d) to obtain isolated nucleic acids capable of hybridizing to a mammalian IRSBP-1 nucleic acid and be used to detect the presence of IRSBP-1 nucleic acid sequences by complementation between the probe and the target nucleic acid.

Such nucleic acid homologs can be obtained in a variety of ways including by screening appropriate expression libraries with antibodies of the present invention; using traditional cloning techniques employing oligonucleotide probes made according to the present invention to screen appropriate libraries; amplifying appropriate libraries or DNA using oligonucleotide primers of the present invention in a polymerase chain reaction or other amplification method; and screening public and/or private databases containing genetic sequences using nucleic acid molecules of the present invention to identify targets. Examples of preferred libraries to screen, or from which to amplify nucleic acid molecules, include but are not limited to mammalian BAC libraries, genomic DNA libraries, and cDNA libraries. Similarly, preferred sequence databases useful for screening to identify sequences in other species homologous to *IRSBP-1* include, but are not limited to, GenBank and the mammalian Gene Index database of The Institute of Genomics Research (TIGR).

Another aspect of the present invention is to provide protein sequences that comprise a mammalian IRSBP-1 protein, and derivatives and fragments thereof. One embodiment of the present invention, therefore, comprises a protein sequence (SEQ ID NO: 3, as in Fig 2) encoded by the rat cDNA clone 52 nucleic acid sequence (SEQ ID NO: 2; as shown in Fig. 1).

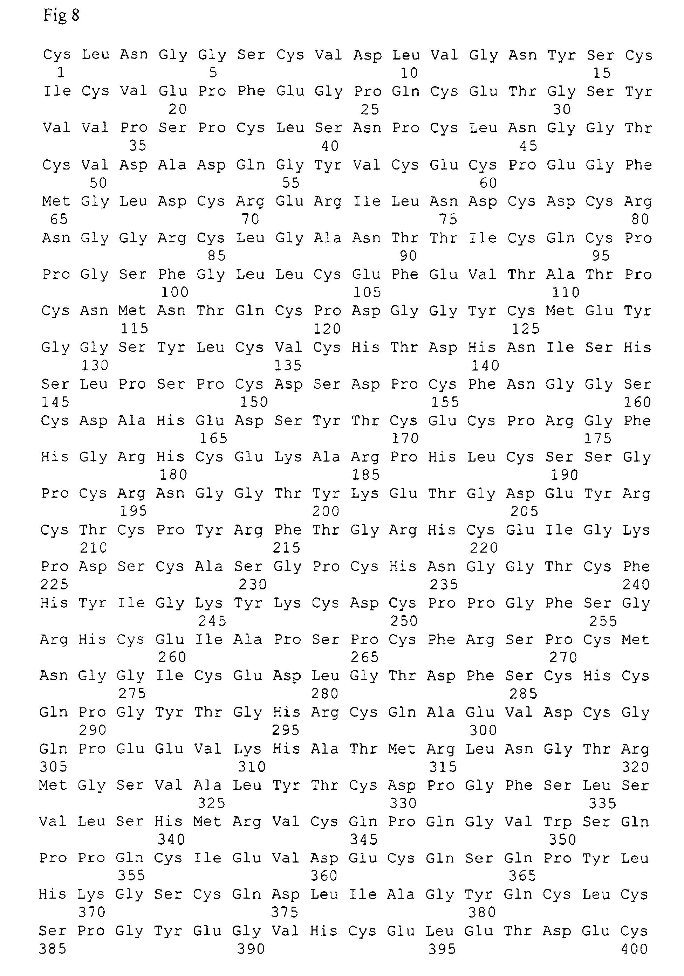

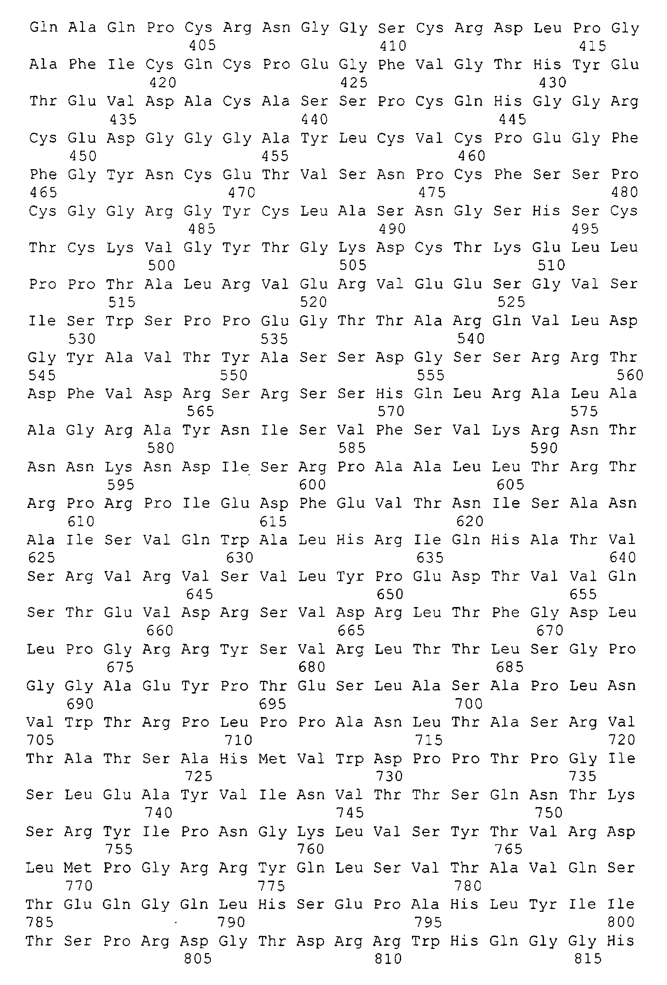

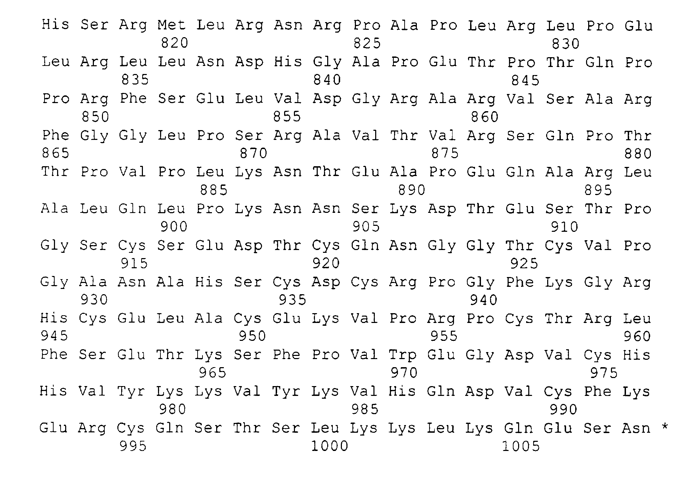

In another embodiment of the present invention, a rat IRSBP-1 protein is provided having an amino acid sequence (SEQ ID NO: 11, illustrated in Fig. 8) derived from the coding region of the rat cDNA clone IRSBP-1 (SEQ ID NO: 8, as in Fig. 4A).

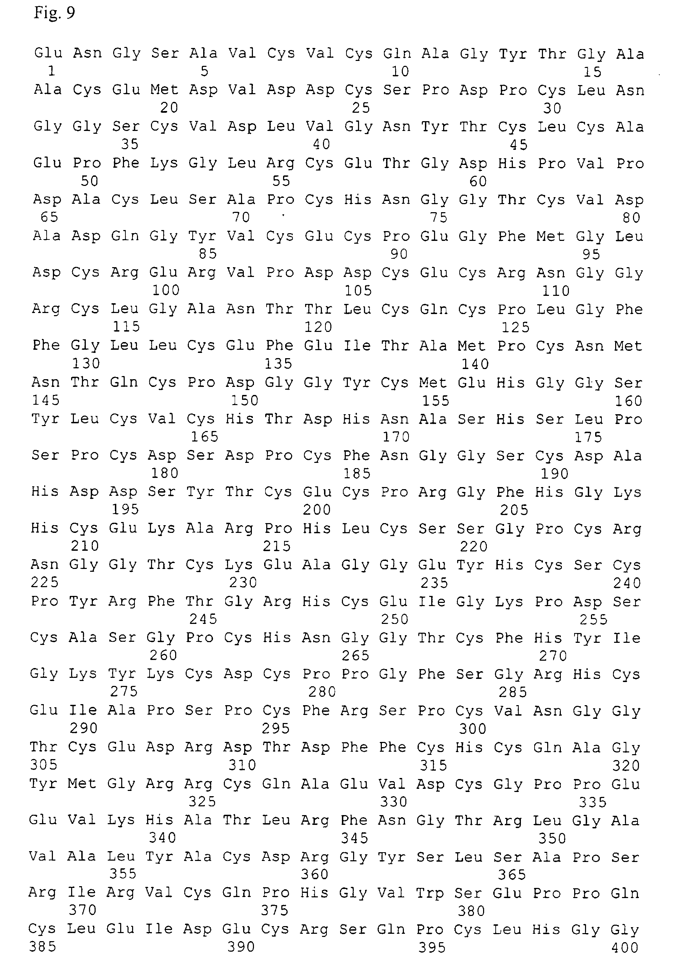

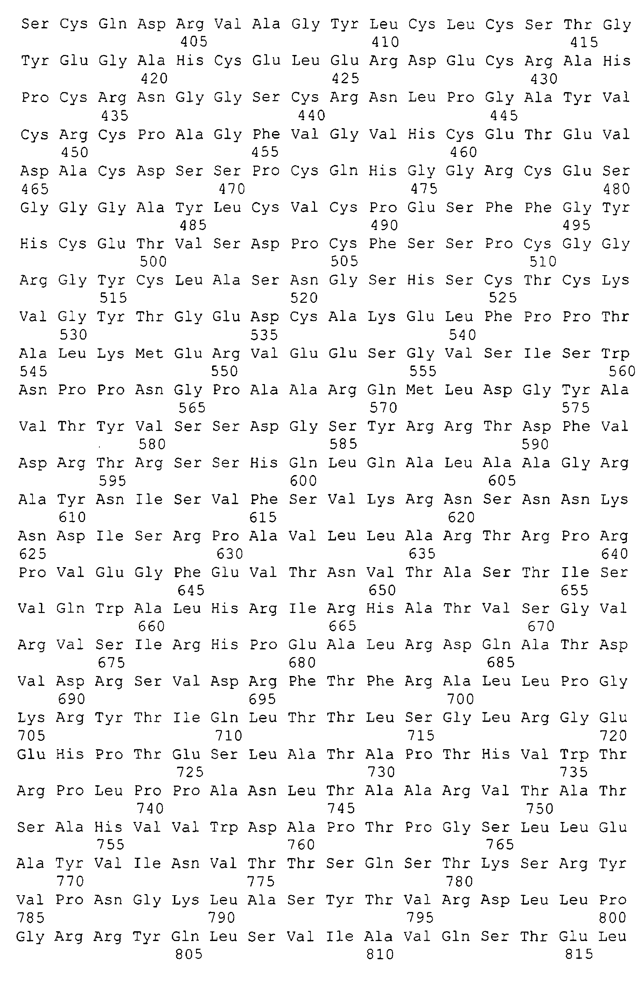

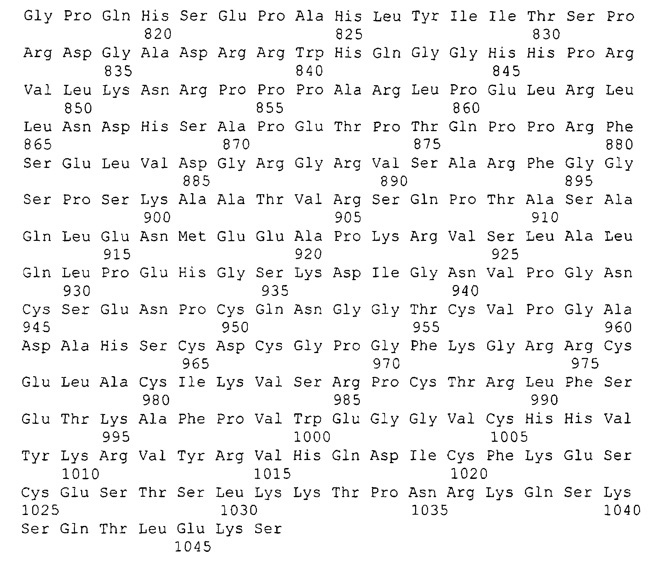

In yet another embodiment of the present invention, a human IRSBP-1 protein sequence is provided (SEQ ID NO: 12, illustrated in Fig 9) that is encoded by a coding region of the human nucleic acid sequence SEQ ID NO: 9, shown in Fig. 6B.

In still other embodiments of the present invention, peptide fragments of a human or animal IRSBP-1 protein are provided, wherein the fragments may be immunogenic peptides, capable of inducing an immune response when administered to an animal, and which will be recognized and bound by an antibody or not immunogenic when administered to an animal.

In one embodiment of the present invention, the peptide fragment is an epitope of the rat IRSBP-1 protein (SEQ ID NO: 3 as in Fig. 2) and has the amino acid sequence: Acetylated Cys-Thr-Ser-Gln-Asn-Thr-Lys-Ser-Arg-Ty-Iso-Pro-Asn-Gly-Lys-Leu (SEQ ID NO: 15) at amino acid positions 62-76 of the rat IRSBP-1 amino acid sequence SEQ ID NO: 3 as shown in Fig. 2.

Further contemplated to be within the scope of the present invention are proteins having substantial similarity to the rat or human protein amino acid sequences SEQ ID NOS: 11 and 12 respectively. Isolated peptides and polypeptides of the present invention may also include any protein fragments thereof, a protein analogue, or any immunologic fragments thereof.

In another embodiment of the present invention, an *IRSBP-1* nucleic acid molecule of the present invention encodes a protein having an amino acid sequence that is at least about 75%, preferably at least about 80%, more preferably at least about 85%, even more preferably at least about 90%, and more preferably still at least about 95% identical to a rat IRSBP-1 protein whose amino acid sequence is disclosed in SEQ ID NO: 11, as well as allelic variants of an *IRSBP-1* nucleic acid molecule encoding a protein having these sequences, including nucleic acid molecules that have been modified to accommodate codon usage properties of the cells in which such nucleic acid molecules are to be expressed.

In an embodiment of the present invention, an *IRSBP-1* nucleic acid molecule of the present invention encodes a protein having an amino acid sequence that is at least about 75%, preferably at least about 80%, more preferably at least about 85%, even more preferably at least about 90%, and more preferably still at least about 95% identical to a human IRSBP-1 protein whose amino acid sequence is disclosed in SEQ ID NO: 12, as well as allelic variants of an *IRSBP-1* nucleic acid molecule encoding a protein having these sequences, including nucleic acid molecules that have been modified to accommodate codon usage properties of the cells in which such nucleic acid molecules are to be expressed.

Isolated peptidyl portions of the subject IRSBP-1 proteins within the scope of the present invention can be obtained by screening peptides recombinantly produced from the corresponding fragment of the nucleic acid encoding such peptides. In addition, fragments can be chemically synthesized using techniques known in the art such as conventional Merrifield solid phase f-Moc or t-Boc chemistry. For example, one of the subject IRSBP-1 proteins may be arbitrarily divided into fragments of desired length with no overlap of the fragments, or preferably divided into overlapping fragments of a desired length. The fragments can be produced recombinantly or by chemical synthesis and tested to identify those peptidyl fragments which can function as either agonists or antagonists of, for example, IRSBP-1 binding to nucleic acids. In an illustrative embodiment, peptidyl portions of IRSBP-1 can tested for nucleic acid-binding activity, as well as preventing inhibitory ability, by expression as, for example, thioredoxin fusion proteins each of which contains a discrete fragment of the IRSBP-1 protein (see, for example, U.S. Patent Nos. 5,270,181 and 5,292,646; and PCT publication WO94/02502).
Furthermore, it is also possible to modify the structure of an IRSBP-1 polypeptide for such purposes as enhancing therapeutic or prophylactic efficacy, or dextran or stability (e.g., shelf life ex vivo and resistance to proteolytic degradation *in vivo*)*.* Such modified peptides are considered functional equivalents of peptides having an activity of, or which antagonize, a IRSBP-1 protein as defined herein. A modified polypeptide can be produced in which the amino acid sequence has been altered, such as by amino acid substitution, deletion, or addition.

For example, it is reasonable to expect that an isolated replacement of a leucine with an isoleucine or valine, an aspartate with a glutamate, a threonine with a serine, or a similar replacement of an amino acid with a structurally related amino acid (i.e. conservative mutations) will not have a major effect on the biological activity of the resulting molecule. Conservative replacements are those that take place within a family of amino acids that are related in their side chains. Genetically encoded amino acids can be divided into four families: (1) acidic = aspartate, glutamate; (2) basic = lysine, arginine, histidine; (3) nonpolar = alanine, valine, leucine, isoleucine, proline, phenylalanine, methionine, tryptophan; and (4) uncharged polar = glycine, asparagine, glutamine, cysteine, serine, threonine, tyrosine. Phenylalanine, tryptophan, and tyrosine are sometimes classified jointly as aromatic amino acids. In similar fashion, the amino acid repertoire can be grouped as (1) acidic = aspartate, glutamate; (2) basic = lysine, arginine histidine, (3) aliphatic = glycine, alanine, valine, leucine, isoleucine, serine, threonine, with serine and threonine optionally be grouped separately as aliphatic-hydroxyl; (4) aromatic = phenylalanine, tyrosine, tryptophan; (5) amide = asparagine, glutamine; and (6) sulfur-containing = cysteine and methionine. (see, for example, Biochemistry, 2nd ed, Ed. by L. Stryer, WH Freeman and Co.:1981). Whether a change in the amino acid sequence of a peptide results in a functional IRSBP-1 homolog can be readily determined by assessing the ability of the variant peptide to, for instance, mediate ubiquitination in a fashion similar to the wild-type IRSBP-1. Peptides in which more than one replacement has taken place can readily be tested in the same manner.

In one embodiment of the present invention, therefore, a host cell is transformed with a nucleic acid comprising the sequences SEQ ID NOS: 5 - 8, or variants thereof. The transformed cell may, but not necessarily, express the transformed nucleic acid to yield rat or human IRSBP-1 polypeptides SEQ ID NOS: 11 or 12 respectively, or any fragment or derivative thereof. A recombinant expression vector suitable for transformation of a host cell means that the recombinant expression vector contains a nucleic acid molecule, or an oligonucleotide fragment thereof, of the present invention coupled to a regulatory sequence selected on the basis of the host cell used for expression. For example, the nucleic acid sequence coding for the IRSBP-1 protein of the present invention may be operatively linked to a regulatory sequence selected to direct expression of the desired protein in an appropriate host cell.

The protein of the present invention may be produced in purified form by any known conventional techniques. For example, rat or human cells may be homogenized and centrifuged. The supernatant is then subjected to sequential ammonium sulfate precipitation and heat treatment. The fraction containing the protein of the present invention is subjected to gel filtration in an appropriately sized dextran or polyacrylamide column to separate the proteins. If necessary, the protein fraction may be further purified by HPLC.

The present invention provides novel compositions comprising nucleotide sequences encoding IRSBP-1 fragments. Also provided are recombinant proteins produced using the novel coding sequences, and methods of using the recombinant proteins.

The DNA nucleic acid molecules of the present invention can be incorporated into cells using conventional recombinant DNA technology. The DNA molecule may be inserted into an expression system to which the DNA molecule is heterologous (i.e. not normally present). Alternatively, as described more fully below, the DNA molecule may be introduced into cells which normally contain the DNA molecule, as, for example, to correct a deficiency or defect in IRSBP-1 expression, or where over-expression of the IRSBP-1 protein is desired.

For expression in heterologous systems, the heterologous DNA molecule is inserted into the expression system or vector in proper sense orientation and correct reading frame. The vector contains the necessary elements for the transcription and translation of the inserted protein-coding sequences.

U.S. Patent No. 4,237,224 to *Cohen & Boyer,* describes the production of expression systems in the form of recombinant plasmids using restriction enzyme cleavage and ligation with DNA ligase. These recombinant plasmids are then introduced by means of transformation and replicated in unicellular cultures including prokaryotic organisms and eukaryotic cells grown in tissue culture.

Recombinant genes may also be introduced into viruses, such as vaccinia virus. Recombinant viruses can be generated by transfection of plasmids into cells infected with virus. Suitable vectors include, but are not limited to, the following viral vectors such as lambda vector system gt11, gt WES.tB, Charon 4, and plasmid vectors such as pBR322, pBR325, pACYC177, pACYC184, pUC8, pUC9, pUC18, pUC19, pLG339, pR290, pKC37, pKC101, SV 40, pBluescript II SK +/- or KS +/(see "Stratagene Cloning Systems" Catalog (1993) from Stratagene, La Jolla, Calif., pQE, pIH821, PGEX, pET series (see *Studier, F. W. et. al.* (1990) "Use of T7 RNA Polymerase to Direct Expression of Cloned Genes" Gene Expression Technology, vol. 185, and any derivatives thereof. Recombinant molecules can be introduced into cells via transformation, particularly transduction, conjugation, mobilization, or electroporation. The DNA sequences are cloned into the vector using standard cloning procedures in the art, as described by *Maniatis et al.* Molecular Cloning: A Laboratory Manual, Cold Springs Laboratory, Cold Springs Harbor, N.Y. (1982).

A variety of host-vector systems may be utilized to express the protein-encoding sequence(s). Primarily, the vector system must be compatible with the host cell used. Host-vector systems include but are not limited to the following: bacteria transformed with bacteriophage DNA, plasmid DNA, or cosmid DNA; microorganisms such as yeast containing yeast vectors; mammalian cell systems infected with virus (e.g., vaccinia virus, adenovirus, etc.); insect cell systems infected with virus (e.g., baculovirus). The expression elements of these vectors vary in their strength and specificities. Depending upon the host-vector system utilized, any one of a number of suitable transcription and translation elements can be used.

Different genetic signals and processing events control many levels of gene expression (e.g., DNA transcription and messenger RNA (mRNA) translation). Transcription of DNA is dependent upon the presence of a promoter which is a DNA sequence that directs the binding of RNA polymerase and thereby promotes mRNA synthesis. The DNA sequences of eukaryotic promoters differ from those of prokaryotic promoters. Furthermore, eukaryotic promoters and accompanying genetic signals may not be recognized in or may not function in a prokaryotic system, and, further, prokaryotic promoters are not recognized and do not function in eukaryotic cells.

Similarly, translation of mRNA in prokaryotes depends upon the presence of the proper prokaryotic signals that differ from those of eukaryotes. Efficient translation of mRNA in prokaryotes requires a ribosome binding site called the Shine-Dalgamo (SD) sequence on the mRNA. This sequence is a short nucleotide sequence of mRNA that is located before the start codon, usually AUG, which encodes the amino-terminal methionine of the protein. The SD sequences are complementary to the 3'-end of the 16S rRNA (ribosomal RNA) and probably promote binding of mRNA to ribosomes by duplexing with the rRNA to allow correct positioning of the ribosome. For a review on maximizing *gene* expression, see *Roberts & Lauer* (1979) Methods in Enzymology 68: 473.

Promoters vary in their "strength" (i.e. their ability to promote transcription). For the purposes of expressing a cloned gene, it is desirable to use strong promoters in order to obtain a high level of transcription and, hence, expression of the gene. Depending upon the host cell system utilized, any one of a number of suitable promoters may be used. For instance, when cloning in E. coli, its bacteriophages, or plasmids, promoters such as the T7 phage promoter, lac promotor, trp promotor, recA promotor, ribosomal RNA promotor, the P_{R} and P_{L} promoters of coliphage lambda and others, including but not limited, to lacUV5, ompF, bla, Ipp, and the like, may be used to direct high levels of transcription of adjacent DNA segments. Additionally, a hybrid trp-lacUV5 (tac) promotor or other E. coli promoters produced by recombinant DNA or other synthetic DNA techniques may be used to provide for transcription of the inserted gene.

Bacterial host cell strains and expression vectors may be chosen which inhibit the action of the promotor unless specifically induced. In certain operons, the addition of specific inducers is necessary for efficient transcription of the inserted DNA. For example, the lac operon is induced by the addition of lactose or IPTG (isopropylthiobeta-D-galactoside). A variety of other operons, such as trp, pro, etc., are under different controls.

Specific initiation signals are also required for efficient gene transcription and translation in prokaryotic cells. These transcription and translation initiation signals may vary in "strength" as measured by the quantity of *gene* specific messenger RNA and protein synthesized, respectively. The DNA expression vector, which contains a promoter, may also contain any combination of various "strong" transcription and/or translation initiation signals. For instance, efficient translation in *E. coli* requires a Shine-Dalgarno (SD) sequence about 7-9 bases 5' to the initiation codon (ATG) to provide a ribosome binding site. Thus, any SD-ATG combination that can be utilized by host cell ribosomes may be employed. Such combinations include but are not limited to the SD-ATG combination from the *cro* gene or the *N* gene of coliphage lambda, or from the *E. coli* tryptophan E, D, C, B or A genes. Additionally, any SD-ATG combination produced by recombinant DNA or other techniques involving incorporation of synthetic nucleotides may be used.

Once the isolated DNA molecule of the present invention has been cloned into an expression system, it is ready to be incorporated into a host cell. Such incorporation can be carried out by the various forms of transformation noted above, depending upon the vector/host cell system. Suitable host cells include, but are not limited to, bacteria, virus, yeast, mammalian cells, and the like.

Recombinant expression vectors can be designed for the expression of the encoded proteins in prokaryotic or eukaryotic cells. The prokaryotic expression system may comprise the host bacterial species *E*. *coli, B. subtilis* or any other host cell known to one of skill in the art. Useful vectors may comprise constitutive or inducible promoters to direct expression of either fusion or non-fusion proteins. With fusion vectors, a number of amino acids are usually added to the expressed target gene sequence such as, but not limited to, a protein sequence for thioredoxin. A proteolytic cleavage site may further be introduced at a site between the target recombinant protein and the fusion sequence. Additionally, a region of amino acids such as a polymeric histidine region may be introduced to allow binding to the fusion protein by metallic ions such as nickel bonded to a solid support, and thereby allow purification of the fusion protein. Once the fusion protein has been purified, the cleavage site allows the target recombinant protein to be separated from the fusion sequence. Enzymes suitable for use in cleaving the proteolytic cleavage site includes, but are not limited to, Factor Xa and thrombin. Fusion expression vectors that may be useful in the present invention include pGex (Amrad Corp., Melbourne, Australia), pRIT5 (Pharmacia, Piscataway, NJ) and pMAL (New England Biolabs, Beverly, MA), that fuse glutathione S-transferase, protein A, or maltose E binding protein, respectively, to the target recombinant protein.

Expression of unfused foreign genes in *E. coli* may be accomplished with recombinant vectors including, but not limited to, the *E. coli* expression vector pUR278 as described in *Ruther et al.* (1983) *E.M.B.O.J.* 2: 1791.

Using the pUR278 vector, the nucleotide sequence coding for the *IRSBP-1* gene product may be ligated in frame with the *lacV* coding region to produce a fusion protein.
Expression of a foreign gene can also be obtained using eukaryotic vectors such as mammalian, yeast or insect cells. The use of eukaryotic vectors permits partial or complete post-translational modification such as, but not only, glycosylation and/or the formation of the relevant inter- or intra-chain disulfide bonds. Examples of vectors useful for expression in the yeast *Saccharomyces cerevisiae* include pYepSec1 as in *Baldari et al.,* (1987), *E*.*M*.*B*.*O*.*J*., 6: 229-234 and pYES2 (Invitrogen Corp., San Diego, CA).

Baculovirus vectors are also available for the expression of proteins in cultured insect cells (F9 cells). The use of recombinant Baculovirus vectors can be, or is, analogous to the methods disclosed in *Richardson C.D.* ed., (1995), "Baculovirus Expression Protocol" Humana Press Inc.; *Smith et al.* (1983) Mol. Cell. Biol. 3: 2156-2165; *Pennock et al.* (1984) Mol. Cell. Biol. 4: 399-406.
Other vectors useful for expressing the IRSBP-1 protein, or an epitope of a IRSBP-1 protein, include viral vectors. Methods for making a viral recombinant vector useful for expressing the IRSBP-1 protein are analogous to the methods disclosed in U.S. Patent Nos. 4,603,112; 4,769,330; 5,174,993; 5,505,941; 5,338,683; 5,494,807; 4,722,848; *Paoletti, E.* (1996) Proc. Natl. Acad. Sci. 93: 11349-11353; *Moss* (1996) Proc. Natl. Acad. Sci. 93: 11341-11348; *Roizman* (1996) Proc. Natl. Acad. Sci. 93: 11307-11302; *Frolov et al.* (1996) Proc. Natl. Acad. Sci. 93: 11371-11377; *Grunhaus et al.* (1993) Seminars in Virology 3: 237-252 and U.S. Patent Nos. 5,591,639; 5,589,466; and 5,580,859 relating to DNA expression vectors, *inter alia.*

Another aspect of the present invention pertains to the use of an isolated nucleic acid molecule for constructing nucleotide probes and primers useful for a variety of functions. For example, synthetic oligonucleotide probes are useful for detecting complementary nucleotide sequences in biological materials such as cells, cell extracts or tissues (as well as in an *in situ* hybridization technique). For example, isolated nucleic acids synthesized according to the present invention can determine whether a cell expresses an mRNA transcript encoding the IRSBP-1 protein. The present invention also contemplates the use of antisense nucleic acid molecules, which are designed to be complementary to a coding strand of a nucleic acid (i.e., complementary to an mRNA sequence) or, alternatively, complimentary to a 5' or 3' untranslated region of the mRNA. Another use of synthetic nucleotides is as primers (DNA or RNA) for a polymerase chain reaction (PCR), ligase chain reaction (LCR), or the like.
Synthesized nucleotides can be produced in variable lengths - the number of bases synthesized will depend upon a variety of factors, including the desired use for the probes or primers. Additionally, sense or anti-sense nucleic acids or oligonucleotides can be chemically synthesized using modified nucleotides to increase the biological stability of the molecule or of the binding complex formed between the anti-sense and sense nucleic acids. For example, acridine substituted nucleotides can be synthesized. Protocols for designing isolated nucleotides, nucleotide probes, and/or nucleotide primers are well-known to those of ordinary skill, and can be purchased commercially from a variety of sources (e.g., Sigma Genosys, The Woodlands, TX or The Great American Gene Co., Ramona, CA).

Nucleotides constructed in accordance with the present invention can be labeled to provide a signal as a means of detection. For example, radioactive elements such as ³²P, ³H, and ³⁵S or the like provide sufficient half-life to be useful as radioactive labels. Other materials useful for labeling synthetic nucleotides include fluorescent compounds, enzymes and chemiluminescent moieties. Methods useful in selecting appropriate labels and binding protocols for binding the labels to the synthetic nucleotides are well known to those of skill in the art. Standard immunology manuals such as Promega: Protocol and Applications Guide, 2nd Edition, 1991 (Promega Corp., Madison, WI; the content of which is incorporated herein in its entirety) may be consulted to select an appropriate labeling protocol without undue experimentation.

It is further contemplated to be within the scope of the present invention to produce and use antibodies specifically reactive with an IRSBP-1 protein or a region thereof. The antibody may be monoclonal or polyclonal and may be produced by conventional methodology using the IRSBP-1 protein, or an immunologic fragment thereof, as an immunogen. For example, a mammal (i.e., a mouse, rabbit, horse, sheep, or goat) may be immunized with a IRSBP-1 protein of the present invention, an immunogenic fragment thereof, or an IRSBP-1 fusion protein or fragment thereof, using an immunization protocol conducive to producing antibodies reactive with the IRSBP-1 protein or a fragment thereof. Following completion of the immunization steps, antiserum reactive with the jointed protein may be collected and, if desired, polyclonal anti-IRSBP-1 antibodies isolated.

One embodiment of the present invention, therefore, is a fragment of an amino acid sequence of the rat IRSBP-1 protein of SEQ ID NOS: 3 or 11, or human IRSBP-1 protein (SEQ ID NO: 12) may be synthesized and used as an immunogen to produce an anti-IRSBP-1 polyclonal antibody. The sequence of the immunogenic peptide synthesized was: Acetylated Cys-Thr-Ser-Gln-Asn-Thr-Lys-Ser-Arg-Tyr-Ile-Pro-Asn-Gly-Lys-Leu (SEQ ID NO: 15) at amino acid positions 746-760 of the rat IRSBP-1 amino acid sequence SEQ ID NO: 11. The polyclonal raised against the peptide SEQ ID NO: 13 was specific for the rat IRSBP-1 protein and cross-reacted with the human IRSBP-1 protein.

Antibodies that specifically bind, for example, IRSBP-1 epitopes can also be used in immunohistochemical staining of tissue samples in order to evaluate the abundance and pattern of expression of IRSBP-1. Anti-IRSBP-1 antibodies can be used diagnostically in immuno-precipitation and immuno-blotting to detect and evaluate IRSBP-1 levels in tissue or bodily fluid as part of a clinical testing procedure. For instance, such measurements can be useful in predictive valuations of the onset or progression of diabetes or cell proliferation disorders. Likewise, the ability to monitor IRSBP-1 levels in an individual can allow determination of the efficacy of a given treatment regimen for an individual afflicted with such a disorder. The level of IRSBP-1 can be measured in cells isolated from bodily fluid, such as in samples of cerebral spinal fluid or blood, or can be measured in tissue, such as produced by biopsy. Diagnostic assays using anti-IRSBP-1 antibodies can include, for example, immunoassays designed to aid in early diagnosis of a diabetic, neoplastic or hyperplastic disorder, e.g. the presence of insulin-responsive negative cells in the sample, e.g. to detect cells in which a lesion of the *IRSBP-1* gene has occurred.

Another application of anti-IRSBP-1 antibodies is in the immunological screening of cDNA libraries constructed in expression vectors, such as λgt11, λgt18-23, λZAP, and λORF8. Messenger libraries of this type, having coding sequences inserted in the correct reading frame and orientation, can produce fusion proteins. For instance, λgt11 will produce fusion proteins whose amino termini consist of .beta.-galactosidase amino acid sequences and whose carboxy termini consist of a foreign polypeptide. Antigenic epitopes of IRSBP-1 can then be detected with antibodies, as, for example, reacting nitrocellulose filters lifted from infected plates with anti-IRSBP-1 antibodies. Phage, scored by this assay, can then be isolated from the infected plate. Thus, the presence of IRSBP-1 homologs can be detected and cloned from other human sources, i.e. to identified other closely homologous human isoforms, as well as to identify IRSBP-1 homologs in other mammals.

It is further contemplated to be within the scope of the present invention for an assay to detect natural serum antibodies specific for the IRSBP-1 protein. These antibodies may be induced as a result of the release of IRSBP-1 or fragments thereof, during the onset of deterioration and destruction of the cells of the islets of Langerhan. The detection of the antibodies will provide a diagnostic indication of the onset of diabetes, cancer and the progressive loss of pancreatic activity.

Moreover, the nucleotide sequence determined from the cloning of subject IRSBP-1 from a human or animal cell line will further allow for the generation of probes designed for use in identifying IRSBP-1 homologs in other animal cell-types, particularly cells associated with the onset and maintenance of diabetes and obesity, cancer or other transformed or immortalized cells, as well as IRSBP-1 homologs from other non-human mammals.

In addition the present invention contemplates nucleotide probes can be generated from a cloned nucleic acid sequence of the IRSBP-1 protein, which allow for histological screening of intact tissue and tissue samples for the presence of IRSBP-1 mRNA. Similar to the diagnostic uses of anti-IRSBP-1 antibodies, the use of probes directed to IRSBP-1 mRNA, or to genomic IRSBP-1 sequences, can be used for both predictive and therapeutic evaluation of allelic mutations which might be manifest in, for example, diabetes or other metabolic disorders directly or indirectly attributed to a failure of the cells to respond or over-respond to insulin as well as neoplastic or hyperplastic disorders such as, but not limited to, unwanted cell growth. Used in conjunction with anti-IRSBP-1 antibody immunoassays, the nucleotide probes can help facilitate the determination of the molecular basis for a disorder or ailment that may involve some abnormality associated with expression (or lack thereof) of an IRSBP-1 protein and perturbation of insulin regulation of a gene expression or activity. For instance, nucleic acid molecules complementary to an IRSBP-1 coding sequence can be used to determine if cells contain IRSBP-1 coding sequences using Southern hybridization analysis. Nucleic acid molecules can also be used to determine the level of expression of IRSBP-1 mRNA in cells using Northern analysis as discussed in Example 8.

For example, the present method provides a method for determining if a subject is at risk for a disorder characterized by unwanted insulin non-responsiveness or cell proliferation. In preferred embodiments, the subject method can be generally characterized as comprising: detecting, in a tissue of a subject (e.g. a human patient), the presence or absence of a genetic lesion characterized by at least one of (i) a mutation of a gene encoding IRSBP-1 or (ii) the mis-expression of the *IRSBP-1* gene. To illustrate, such genetic lesions can be detected by ascertaining the existence of at least one of (i) a deletion of one or more nucleotides from the *IRSBP-1* gene, (ii) an addition of one or more nucleotides to the *IRSBP-1* gene, (iii) a substitution of one or more nucleotides of the *IRSBP-1* gene, (iv) a gross chromosomal rearrangement of the *IRSBP-1* gene, (v) a gross alteration in the level of a messenger RNA transcript of the *IRSBP-1* gene, (vi) the presence of a non-wild type splicing pattern of a messenger RNA transcript of the *IRSBP-1* gene, and (vii) a non-wild type level of the IRSBP-1 protein. In one aspect of the invention there is provided a probe/primer comprising an oligonucleotide containing a region of nucleotide sequence which is capable of hybridizing to a sense or antisense sequence of the rat or human IRSBP-1 SEQ ID NOS: 5 - 8, or naturally occurring mutants thereof, or 5' or 3' flanking sequences or intronic sequences naturally associated with the *IRSBP-1* gene. The probe is exposed to nucleic acid of a tissue sample; and the hybridization of the probe to the sample nucleic acid is detected. In certain embodiments, detection of the lesion comprises utilizing the probe/primer in, for example, a polymerase chain reaction (PCR) (see, e.g., U.S. Patent Nos. 4,683,195 and 4,683,202), or, alternatively, in a ligation chain reaction (LCR) (see, e.g., *Landegran et al.* (1988) Science 241: 1077-1080; and *Nakazawa et al.* (1994) Proc. Natl. Acad. Sci. 91: 360-364), the later of which can be particularly useful for detecting even point mutations in the *IRSBP-1* gene and which are incorporated herein in their entirety. Alternatively, or additionally, the level of IRSBP-1 protein can be detected in an immunoassay.

The IRSBP-1 polypeptides of the invention can be used in therapeutic applications. Since IRSBP-1 increases the transcription of IGFBP-3, IRSBP-1 can be used to treat diseases (e.g., diabetes) associated with low levels of IGFBP-3. Further, many diseases are associated with an excess of circulating IGF-1 or IGF-II, for example, some cancers and type II diabetes. IRSBP-1 can be used in patients with low levels of IGFBP-3 or high levels of IGF. Introduction of the gene encoding IRSBP-1 (or a functional derivative) into cells using either retroviral vectors or liposomes results in increased production of IGFBP-3. Many methods of delivering expressible coding sequences to cells are known in the art. Methods for gene therapy are described in U.S. Patent No. 5,399,346, issued to *Anderson et al*. and U.S. Patent No. 5,766,899, issued to *Kuo et al*. describes methods for gene delivery into liver cells. The use of amphipathic compounds to deliver DNA is described in U.S. Patent No. 5,744,335 issued to *Wolf et al.* and which are incorporated herein in their entirety. Adenovirus vectors and adeno-associated virus vectors are useful in gene delivery *in vivo.*

Further, IRSBP-1 encoding sequences of the invention are useful in increasing production of recombinant IGFBP-3 for treatment of the aforementioned diseases, including GH deficiencies and complications caused by increased unbound IGF, can be accomplished by administration of recombinant IGFBP-3 (for example, produced in cell culture) via pharmaceutical compositions. Production of IGFBP-3 from recombinant cells can be increased by transfecting such cells with an IRSBP-1 encoding sequence either under the control of its own or a heterologous promoter.

IRSBP-1 polypeptides of the present invention are also useful in the treatment of growth hormone disorders, especially those where IGFBP-3 levels are below normal. IRSBP-1 is formulated into a pharmaceutical composition for parenteral administration, and a therapeutical dose is administered, with the result of raising IGFBP-3 and IRSBP-1 levels in the treated patient.

The presence of micro-satellite DNA downstream of the IRSBP-1 coding sequence is also further noted. Expression of the IRSBP-1 coding sequence is greater in the presence than absence of this micro-satellite DNA. Probes and/or primers for analysis of this region may allow the identification of genetic diseases associated with aberrant IRSBP-1 expression.

In a diagnostic embodiment of the present invention, therefore the nucleotide sequence of the isolated DNA molecule of the present invention may be used as a probe in nucleic acid hybridization assays for the detection of the *IRSBP-1* gene in various patient body fluids. The nucleotide sequence of the present invention may be used in any nucleic acid hybridization assay system known in the art, including, but not limited to, Southern blots (*Southern, E.M* (1975) J. Mol. Biol. 98: 508; Northern blots (*Thomas et al.* (1980) Proc. Natl. Acad. Sci. 77: 5201-05); Colony blots (*Grunstein et al,* (1975) Proc. Natl. Acad. Sci. 72: 3961-65. Alternatively, the isolated DNA molecule of the present invention can be used in a gene amplification detection procedure such as a polymerase chain reaction (*Erlich et al.* (1991) "Recent Advances in the Polymerase Chain Reaction" Science 252: 1643-51 or in restriction fragment length polymorphism (RFLP) diagnostic techniques, as described in *Watson et al.,* (2d ed. 1992), Recombinant DNA, Scientific American Books, 519-522, 545-547.

Specifically, for example, the DNA molecules of the invention can be used in prenatal or postnatal diagnosis of the human diseases associated with defects in response to variation in the level of insulin. A probe for the DNA encoding IRSBP-1 can be designed using the DNA molecule of the invention, and used to probe the DNA obtained from amniotic fluid or chorionic tissue and amplified by PCR, LCR or any other known amplification technique for the presence of the *IRSBP-1* gene or a variant thereof, as noted above. Similar procedures can be used in postnatal diagnostic work, as, for example, to diagnose the source of an IRSBP-1 deficiency in a person who is diabetic.

Another potentially useful application of the DNA molecule of the present invention is the possibility of increasing the amount of IRSBP-1 protein present in a mammal by gene transfer (so-called "gene therapy"). Of course, in most instances, this gene would be transferred into the animal host along with promoters, inducers, and the like (which are well known and recognized techniques in the field of genetic engineering, as noted supra) to allow the cell to initiate and continue production of the genetic product protein. The DNA molecule of the present invention can be transferred into the extra-chromosomal or genomic DNA of the host.

Another aspect of the invention relates to the use of the isolated nucleic acid in "antisense" therapy. An antisense construct of the present invention can be delivered, for example, as an expression plasmid which, when transcribed in the cell, produces RNA which is complementary to at least a unique portion of the cellular mRNA which encodes a IRSBP-1-protein, e.g. the rat or human IRSBP-1 nucleic acid sequences represented in SEQ ID NOS: 2, 5 - 8. Alternatively, the antisense construct can be an oligonucleotide probe which is generated *ex vivo* and which, when introduced into the cell causes inhibition of expression by hybridizing with the mRNA and/or genomic sequences encoding one of the subject IRSBP-1 proteins. Such oligonucleotide probes are preferably modified oligonucleotides that are resistant to endogenous nucleases, e.g. exonucleases and/or endonucleases, and are therefore stable *in vivo.* Exemplary nucleic acid molecules for use as antisense oligonucleotides are phosphoramidate, phosphorothioate and methylphosphonate analogs of DNA (see also U.S. Patent Nos. 5,176,996; 5,264,564; and 5,256,775). Additionally, general approaches to constructing oligomers useful in antisense therapy have been reviewed, for example, by *van der Krol et al.* (1988) Biotechniques 6: 958-976; and *Stein et al.* (1988) Cancer Res. 48: 2659-2668.

Accordingly, the modified oligomers of the invention are useful in therapeutic, diagnostic, and research contexts. Inhibition of cell proliferation may result, but this condition may be desirable where, for example, proliferation may lead to a pathological condition such as, but not limited to a blockage of a blood vessel after angioplasty, or proliferation of endothelial cells for angiogenesis in tumor formation. An increase in cell regulation may result, but this condition may be desirable where, for example, a deterioration or deficiency in the number of cells results in a pathological condition such as, but not limited to, a progressive decrease in neural cells, or muscular atrophy. In therapeutic applications, the oligomers are utilized in a manner appropriate for antisense therapy in general. For such therapy, the oligomers of the invention can be formulated for a variety of loads of administration, including systemic and topical or localized administration. Techniques and formulations generally may be found in Remmington's Pharmaceutical Sciences, Meade Publishing Co., Easton, Pa. For systemic administration, injection is preferred, including intramuscular, intravenous, intraperitoneal, and subcutaneous for injection, the oligomers of the invention can be formulated in liquid solutions, preferably in physiologically compatible buffers such as Hank's solution or Ringer's solution. In addition, the oligomers may be formulated in solid form and redissolved or suspended immediately prior to use. Lyophilized forms are also included.

Systemic administration can also be by transmucosal or transdermal means, or the compounds can be administered orally. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays or using suppositories. For oral administration, the oligomers are formulated into conventional oral administration forms such as capsules, tablets, and tonics. For topical administration, the oligomers of the invention are formulated into ointments, salves, gels, or creams as generally known in the art.

In addition to use in therapy, the oligomers of the invention may be used as diagnostic reagents to detect the presence or absence of the target DNA or RNA sequences to which they specifically bind. Such diagnostic tests are described in further detail below.

It is further contemplated to be within the scope of the present invention for TRSBP-1-expression vectors to be used as a part of a gene therapy protocol to reconstitute IRSBP-1 function in a cell in which IRSBP-1 is mis-expressed, or alternatively, to provide an antagonist of the naturally-occurring IRSBP-1 or an antisense construct. For instance, expression constructs of the subject IRSBP-1-proteins may be administered in any biologically effective carrier, e.g. any formulation or composition capable of effectively transfecting cells in vivo with a recombinant IRSBP-1-gene. Approaches include insertion of the subject gene in viral vectors including recombinant retroviruses, adenovirus, adeno-associated virus, and herpes simplex virus-1, or recombinant bacterial or eukaryotic plasmids. Viral vectors can be used to transfect cells directly; plasmid DNA can be delivered with the help of, for example, cationic liposomes (lipofectin) or derivatized (e.g. antibody conjugated), polylysine conjugates, gramacidin S, artificial viral envelopes or other such intracellular carriers, as well as direct injection of the gene construct or CaPO₄ precipitation carried out in vivo. It will be appreciated that because transduction of appropriate target cells represents the critical first step in gene therapy, choice of the particular gene delivery system will depend on such factors as the phenotype of the intended target and the route of administration, e.g. locally or systemically.

A preferred approach for *in vivo* introduction of nucleic acid encoding one of the subject proteins into a cell is by use of a viral vector containing nucleic acid, e.g. a cDNA, encoding the gene product. Infection of cells with a viral vector has the advantage that a large proportion of the targeted cells can receive the nucleic acid. Additionally, molecules encoded within the viral vector, e.g., by a cDNA contained in the viral vector, are expressed efficiently in cells that have taken up viral vector nucleic acid.

Retrovirus vectors and adeno-associated virus vectors are generally understood to be the recombinant gene delivery system of choice for the transfer of exogenous genes in vivo, particularly into humans. These vectors provide efficient delivery of genes into cells, and the transferred nucleic acids are stably integrated into the chromosomal DNA of the host. A major prerequisite for the use of retroviruses is to ensure the safety of their use, particularly with regard to the possibility of the spread of wild-type virus in the cell population. The development of specialized cell lines (termed "packaging cells") that produce only replication-defective retroviruses has increased the utility of retroviruses for gene therapy, and defective retroviruses are well characterized for use in gene transfer for gene therapy purposes (for a review see *Miller, A. D.* (1990) Blood 76: 271). Thus, recombinant retrovirus can be constructed in which part of the retroviral coding sequence (gag, pol, env) has been replaced by nucleic acid encoding an IRSBP-1 proteins, thereby rendering the retrovirus replication defective. The replication defective retrovirus is then packaged into virions that can be used to infect a target cell through the use of a helper virus by standard techniques. Protocols for producing recombinant retroviruses and for infecting cells *in vitro* or *in vivo* with such viruses can be found in Current Protocols in Molecular Biology, *Ausubel et al.* (1989) (eds.) Greene Publishing Associates, Sections 9.10-9.14 and other standard laboratory manuals. Examples of suitable retroviruses include pLJ, pZIP, pWE and pEM which are well known to those skilled in the art. Examples of suitable packaging virus lines for preparing both ecotropic and amphotropic retroviral systems include psiCrip, psiCre, psi2 and psiAm. Retroviruses have been used to introduce a variety of genes into many different cell types, including neural cells, epithelial cells, endothelial cells, lymphocytes, myoblasts, hepatocytes, bone marrow cells, *in vitro* and/or *in vivo* (see for example *Eglitis, et al.* (1985) Science 230: 1395-1398; *Danos & Mulligan* (1988) Proc. Natl. Acad. Sci. 85: 6460-6464; *Wilson et al.* (1988) Proc. Natl. Acad. Sci. 85: 3014-3018; *Armentano et al.* (1990) Proc. Natl. Acad. Sci. 87: 6141-6145; *Huber et al.* (1991) Proc. Natl. Acad. Sci. 88: 8039-8043; *Ferry et al.* (1991) Proc. Natl. Acad. Sci. 88: 8377-8381; *Chowdhury et al.* (1991) Science 254: 1802-1805; *van Beusechem et al.* (1992) Proc. Natl. Acad. Sci. 89: 7640-7644; *Kay et al.* (1992) Human Gene Therapy 3: 641-647; *Dai et al.* (1992) Proc. Natl. Acad. Sci. 89: 10892-10895; *Hwu et al.* (1993) J. Immunol. 150: 4104-4115; U.S. Patent No. 4,868,116; U.S. Patent No. 4,980,286; PCT Application WO 89/07136; PCT Application WO 89/02468; PCT Application WO 89/05345; and PCT Application WO 92/07573).

Furthermore, it has also been shown that it is possible to limit the infection spectrum of retroviruses and consequently of retroviral-based vectors, by modifying the viral packaging proteins on the surface of the viral particle (see, for example PCT publications WO93/25234, WO94/06920, and WO94/11524) For instance, strategies for the modification of the infection spectrum of retroviral vectors include: coupling antibodies specific for cell surface antigens to the viral env protein (*Roux et al.* (1989) Proc. Natl. Acad. Sci. 86: 9079-9083; *Julan et al.* (1992) J. Gen. Virol. 73: 3251-3255; and *Goud et al.* (1983) Virology 163: 251-254); or coupling cell surface ligands to the viral env proteins (*Neda et al.* (1991) J. Biol. Chem. 266: 14143-14146). Coupling can be in the form of the chemical cross-linking with a protein or other variety (e.g. lactose to convert the env protein to an asialoglycoprotein), as well as by generating fusion proteins (e.g. single-chain antibody/env fusion proteins). This technique, while useful to limit or otherwise direct the infection to certain tissue types, and can also be used to convert an ecotropic vector into an amphotropic vector. Moreover, use of retroviral gene delivery can be further enhanced by the use of tissue- or cell-specific transcriptional regulatory sequences that control expression of the IRSBP-1-gene of the retroviral vector.

Another viral gene delivery system useful in the present invention utilizes adenovirus-derived vectors. The genome of an adenovirus can be manipulated such that it encodes a gene product of interest, but is inactivated in terms of its ability to replicate in a normal lytic viral life cycle (see, for example, *Berkner et al.* (1988) BioTechniques 6: 616; *Rosenfeld et al.* (1991) Science 252: 43 1434; and *Rosenfeld et* al. (1992) Cell 68: 143-155). Suitable adenoviral vectors derived from the adenovirus strain Ad type 5 dl324 or other strains of adenovirus (e.g., Ad2, Ad3, Ad7 etc.) are well known to those skilled in the art. Recombinant adenoviruses can be advantageous in certain circumstances in that they are not capable of infecting nondividing cells and can be used to infect a wide variety of cell types, including airway epithelium (*Rosenfeld et al.* (1992) cited supra), endothelial cells (*Lemarchand et al.* (1992) Proc. Natl. Acad. Sci. 89: 6482-6486), hepatocytes (*Herz & Gerard* (1993) Proc. Natl. Acad. Sci. 90: 2812-2816) and muscle cells (*Quantin et al.* (1992) Proc. Natl. Acad. Sci. 89: 2581-2584). Furthermore, the virus particle is relatively stable and amenable to purification and concentration, and as above, can be modified so as to affect the spectrum of infectivity. Additionally, introduced adenoviral DNA (and foreign DNA contained therein) is not integrated into the genome of a host cell but remains episomal, thereby avoiding potential problems that can occur as a result of insertional mutagenesis in situations where introduced DNA becomes integrated into the host genome (e.g., retroviral DNA). Moreover, the carrying capacity of the adenoviral genome for foreign DNA is large (up to 8 kilobases) relative to other gene delivery vectors (*Berkner et al.* supra; *Haj-Ahmand & Graham* (1986) J. Virol. 57:267). Most replication-defective adenoviral vectors currently in use and therefore favored by the present invention are deleted for all or parts of the viral E1 and E3 genes but retain as much as 80% of the adenoviral genetic material (see, e.g., *Jones et al*. (1979) Cell 16:683; *Berkner et al.,* supra; and *Graham et al.* in Methods in Molecular Biology, *E. J. Murray,* (1991) Ed. (Humana, Clifton, N.J.) vol. 7. pp. 109-127). Expression of the inserted IRSBP-1-gene can be under control of, for example, the E1A promoter, the major late promoter (MLP) and associated leader sequences, the E3 promoter, or exogenously added promoter sequences.

Yet another viral vector system useful for delivery of, for example, the subject IRSBP-1-gene, is the adeno-associated virus (AAV). Adeno-associated virus is a naturally occurring defective virus that requires another virus, such as an adenovirus or a herpes virus, as a helper virus for efficient replication and a productive life cycle. (For a review see *Muzyczka et al.* (1992) Curr. Topics in Micro. and Immunol. 158:97-129). It is also one of the few viruses that may integrate its DNA into nondividing cells, and exhibits a high frequency of stable integration (see for example *Flotte et al.* (1992) Am. J. Respir. Cell. Mol. Biol. 7:349-356; *Samulski et al*. (1989) J. Virol. 63:3822-3828; and *McLaughlin et al.* (1989) J. Virol. 62:1963-1973). Vectors containing as little as 300 base pairs of AAV can be packaged and can integrate. Space for exogenous DNA is limited to about 4.5 kb. An AAV vector such as that described in *Tratschin et al.* (1985) Mol. Cell. Biol. 5:3251-3260 can be used to introduce DNA into cells. A variety of nucleic acids have been introduced into different cell types using AAV vectors (see for example *Hermonat et al.* (1984) Proc. Natl. Acad. Sci. 81:6466-6470; *Tratschin et al.* (1985) Mol. Cell. Biol. 4:2072-2081; *Wondisford et al.* (1988) Mol. Endocrinol. 2:32-39; *Tratschin et al.* (1984) J. Virol. 51:611-619; and *Flotte et al.* (1993) J. Biol. Chem. 268:3781-3790).

Other viral vector systems that may have application in gene therapy have been derived from such as, but not limited to, herpes virus, vaccinia virus, and several RNA viruses. In particular, herpes virus vectors may provide a unique strategy for persistence of the recombinant *IRSBP-1* gene in cells of the central nervous system.

In addition to viral transfer methods, such as those illustrated above, non-viral methods can also be employed to cause expression of an IRSBP-1-protein, or an IRSBP-1 antisense molecule, in the tissue of an animal. Most non-viral methods of gene transfer rely on normal mechanisms used by mammalian cells for the uptake and intracellular transport of macromolecules. In preferred embodiments, non-viral gene delivery systems of the present invention rely on endocytic pathways for the uptake of the subject *IRSBP-1* gene by the targeted cell. Exemplary gene delivery systems of this type include liposomal derived systems, poly-lysine conjugates, and artificial viral envelopes.

In a representative embodiment, a gene encoding one of the subject IRSBP-1 proteins can be entrapped in liposomes bearing positive charges on their surface (e.g., lipofectins) and (optionally) which are tagged with antibodies against cell surface antigens of the target tissue (*Mizuno et al.* (1992) NO Shinkei Geka 20:547-551; PCT publication WO91/06309; Japanese patent application 1047381; and European patent publication EP-A-43075). For example, lipofection of papilloma-virus infected epithelial cells can be carried out using liposomes tagged with monoclonal antibodies against, for example, squamous cells.

In similar fashion, the gene delivery system comprises an antibody or cell surface ligand that is cross-linked with a gene binding agent such as polylysine (see, for example, PCT publications WO93/04701, WO92/22635, WO92/20316, WO92/19749, and WO92/06180). For example, an *IRSBP-1* gene construct encoding an antagonistic form of the protein, e.g. a dominant negative mutant, can be used to transfect HPV-infected squamous cells in vivo using a soluble polynucleotide carrier comprising an HPV viral coat protein conjugated to a polycation, e.g. poly-lysine (see U.S. Patent No. 5,166,320). It will also be appreciated that effective delivery of the subject nucleic acid constructs via receptor-mediated endocytosis can be improved using agents adenovirus or fusogenic peptides of the influenza HA gene product can be used as part of the delivery system to induce efficient disruption of DNA-containing endosomes (*Mulligan et al.* (1993) Science 260-926; *Wagner et al.* (1992) Proc. Natl. Acad. Sci. 89:7934; and *Christiano et al.* (1993) Proc. Natl. Acad. Sci. 90:2122).

In clinical settings, the gene delivery systems can be introduced into a patient by any of a number of methods, each of which is familiar in the art. For instance, a pharmaceutical preparation of the gene delivery system can be introduced systemically, e.g. by intravenous injection, and specific transduction of the gene into the target cells relies predominantly on the specificity of transfection provided by the gene delivery vehicle, cell-type or tissue-type expression due to the transcriptional regulatory sequences controlling expression of the gene, or a combination thereof. In other embodiments, initial delivery of the recombinant gene is more limited with introduction into the animal being quite localized. For example, the gene delivery vehicle can be introduced by catheter (see U.S. Patent No. 5,328,470) or by stereotactic injection (e.g. *Chen et al.* (1994) Proc. Natl: Acad. Sci. 91: 3054-3057).
Moreover, the pharmaceutical preparation can consist essentially of the gene delivery system in an acceptable diluent, or can comprise a slow release matrix in which the gene delivery vehicle is imbedded. Alternatively, where the complete gene delivery system can be produced intact from recombinant cells, e.g. retroviral packages, the pharmaceutical preparation can comprise one or more cells which packages, the pharmaceutical preparation can comprise one or more cells which produce the gene delivery system. In the case of the latter, methods of introducing the viral packaging cells may be provided by, for example, rechargeable or biodegradable devices. The generation of such implants is generally known in the art. See, for example, Concise Encyclopedia of Medical & Dental Materials, ed. by David Williams (MIT Press: Cambridge, Mass., 1990); *Sabel et al.* U.S. Patent No. 4,883,666; *Aebischer et al.* U.S. Patent No. 4,892,538; *Aebischer et al.* U.S. Patent No. 5,106,627; *Lim* U.S. Patent No. 4,391,909; *Sefton* U.S. Patent No. 4,353,888; and *Aebischer et al.* (1991) Biomaterials 12:50-55).

Another aspect of the present invention concerns transgenic animals, such as, but not limited to animal models for diabetes, obesity, mood disorders, developmental and, proliferative diseases, that are comprised of cells (of that animal) which contain a transgene of the present invention and which preferably (though optionally) express the subject IRSBP-1 in one or more cells in the animal. In embodiments of the present invention, therefore, the expression of the transgene is restricted to specific subsets of cells, tissues or developmental stages utilizing, for example, cis-acting sequences that control expression in the desired pattern. In the present invention, such mosaic expression of the subject IRSBP-1 proteins can be essential for many forms of lineage analysis and can additionally provide a means to assess the effects of IRSBP-1 mutations or overexpression that might grossly alter development in small patches of tissue within an otherwise normal embryo. Toward this end, tissue-specific regulatory sequences and conditional regulatory sequences can be used to control expression of the transgene in certain spatial patterns. Moreover, temporal patterns of expression can be provided by, for example, conditional recombination systems or prokaryotic transcriptional regulatory sequences.

Genetic techniques that allow for the expression of transgenes can be regulated via site-specific genetic manipulation in vivo are well known to those skilled in the art. For instance, genetic systems are available which allow for the regulated expression of a recombinase that catalyzes the genetic recombination a target sequence. As used herein, the phrase "target sequence" refers to a nucleotide sequence that is genetically recombined by a recombinase. The target sequence is flanked by recombinase recognition sequences and is generally either excised or inverted in cells expressing recombinase activity. Recombinase catalyzed recombination events can be designed such that recombination of the target sequence results in either the activation or repression of expression of the subject receptor. For example, excision of a target sequence that interferes with the expression of the receptor can be designed to activate expression of that protein. This interference with expression of the subject protein can result from a variety of mechanisms, such as spatial separation of the *IRSBP-1* gene from the promoter element or an internal stop codon. Moreover, the transgene can be made wherein the coding sequence of the *IRSBP-1* gene is flanked by recombinase recognition sequences and is initially transfected into cells in a 3' to 5' orientation with respect to the promoter element. In such an instance, inversion of the target sequence will reorient the subject *IRSBP-1* gene by placing the 5' end of the coding sequence in an orientation with respect to the promoter element that allow for promoter driven transcriptional activation.

In an illustrative embodiment, either the cre/loxP recombinase system of bacteriophage P1 (*Lakso et al.* (1992) Proc. Natl. Acad. Sci. 89:6232-6236; *Orban et al.* (1992) Proc. Natl. Acad. Sci. 89:6861-6865) or the FLP recombinase system of Saccharomyces cerevisiae (*O'Gorman et al.* (1991) Science 251:1351-1355; PCT publication WO 92/15694), can be used to generate in vivo site-specific genetic recombination systems. Cre recombinase catalyzes the site-specific recombination of an intervening target sequence located between *loxP* sequences. *loxP* sequences are 34 base pair nucleotide repeat sequences to which the Cre recombinase binds and are required for Cre recombinase mediated genetic recombination. The orientation of *loxP* sequences determines whether the intervening target sequence is excised or inverted when Cre recombinase is present (*Abremski et al.* (1984) J. Biol. Chem. 259:1509-1514); catalyzing the excision of the target sequence when the *loxP* sequences are oriented as direct repeats and catalyzes inversion of the target sequence when *loxP* sequences are oriented as inverted repeats.

Accordingly, genetic recombination of the target sequence is dependent on expression of the Cre recombinase. Expression of the recombinase can be regulated by promoter elements which are subject to regulatory control, e.g., tissue-specific, developmental stage-specific, inducible or repressible by externally added agents. This regulated control will result in genetic recombination of the target sequence only in cells where recombinase expression is mediated by the promoter element. Thus, the activation of expression of the recombinant *UBC9* gene can be regulated via regulation of recombinase expression.

Use of the these recombinase system to regulate expression of, for example, a dominant negative *IRSBP-1* gene, or an antisense gene, requires the construction of a transgenic animal containing transgenes encoding both the Cre recombinase and the subject gene. Animals containing both the Cre recombinase and the *IRSBP-1* genes can be provided through the construction of "double" transgenic animals. A convenient method for providing such animals is to mate two transgenic animals each containing a transgene, e.g., one harboring the *IRSBP-1* gene, and the other harboring the recombinase gene.

One advantage derived from initially constructing transgenic animals containing a *IRSBP-1* transgene in a recombinase-mediated expressible format derives from the likelihood that the subject IRSBP-1 protein, whether antagonistic or agonistic, will be deleterious upon expression in the transgenic animal. In such an instance, a founder population, in which the subject transgene is silent in all tissues, can be propagated and maintained. Individuals of this founder population can be crossed with animals expressing the recombinase in, for example, one or more tissues, or in a developmentally restricted pattern. Thus, the creation of a founder population in which, for example, an antagonistic *IRSBP-1* transgene is silent will allow the study of progeny from that founder in which disruption of IRSBP-1-mediated insulin responsiveness in a particular tissue or at certain developmental stages could result in, for example, a lethal phenotype.

Similar conditional transgenes can be provided using prokaryotic promoter sequences which require prokaryotic proteins to be simultaneous expressed in order to facilitate expression of the transgene. Operators present in prokaryotic cells have been extensively characterized in vivo and in vitro and can be readily manipulated to place them in any position upstream from or within a gene by standard techniques. Such operators comprise promoter regions and regions which specifically bind proteins such as activators and repressors. One example is the operator region of the *lexA* gene of *E. coli* to which the LexA polypeptide binds. Other exemplary prokaryotic regulatory sequences and the corresponding trans-activating prokaryotic proteins are given in U.S. Patent No. 4,833,080. Thus, as described above for the recombinase-mediated activation, silent transgenic animals can be created which harbor the subject transgene under transcriptional control of a prokaryotic sequence that is not appreciably activated by eukaryotic proteins. Breeding of this transgenic animal with another animal that is transgenic for the corresponding prokaryotic trans-activator, can permit activation of the IRSBP-1 transgene. Moreover, expression of the conditional transgenes can be induced by gene therapy-like methods (such as described above) wherein a gene encoding the trans-activating protein, e.g. a recombinase or a prokaryotic protein, is delivered to the tissue and caused to be expressed, such as in a cell-type specific manner. By this method, the IRSBP-1 transgene could remain silent into adulthood until "turned on" by the introduction of the trans-activator.

Additionally, inducible promoters can be employed, such as the tet operator and the metallothionein promoter which can be induced by treatment with tetracycline and zinc ions, respectively (*Gossen et al.* (1992) Proc. Natl. Acad. Sci. 89:5547-5551; and *Walden et al.* (1987) Gene 61:317-327).

Methods of making knock-out or disruption transgenic animals are also generally known. See, for example, Manipulating the Mouse Embryo, (Cold Spring Harbor Laboratory Press, Cold Spring Harbor, N.Y., 1986). Recombinase dependent knockouts can also be generated, e.g. by homologous recombination to insert recombinase target sequences flanking portions of an endogenous *IRSBP-1* gene, such that tissue specific and/or temporal control of inactivation of an IRSBP-1 allele can be controlled as above. Furthermore, the present invention, by making available purified and recombinant forms of the subject IRSBP-1 proteins, will allow the development of assays which can be used to screen for drugs which either agonize or antagonize the function of IRSBP-1 *in vivo.*

Assays for the measurement of IRSBP-1 can be generated in many different forms, and include assays based on cell-free systems, e.g. purified proteins or cell lysates, as well as cell-based assays which utilize intact cells. Such agents can be used, for example, in the treatment of diabetic or feeding disorders, proliferative and/or differentiative disorders, and to modulate cellular metabolism.

In many drug screening programs which test libraries of compounds and natural extracts, high throughput assays are desirable in order to maximize the number of compounds surveyed in a given period of time. Assays which are performed in cell-free systems, such as may be derived with purified or semi-purified proteins or with lysates, are often preferred as "primary" screens in that they can be generated to permit rapid development and relatively easy detection of an alteration in a molecular target which is mediated by a test compound. Moreover, the effects of cellular toxicity and/or bioavailability of the test compound can be generally ignored in the in vitro system, the assay instead being focused primarily on the effect of the drug on the molecular target as may be manifest in an alteration of binding affinity with other proteins or change in enzymatic properties of the molecular target. Accordingly, potential inhibitors of IRSBP-1 function can be detected in a cell-free assay generated by constitution of a functional IRSBP-1/target nucleic acid sequence in a cell lysate.

Another aspect of the present invention concerns three-dimensional molecular models of the subject IRSBP-1 proteins, and their use as templates for the design of agents able to inhibit at least one biological activity of the IRSBP-1 protein. An integral step to designing inhibitors of the subject IRSBP-1 involves construction of computer graphics models of the IRSBP-1 that can be used to design pharmacophores by rational drug design. For instance, for an inhibitor to interact optimally with the subject protein, it will generally be desirable that it have a shape which is at least partly complimentary to that of a particular binding site of the protein, as for example those portions of the human IRSBP-1 that are involved in recognition of a particular region of a nucleic acid sequence. Additionally, other factors, including electrostatic interactions, hydrogen bonding, hydrophobic interactions, desolvation effects, and cooperative motions of ligand and enzyme, all influence the binding effect and should be taken into account in attempts to design bioactive inhibitors.

A computer-generated molecular model of the subject protein can be created by homology modeling, and then calculate the structure of the protein and velocities of each atom at a simulation temperature. Computer programs for performing energy minimization routines are commonly used to generate molecular models. For example, both the CHARMM (Brooks et al. (1983) J. Comput. Chem. 4:187-217) and AMBER (*Weiner et* al (1981) J. Comput. Chem. 106: 765) algorithms handle all of the molecular system setup, force field calculation, and analysis (see also, *Eisenfield et al.* (1991) Am. J. Physiol. 261:C376-386; *Lybrand* (1991) J Pharm. Belg. 46:49-54; *Froimowitz* (1990) Biotechniques 8:640-644; *Burbam et al.* (1990) Proteins 7:99-111; *Pedersen* (1985) Environ Health Perspect. 61:185-190; and *Kini et al.* (1991) J. Biomol. Struct. Dyn. 9:475-488).

Moreover, a number of programs are presently available for virtual design of IRSBP-1 protein inhibitors. For instance, the increasing availability of biomacromolecule structures of potential pharmacophoric molecules that have been solved crystallographically has prompted the development of a variety of direct computational methods for molecular design, in which the steric and electronic properties of substrate binding sites are used to guide the design of potential inhibitors (*Cohen et al.* (1990) J. Med. Cam. 33: 883-894; *Kuntz et al.* (1982) J. Mol. Biol. 161: 269-288; *Desjarlais* (1988) J. Med. Cam. 31: 722-729; *Bartlett et al.* (1989) Spec. Publ., Roy. Soc. Chem. 78: 182-196; *Goodford et al.* (1985) J. Med. Cam. 28: 849-857; *Desjarlais et al.* J. Med. Cam. 29: 2149-2153). Most algorithms of this type provide a method for finding a wide assortment of chemical structures that are complementary to the shape of a binding site of the subject protein. Each of a set of small molecules from a particular database, such as the Cambridge Crystallographic Data Bank (CCDB) (*Allen et al.* (1973) J. Chem. Doc. 13: 119), is individually docked to a nucleic acid or other ligand binding site of the IRSBP-1 protein in a number of geometrically permissible orientations with use of a docking algorithm. In an illustrative embodiment, a set of computer algorithms called DOCK, can be used to characterize the shape of invaginations and grooves that form the active sites and recognition surfaces of the subject protein (*Kuntz et al.* (1982) J. Mol. Biol. 161: 269-288). The program can also search a database of small molecules for templates whose shapes are complementary to particular binding sites of the protein (*Desjarlais et al.* (1988) J. Med. Chem. 31: 722-729). These templates normally require modification to achieve good chemical and electrostatic interactions (*Desjarlais et al.* (1989) ACS Symp. Ser. 413: 60-69). However, the program has been shown to position accurately known cofactors for inhibitors based on shape constraints alone.

Other exemplary virtual drug design programs include GRID (*Goodford* (1985, J. Med. Chem. 28:849-857); *Boobbyer et al.* (1989) J. Med. Chem. 32:1083-1094), CLIX *Lawrence et al.* (1992) Proteins 12:31-41), GROW (*Moon et al.* (1991) Proteins 11:314-328), the multiple copy simultaneous search method (MCSS) (described by *Miranker et al.* (1991) Proteins 11: 29-34), and NEWLEAD (*Tschinke et al.* (1993) J. Med. Chem. 36: 3863,3870).

### Example 1: Cloning of cDNAs containing an IRSBP coding sequence.

An isolated nucleic acid with the IRE associated with the IGFBP-3 gene and comprising the nucleotide sequence SEQ ID NO: 1 was multimerized as follows. Two antiparallel oligonucleotides, one representing the sense strand of SEQ ID NO: 1, and the other its antisense complement were annealed. The resulting double-stranded DNA was phosphorylated with T4 polynucleotide kinase, concatemerized with T4 DNA ligase at 22°C for 5 minutes, and electrophoretically fractionated on a polyacrylamide gel. A fragment containing three contiguous copies of the annealed oligonucleotides was inserted into the pHISi reporter vector (Clontech, Palo Alto, CA), and transformed into the yeast *Saccharomyces cerevisiae* strain YM4271 (Clontech, Palo Alto, CA).

Southern blotting confirmed the integration of the multimerized IRE nucleic acid sequence into the yeast genome. Southern blotting was carried out using prehybridization and hybridization buffers containing 1% w/v BSA, 1 mM EDTA. 0.5 M NaHPO₄, pH 7.2, and 7% w/v SDS. Hybridization was done with the radiolabeled multimerized IRE of IGFBP-3 with ³²P as the radiolabel. The radioactive probe was added at a concentration of 1-2 x 10⁶ cpm/ml. After hybridization, blots were washed twice with 2X SSC, 0.1 % SDS for 30 mins., followed by a 30 min. wash with 0.1X SSC, 0.1% SDS at 50°C, and autoradiography. The procedure was as described in *Ausubel et al.* (1993).

After confirming the integration of the IGFBP-3 IRE nucleic acid sequence (SEQ ID NO: 1) into the yeast genome, a rat liver cDNA library was screened using a yeast one-hybrid system. The yeast one-hybrid system is an *in vivo* genetic assay that uses growth selection based on reconstruction and activation of the nutritional reporter gene *HIS3.* To this aim, a nucleic acid fragment comprising three contiguous repeats of the IGFBP-3 IRE (SEQ ID NO: 1) was inserted in the region 5' upstream of a *HIS3* reporter under the control of a *GAL4*-responsive promoter. The construct was transformed into yeast cells.

The one-hybrid screening procedure used herein is described by *Chong et al.,* (1995) Cell 80: 949-957 and *Li & Herskowitz,* (1993) Science 1252: 1870-1873. To search for genes encoding insulin-responsive binding proteins, yeast containing the IRE target nucleic acid sequence (SEQ ID NO: 1) were transformed with DNA purified from an activation domain (AD) library that contained fusions between a target-independent activation domain (GAL4 AD) and cDNA derived from a normal rat liver. Colonies of yeast were selected on His⁻Leu⁻ plates and the plasmids were isolated from the yeast. Positive clones were confirmed by retransformation of the cDNA into yeast containing a *Lac* Z reporter gene with tandem repeats of the IRE target nucleic acid, and tested for transcriptional activation of the GAL4 promoter.

Seventy-nine clones were found which grew on His⁻Leu⁻ plates containing 15 mM 3-AT (3-amino-1,2,4-triazole, Sigma Chemical Co., St. Louis, MO). The plasmids from those clones were isolated as described by *Hoffman & Winsten,* (1987) Gene 57: 267-272 and transformed into *E. coli* according to *Kaiser & Auser* (1993) Biotechniques 14: 552. The isolated cDNAs were sequenced using automated sequencing. The sequences were identified by an NCBI BLAST search for similarity to sequences reported in GenBank.

Sixty-six out of the seventy-nine clones were identified as secretory or structural proteins, membrane proteins, or enzymes. Two of the clones contained cDNAs encoding the known transcription factors NFkB p65 and HBP1. Eleven out of seventy-nine clones contained novel sequences. *Gel shift mobility assays.*

cDNAs from the 11 novel clones were subcloned into the plasmid pSPUTK (Stratagene). The coding regions of the cDNA clones were translated into protein using coupled transcriptional *in vitro* translation as described by *Hook et al.* (1996) Peptide Research 9: 183-187. After translation, the proteins were tested for their ability to bind to the IRE element of IGFBP-3 (SEQ ID NO: 1) by gel mobility shift analysis.

Gel mobility shift assays were done essentially as described in *Villafuerte et al,* (1997) J. Biol. Chem. 272: 5024-5030. ³²P-ATP-labeled oligonucleotides corresponding to nucleotide positions -1150 to -1117 bp-fragment of the rat IGFBP-3 (SEQ ID NO: 1) gene were incubated with the proteins derived from the cDNA clones at concentrations of approximately 20 ng protein per lane in 25 µl of binding buffer containing 10 mM Tris, pH 7.6, 50 mM KCI, 1mM EDTA, 0.5 mM dithiothreitol, 0.2% Nonidet P-40, 20 µg of bovine serum albumin, 36 µg of salmon sperm DNA, and 10% glycerol at 25°C for 20 mins. Incubations were carried out with or without unlabeled competitor DNA. Protein-DNA complexes were separated from free probe on 6% polyacrylamide gels in 0.25.x TBE at 12 V/cm for 2-3 hours, and visualized by autoradiography.

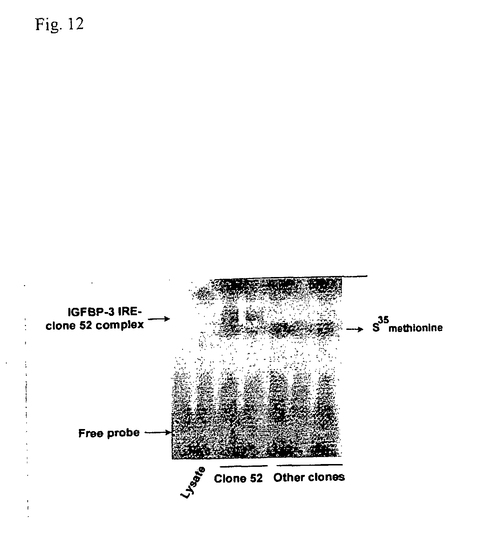

One clone of the eleven, clone 52 (SEQ ID NO: 2, shown in Fig. 1) contained a 952 bp cDNA insert that encoded a polypeptide (SEQ ID NO: 3, shown in Fig 2) that formed a DNA-protein complex. No other clones contained cDNA sequences that after translation with reticulocyte lysate, could produce the gel shift in the gel mobility shift experiments as shown in Fig. 12. The clone 52 nucleic acid sequence (SEQ ID NO: 2) comprises 952 bp of sequence capable of hybridizing to a second IRSBP-1 nucleic acid sequence (SEQ ID NO: 5 shown in Figs. 4A, B) and to a region of the human genomic sequence having the accession number AC005237.

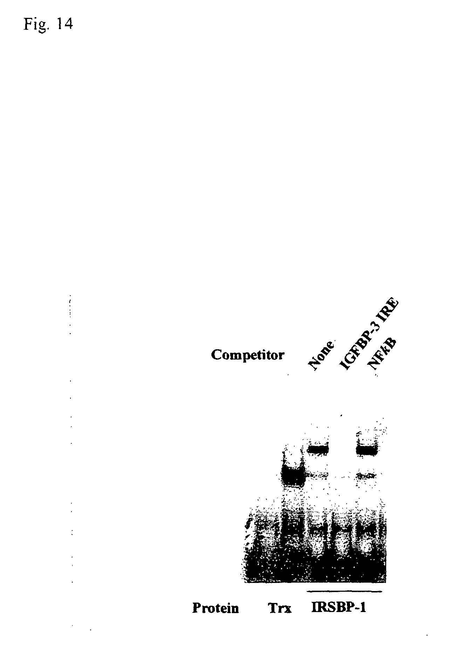

The specificity of binding was established by using labeled or unlabeled (25 pmoles) IGFBP-3 IRE (SEQ ID NO: 1) as shown in Fig. 14. Excesses of unlabeled IGFBP-3 IRE and NFκB (Stratagene,La Jolla, CA) were incubated with about 20 ng protein per lane. The double-stranded DNA competitors were added at molar concentrations between about 10-fold and about 100-fold greater than labeled oligonucleotides, or 2.5 pmole, 12.5 pmole and 25 pmole, and electrophoresed on a 6% polyacrylamide gel.

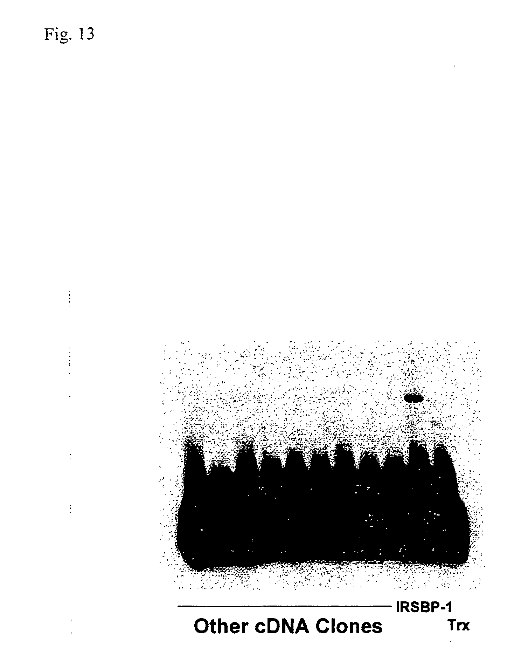

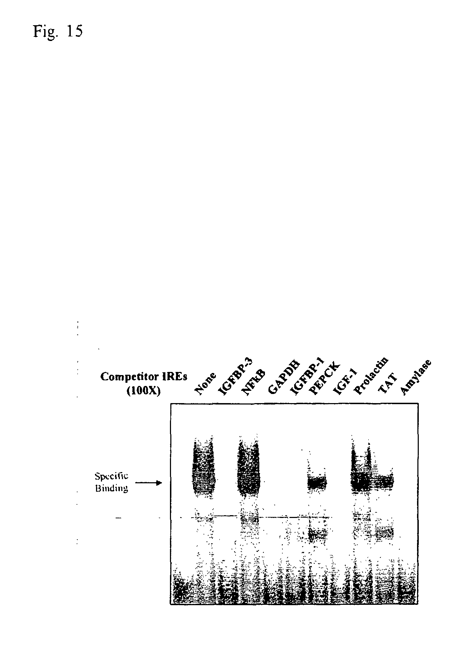

cDNA coding sequences were also expressed as thioredoxin (Trx) fusion proteins in *E. coli.* The clone 52 cDNA (SEQ ID NO: 2) was subcloned in-frame into a prokaryotic expression vector (pET-32a from Novagen, Madison, WI), transformed into the AD494(DE3) strain of *E. coli,* and grown in culture until OD₆₀₀0.6. IPTG (isopropyl-β-thiogalactopyranoside) was added to a final concentration of 1 mM three hours before harvest. The thioredoxin-clone 52 fusion protein was purified by affinity chromatography on immobilized His-bound metal chelation resin (Novagen), and used in a gel-shift assays, as shown in Figs. 13 and 14. The fusion proteins were tested in additional gel mobility shift experiments with the IGFBP-3 IRE nucleic acid (SEQ ID NO: 1) as describe above. While not wishing to be bound by any particular theory, since the IRE of IGFBP-3 (SEQ ID NO: 1) includes an AGGAAAGTCTCCTT palindrome, and the leucine zipper encourages dimerization, gel shift bands seen in Figs. 12-14 reflect binding of IRSBP-1 to the IGFBP-3 IRE as a homodimer and monomer, respectively. Competiton assays demonstrate that IREs associated with other insulin-responsive genes compete with the IRE of IGFBP-3 (SEQ ID NO: 1) for binding to IRSBP-1, as shown in Fig. 15.

### Example 2: Sequencing of cDNA clones

Single-strand sequencing of cDNA clones was with an Applied Biosystems Automated DNA Sequencer (Applied Biosystems,, Foster City, CA) and a PCR-based fluorescent dideoxy method, according to the recommendations of the manufacturer. The partial rat IRSBP-1 cDNA clone 52 (SEQ ID NO: 2) is shown in Fig. 1. The longest open-reading frame amino acid sequence derived from SEQ ID NO: 2 is SEQ ID NO: 3, as shown in Fig.2.

### Example 3: Expression of clone 52 mRNAs using Northern blot analysis

Total RNA was isolated from cultured hepatic non-parenchymal cells using a Tri-Reagent Kit (Molecular Research Center, Cincinnati, OH) according to the manufacturer's protocol. Clone 52 cDNA (SEQ ID NO: 2) containing the 952 bp cDNA nucleic acid obtained from the yeast one-hybrid screen as described in Example 1, was random primer labeled with [³²P] dCTP and used as a probe to hybridize with RNA electrophoresed on 1.2% formaldehyde-agarose gel using protocols described by *Ausubel et al.* (1993).

Northern analysis showed that hepatic cells expressed an mRNA species of at least 3.5 kb in length.

### Example 4: Interactions with other insulin-response binding proteins

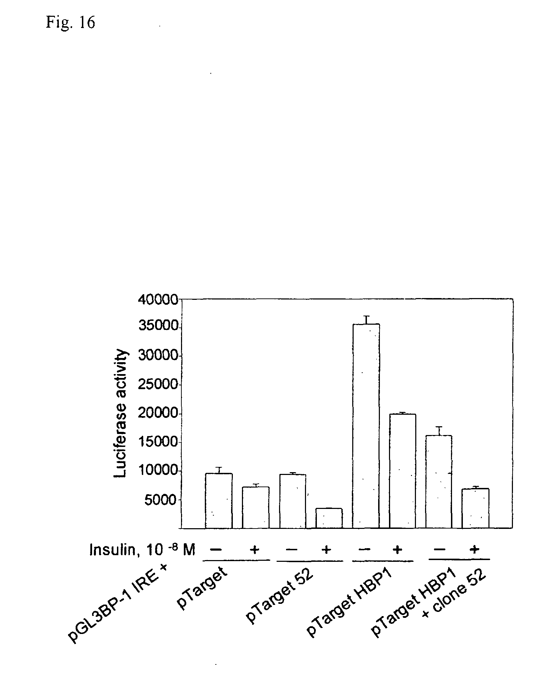

CHO cells were co-transfected with IGFBP-1 IRE reporter or PEPCK reporter constructs and combinations of vectors containing an IRSBP-1 cDNA (SEQ ID NO: 2) or DNA encoding for HBP1. Cells were prepared and transfected as described in Example 6. Luciferase activity was measured as described in Example 6. The IREs identified from the IGFBP-1 and PEPCK genes are described in *Cichy et al.* (1998) J. Biol. Chem. 273: 6483-6487; *O'Brien et al.* (1994) J. Biol. Chem. 269: 30419-30428. HBP-1 induced the formation of luciferase in the absence of IRSBP-1 or insulin, (as shown in Fig. 16). Cotransfection with the IRSBP-1-encoding cDNA reduced the activation of the IGFBP-1 IRE and decreased the luciferase expression. Induction of cellular IRSBP-1 by insulin reduced luciferase levels still further.

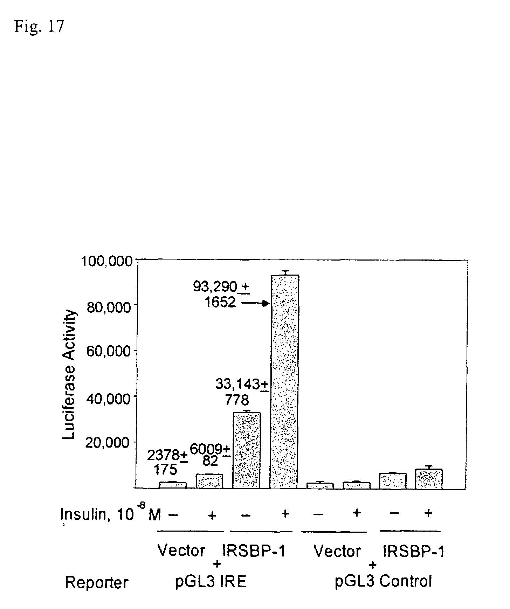

The IREs were used to construct multimers as described in Example 1, inserted into the pGL3 promoter vector (Promega., Madison, WI) and transfected into CHO cells. Such cells showed an increase in luciferase reporter activity augmented by the addition of insulin to the media, as shown in Fig. 17.

### Example 5: Screening of bacteriophage human and rat cDNA libraries

Because the 952 bp IRSBP-1-encoding clone 52 cDNA (SEQ ID NO: 2) encodes a protein (SEQ ID NO: 3) that contains a binding domain with limited transactivating properties, at least one longer cDNA sequence (SEQ ID NOS: 5 and 6) were obtained by screening a lambda bacteriophage rat brain cDNA library (Uni-Zap XP library, Stratagene, La Jolla, CA) and then extending the cDNAs thus obtained by 5' RACE.

The 952 bp clone 52 cDNA of SEQ ID NO: 2 was used as the nucleic acid probe to screen about 10⁸ plaques. The phage and host *E. coli* were spread on agarose plates and incubated to form plaques, nitrocellulose filters were applied, and the phage particles and unpackaged DNA were adsorbed to the filter to produce a replica of the plate surface. The filters were treated with NaOH to denature the phage DNA, which was then hybridized with the cDNA probe. After isolation of positive plaques that hybridized to the probe, the pBluescript phagemid was rescued with VCSM12 helper phage. The final product is a double-stranded pBluescript phagemid with an inserted DNA. Subsequent rescreening of the library combined with 5' RACE extensions yielded isolated nucleic acids comprising the nucleotide sequence of SEQ ID NO: 5, as shown in Figs. 4A and 4B, or fragments, variants or derivatives thereof, such as SEQ ID NO: 6. Automated sequencing confirmed regions within SEQ ID NOS: 5 and 6 having substantially similarity to the nucleotide sequence of clone 52 (SEQ ID NO: 2).

To obtain an isolated nucleic acid encoding a region of the human IRSBP-1, a 3404 bp rat cDNA (SEQ ID NO: 14) was used to screen a human lambda phage cDNA library (Uni-Zap XR human liver cDNA library, Stratagene, La Jolla, CA). Two clones hybridizing to the 3404 bp rat IRSBP-1-specific probe were obtained. One was about 2480 bp long and another clone was about 1700 bp long. Subsequent 5' extensions were obtained by 5' RACE techniques, using the SMART RACE cDNA amplification system from Clontech, Inc, and polyA mRNA isolated from the human cerebellum, until the 4584 bp sequence (SEQ ID NO: 7, shown in Fig 6) was obtained. Comparison of the human IRSBP-1 nucleic acid sequence (SEQ ID NO: 7) the rat IRSBP-1 sequence (SEQ ID NO: 5) showed at least 75% similarity when aligned.

Comparison of the human IRSBP-1 nucleic acid sequence (SEQ ID NO: 7) with the human genomic DNA sequence Accession No. AC005237 showed that the human cDNA sequence SEQ ID NO: 7 was derived from at least 26 exons (SEQ ID NOS: 16 - 41, as shown in Table 1).

**Table 1.**

| Exon positions of the human IRSBP-1 nucleic acid sequence SEQ ID NO: 7 | | |
|---|---|---|
| Nucleotide Positions of Human eDNA SEQ ID NO: 7 | Position in Genomic Sequence Accession No. AC005237 | SEQ ID NO: |
| 1 - 62 | 8096 - 8157 | 16 |
| 63 -177 | 8268 - 8382 | 17 |
| 178 - 302 | 16284 - 16409 | 18 |
| 303 - 407 | 16631 - 16735 | 19 |
| 408 - 521 | 16991 - 17104 | 20 |
| 522 - 638 | 17786 - 17902 | 21 |
| 639 - 755 | 19713 - 19829 | 22 |
| 756 - 873 | 20009 - 20126 | 23 |
| 874 - 986 | 20324 - 20437 | 24 |
| 987-1160 | 21118 - 21295 | 25 |
| 1161 - 1274 | 30759 - 30873 | 26 |
| 1275 - 1388 | 31556 - 31669 | 27 |
| 1389 - 1502 | 31981 - 32094 | 28 |
| 1503 - 1616 | 32811 - 32925 | 29 |
| 1617-1913 | 33264 - 33563 | 30 |
| 1914 - 2097 | 35731 - 35914 | 31 |
| 2098 - 2210 | 36876 - 36991 | 32 |
| 2211 - 2492 | 37887 - 38168 | 33 |
| 2493 - 2637 | 39543 - 39687 | 34 |
| 2638 - 2720 | 39763 - 39845 | 35 |
| 2721 - 2819 | 41233 - 41331 | 36 |
| 2820 - 2936 | 49644 - 49760 | 37 |
| 2937 - 3025 | 50244 - 50331 | 38 |
| 3026 - 3108 | 55359 - 55442 | 39 |
| 3109 - 3146 | 56332 - 56369 | 40 |
| 3147 - 4582 | 59605 - 61040 | 41 |

### Example 6: Metabolic Activity of the IRSBP-1 Protein

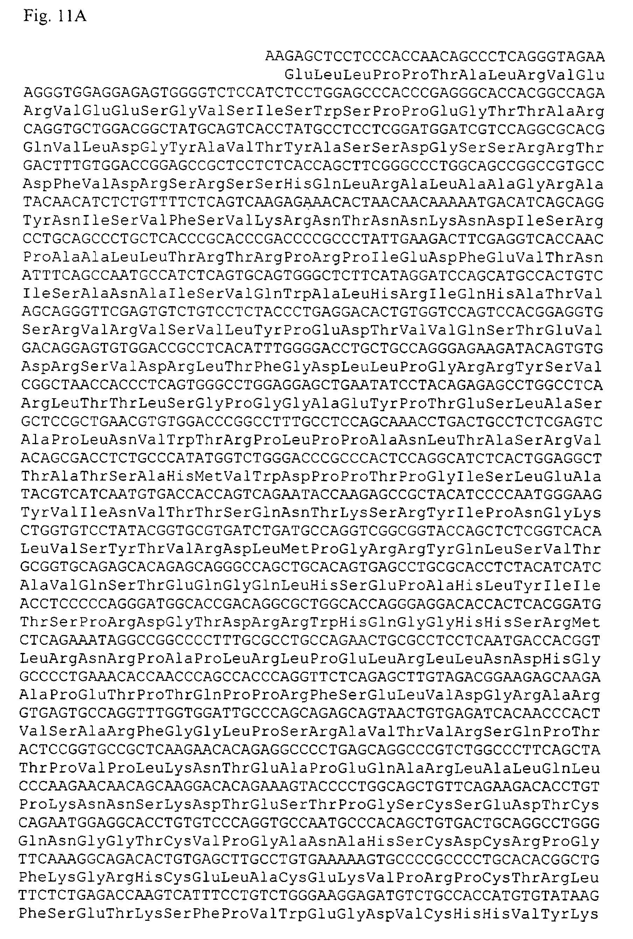

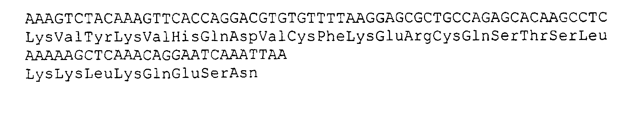

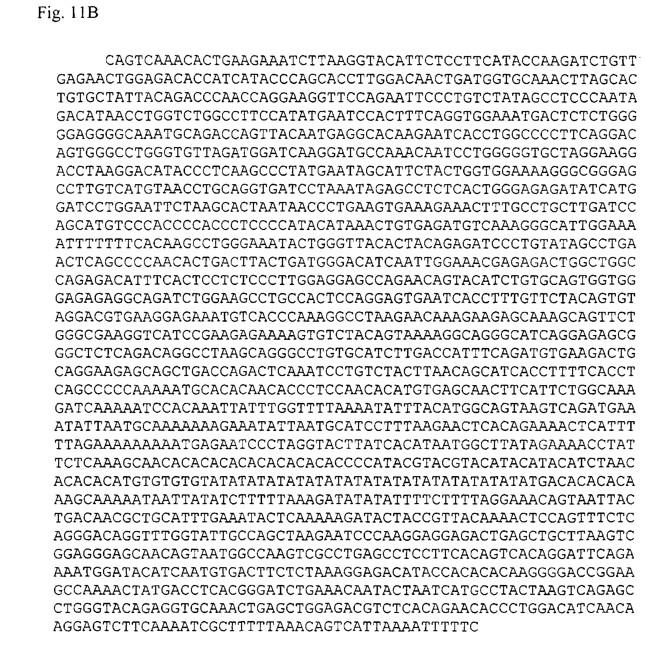

To investigate the biological effects of IRSBP-1, the partial cDNA sequence encoding IRSBP-1 (SEQ ID NO: 14 shown in Fig. 11) was subcloned into the pCMV-Tag epitope tagging mammalian expression vector (Stratagene, La Jolla, CA) and transfected into L6 myoblasts to establish IRSBP-1-stably transfected cell lines. G418-resistant clones were isolated and tested for [³H] 2-deoxyglucose uptake (Fig. 17).

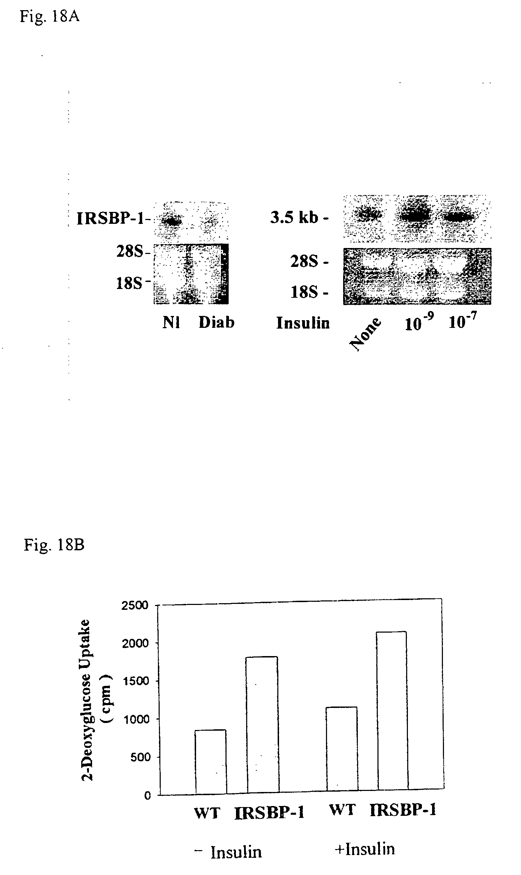

The insulin induction of the expression of the *IRSBP-1* gene was shown by Northern analysis wherein from about 10⁻⁹ M to about 10⁻⁷ M insulin increased the formation of IRSBP-1-specific mRNA of about 3.5 kb, as shown in Fig. 18A. Normal liver parenchymal cells have elevated levels of the IRSBP1-specific mRNA compared to cells from a rat having diabetes mellitus, also as shown in Fig. 18A.

In addition, the 3.4 kb cDNA region (SEQ ID NO: 14 as shown in Fig. 11) and derived from SEQ ID NO: 5 and obtained from the lambda bacteriophage cDNA library screening, was also tested for the ability to induce a luciferase reporter gene expressed under the regulatory control of the IGFBP-3 IRE (Fig. 18B). cDNAs found to produce proteins that bound to IRE sequences were subcloned into plasmid pTARGET, a mammalian expression vector containing Kozak initiation sequences (Promega, Madison, WI). cDNAs subcloned into pTARGET and a chimeric construct containing the IGFBP-3 promoter region (SEQ ID NO: 1) attached to the firefly luciferase reporter gene (pGL2-Basic, Promega) were co-transfected into chinese hamster ovary (CHO) cells. The chimeric construct contains three tandem copies of the IRE region of the IGFBP-3 promoter sequence (SEQ ID NO: 1) attached to the pGL3 promoter vector (Promega, Madison, WI). The cloning of the IGFBP-3 promoter region into pGL2-Basic was as described in *Villafuerte et al.,* (1997) J. Biol. Chem. 272: 5024-5030.

Transient transfections of the IGFBP-3 IRE-luciferase construct together with the cDNA clones in pTARGET were undertaken with CHO cells when the cells reached 60-70% confluence. Lipofectin (Life Technologies, Rockvillc, MD) and DNA complexes were mixed at a 15 µg to 2.5 µg ratio and incubated with the cells overnight. Medium was replaced with serum-free DMEM medium, with or without the addition of 10⁻⁶M human recombinant insulin (Life Technologies, Rockville, MD) for 24 hours, and cell extracts were assayed for gene activity using a luciferase assay system (Promega, Madison WI, and following the manufacturer's recommended protocol) and measured using a Microsure 100 luminometer (LKB Wallac, Turku, Finland). All readings were within the linear range of the instrument when compared with known luciferase concentrations.

The 3.4 kb cDNA region (SEQ ID NO: 14) increased IGFBP-3 IRE-induced reporter activity 4-fold, and addition of 10⁻⁶ M insulin increased the activity further by 12-fold (Fig. 17). There was a 110% increase in glucose uptake in cells stably transfected with nucleic acid SEQ ID NO 14, as compared to wild type cells as shown in Fig. 18. With the addition of 10⁻⁶ M insulin, there was a 30% further increase in glucose uptake in wild type cells and a 16% further increase in clone 52-transfected cells. These experiments indicate that IRSBP-1 is functionally insulin-mimetic.

### Example 7: Determination of IRSBP-1 biological activity

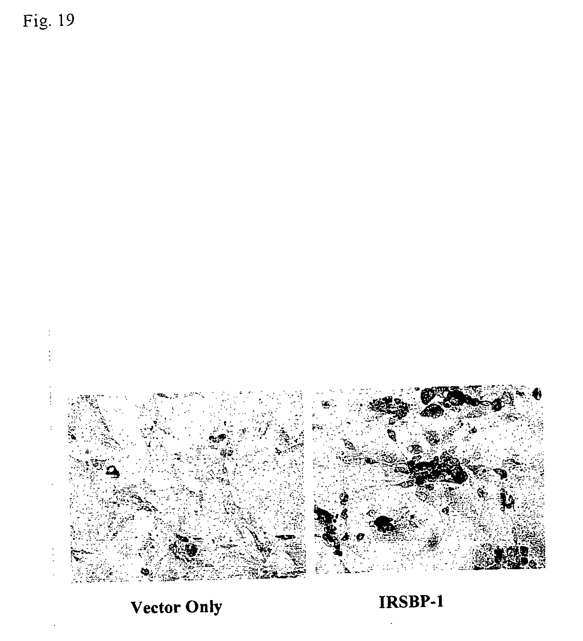

To examine the ability of clone 52 to allow storage of substrates related to insulin action, wild type L6 myoblasts and the clone 52-stably transfected cells were stained with the periodic acid-Schiff base stain specific for glycogen. As shown in Fig 19, the IRSBP-1-stably transfected cell line showed intense periodic acid Schiff base (+) materials in the cytoplasm, consistent with glycogen accumulation in the clone 52-transfected cells. Thus, clone 52 mediates storage of ingested substrates, and it can act as an insulin substitute at the target organ level.

### Example 8 : IRSBP-1 expression correlates to tissue-specific glucose utilization

*In vivo* disposition of glucose in various organs and tissues was studied. Following oral glucose load, tracer and forearm catheterization techniques showed that glucose is taken up by splanchnic tissues, including liver and gut (29%), muscle (26%), brain (23%), kidney (7%). heart (4%), fat (3%) and others (8%).

The RNase protection assay used a Kpn1-XhoI fragment (SEQ ID NO: 4, shown in Fig. 3) from clone 52 cDNA (SEQ ID NO: 1) inserted in pGem7Z and transcribed *in vitro* to give a riboprobe (antisense probe). The assay was carried out using the Hybspeed RPA Kit (Ambion, Inc., Austin, TX) according to the manufacturer's directions. Briefly, RNA extracted from various tissues of the rat were hybridized with the [³²P] UTP-labeled clone 52 probe-derived RNA at 45°C in the presence of 40 mM PIPES and 80% formamide. The unhybridized probe was degraded with RNase A and T, and protected RNA was purified and resolved on a sequencing gel.

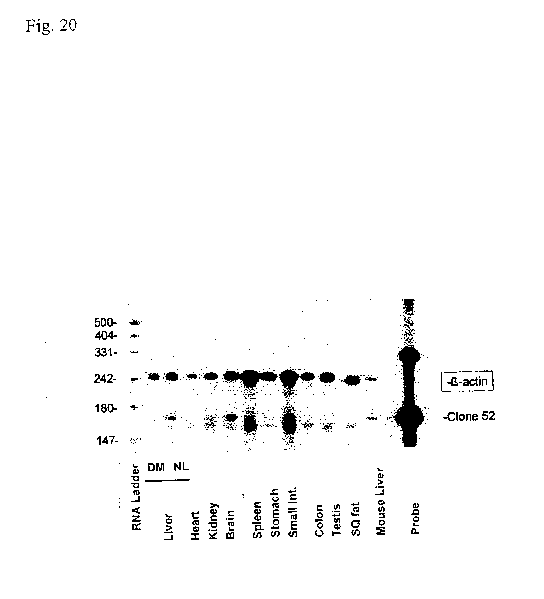

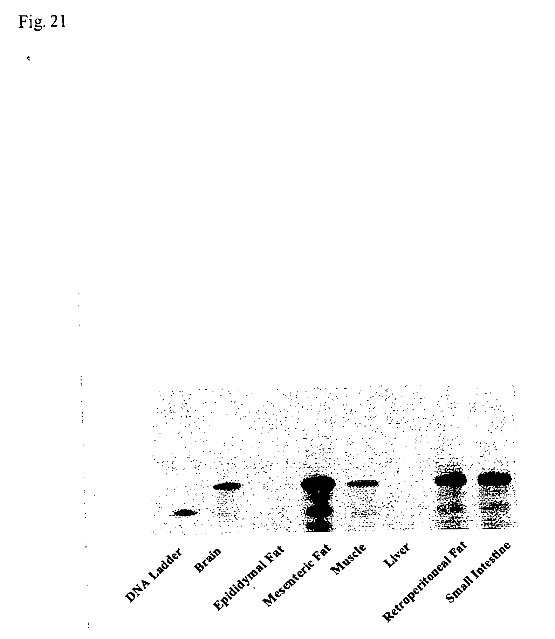

Using an ribonuclease protection assay, both insulin-dependent (muscles, adipose tissue, liver) and non-insulin dependent tissues or organs in which insulin were shown not to be required for utilization of glucose (brain, kidney, gut) expressed IRSBP-1 mRNA (Figs. 20 and 21). Thus, the common denominator for IRSBP-1 expression is the dependence of the organ or tissues on glucose for energy utilization.

At the tissue level, studies have shown that when comparing the glucose metabolic rates of different adipose regions, measured as the sum of glucose converted to CO₂, triglycerides and lactate, the mesenteric fat cells metabolized significantly more glucose per cell than other fat depots. The hierarchy of the glucose metabolic rate in the different adipose depot is as follows: mesenteric > retroperitoneal > epididymal > subcutaneous fat. As shown in Figs. 20 and 21, IRSBP-1 expression is highest in mesenteric fat, followed by retroperitoneal, epididymal and subcutaneous fat. Thus, the mRNA abundance of IRSBP-1 in adipose tissues reflects the glucose utilization rates in those tissues. Since diabetes is characterized by resistance to insulin action on glucose uptake and utilization in adipocytes and skeletal muscle, the studies illustrated in Figs. 18 - 21 indicate that over-expression of IRSBP-1 may overcome the problems of both poor glucose uptake and poor glucose utilization.

### Example 9: IRSBP-1 and its role in the onset and maintenance of obesity

IRSBP-1 could also be used for the treatment of obesity and complications associated with obesity. The organ systems and the specific diseases associated with obesity include the following: (1) cardiovascular system - hypertension, congestive heart failure, cor pulmonale, varicose veins, pulmonary embolism, coronary heart disease; (2) Endocrine-insulin resistance, glucose intolerance, type II diabetes mellitus, dyslipidemia, polycystic ovary syndrome, infertility, amenorrhea; (3) Musculoskeletal - immobility, degenerative arthritis, low back pain; (4) Integument - venous stasis of legs, cellulitis, intertrigo, carbuncles; (5) Respiratory system -dyspnea and fatigue, obstructive sleep apnea, hypoventilation (pickwickian) syndrome; (6) Gastrointestinal - gastroesophageal reflux disease, hepatic steatosis, nonalcoholic steatohepatitis, cholelithiasis, hernias, colon cancer; (7) Psychosocial - work disability, depression; (8) Genitourinary - urinary stress incontinence, hypogonadism, breast and uterine cancer; (9) Neurologic - stroke, meralgia paresthetica, idiopathic interacranial hypertension. Any of the above conditions, when associated with obesity, could be used as indications for the effective use of IRSBP-1 agonists or antagonists.

Using *in-situ* hybridization to localize IRSBP-1 mRNA in the brain, IRSBP-1 expression was detected in the areas of the brain known to be involved in ingestive, autonomic and neuroendocrine functions of feeding and satiety, as described in the Examples 10 - 12 below. Regulation of body weight requires a balance among energy intake, expenditure, and storage. The brain appears to define the set point around which body weight is regulated. The levels of IRSBP-1 mRNA in the lateral hypothalamus and the nucleus of the solitary tract are differentially regulated in obese as compared to lean Zucker rats, showing a significant interactive role of IRSBP-1 in modulating body weight.

### Example 10: IRSBP-1 expression is detected in many areas of the brain, including areas associated with feedings and satiety.

### In-situ hybridization of rat brains using ³⁵S-labeled IRSBP-1 riboprobe.

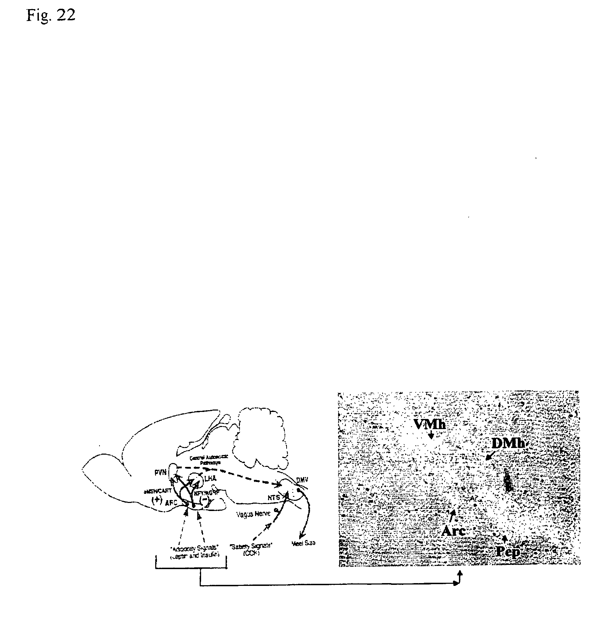

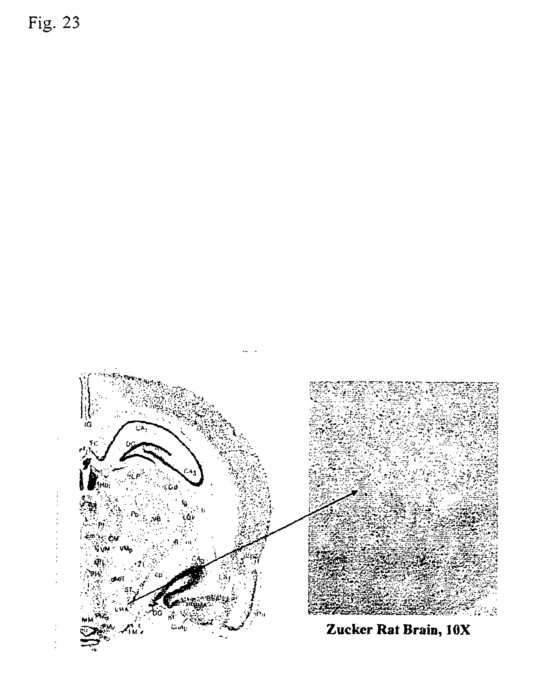

Rat brains were obtained and fixed by immersion in 4% paraformaldehyde in 0.1 M NaPO₄, sectioned on a cryostat to 5-10 µM thickness, and mounted on slides. Sense and antisense ³⁵S-labeled IRSBP-1 riboprobes were generated by *in vitro* transcription with ³⁵S-UTP, and derived from the linearized fragment SEQ ID NO: 4 of IRSBP-1 cDNA. Following proteinase K treatment, prehybridization of the various brain sections was performed for 3 hrs at 42° C, in a buffer containing 10 mM DTT, 0.3 M NaCl, 20 mM Tris pH8, 5 mM EDTA, 1x Denhardt's, 10% Dextran sulfate, and 50% formamide. This was followed by addition of ^{3S}S-labeled probe (600,000 cpm/slide) and tRNA (200 µg/ml) for hybridization. Hybridization was done overnight at 55° C; slides were then washed, treated with RNAse A, dehydrated, and coated with photographic emulsion. The slides were exposed and developed after 4-12 weeks. Both sagittal (Fig. 22) and coronal (Fig. 23) sections of brains from normal Sprague-Dawley rats, obese *fa*/*fa* Zucker rats and lean *fa*/+ Zucker rats were compared.

### Example 11: IRSBP-1 is expressed in the hypothalamus and nucleus of the solitary tract

Information about the qualities of food are relayed by the primary senses of smell, sight, and taste to the nucleus of the solitary tract in the medulla. The nucleus of the solitary tract (NTS) integrates afferent and efferent information and connects with nearby vagal and sympathetic centers that control metabolism in peripheral organs. The NTS communicates rostrally with the central nucleus of the amygdala. The central nucleus is integrated into the limbic and autonomic systems throughout the brain, including the hypothalamus (paraventricular nucleus - PVN, lateral hypothalamus - LH, ventromedial hypothalamus - VMH) and brainstem. Stimulation of PVN, VMH or LH alters sympathoadrenal and vagal activities

Brain lesions of the ventromedial hypothalamus produced hyperphagic obesity. Lesions of the lateral hypothalamus caused hypophagia and weight loss. The central administration of insulin also changed the level of defended body weight rather than a simple suppression of food intake. Thus, the hypothalamic pathways that are sensitive to adiposity signals have anatomical connections with caudal brainstem neurons (solitary tract nucleus) that respond to meal-related signals and regulate meal size.

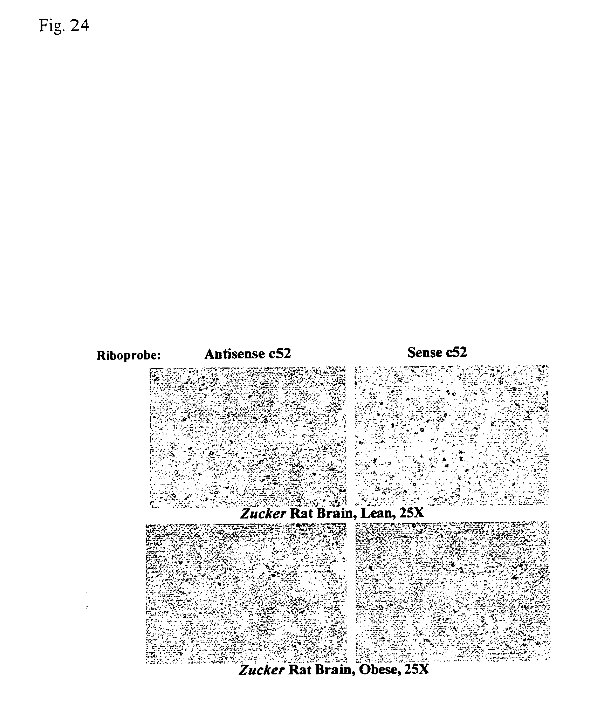

The results from *in-situ* hybridization with a IRSBP-1 riboprobe indicated that IRSBP-1 mRNA is highly expressed in multiple areas of the hypothalamus. As shown in Figs.. 22 - 25, a sagittal cut through the thalamic portion of the diencephalon of a normal Sprague Dawley rat showed that IRSBP-1 is expressed in the ventromedial and dorsomedial hypothalamus, arcuate nucleus and periventricular nucleus (Fig. 22). A coronal cut through the thalamic region also showed that IRSBP-1 mRNA is expressed in the lateral hypothalamus (Fig. 23). Furthermore, comparison of the brain sections from obese and lean Zucker rats showed that the number of silver grains, representing IRSBP-1 mRNA, is higher in the obese than the lean rats (Fig. 24), showing that IRSBP-1 is regulated in the feeding center of the brain, and has a potential role in regulating weight of animals.

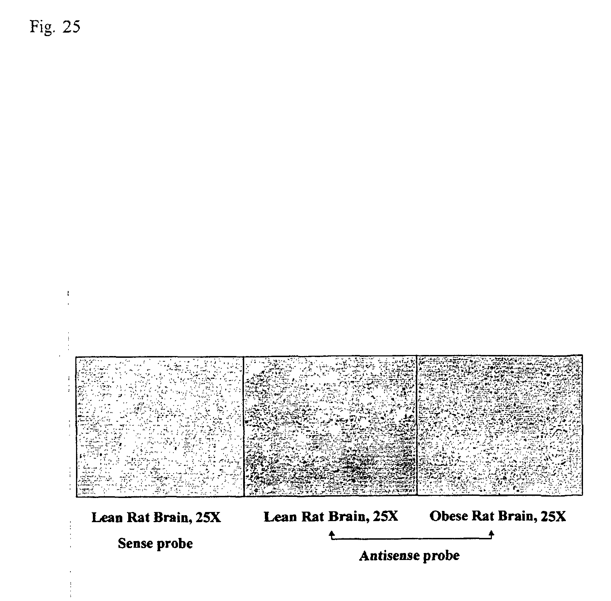

Since all of the information from the neural pathways related to feeding and satiety are integrated in the nucleus of the solitary tract (NTS) of the hindbrain, the expression of IRSBP-1 in the NTS was investigated. As shown in Fig. 25, a sagittal cut through the medullary section of the brain showed that IRSBP-1 mRNA is higher in the NTS of the lean rats, compared to the obese Zucker rats. This further shows that IRSBP-1 is involved not only in altering sympathoadrenal and vagal activities throughout the hypothalamus, but that it has also a potential role in integrating the limbic and autonomic systems involved in maintenance of energy balance.

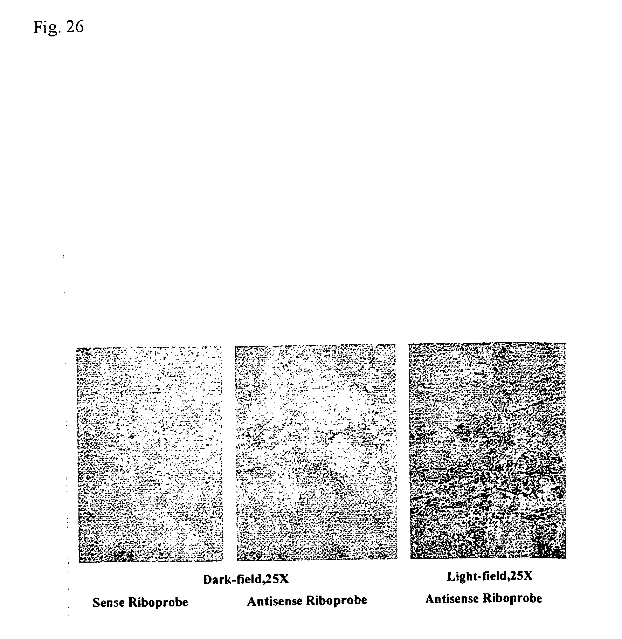

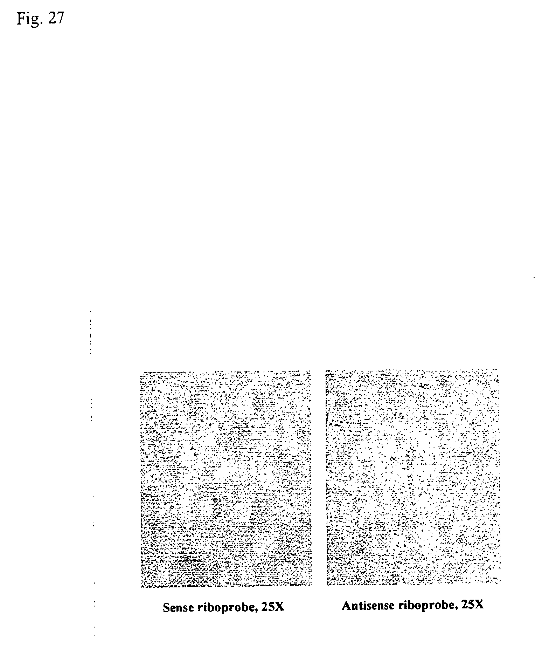

Differential IRSBP-1 expression was also seen in the in situ hybridization, with labeled antisense IRSBP-1 probe, of the pyramidal tract and decussations of the pyramidal tract of obese and lean Zucker rats (Figs. 26 and 27).

### Example 12: Role of IRSBP-1 in the glucose and insulin regulation of food intake and body weight

IRSBP-1 mRNA is highly expressed in the olfactory bulb and amygdala of normal rats. The glucostatic hypothesis proposed that short-term changes in plasma glucose levels can be detected by the brain and will lead to alterations in food intake. Although virtually all neurons require glucose, only select populations in various areas of the brain respond to changes in glucose concentration by changes in their firing rates. An increase in plasma glucose leads to increased plasma norepinephrine levels and sympathetic nervous system activation, and this is mediated by the glucoresponsive neurons in the hypothalamus.

Since IRSBP-1-specific mRNA appears to be concentrated in selected areas of the hypothalamus, and acts to increase glucose uptake and utilization, as shown in Example 12 above, it is likely that IRSBP-1 has a critical role in mediating the autonomic nervous system activation associated with food intake. With a diet high in fat and sucrose, the plasma norepinephrine response to glucose is predictive of later weight gain, with a high responder becoming obese and a low responder becoming resistant to obesity. IRSBP-1 appears to be a factor that determines basal glucose metabolism in the peripheral tissues, and is concomitantly expressed in regions of the brain that modulate food intake. The efficiency of cellular functions related to IRSBP-1 activity will have a significant impact on overall energy homeostasis.

In addition to the roles of IRSBP-1 in affecting glucose metabolism, synthesis of IRSBP-1 is stimulated by addition of insulin; therefore we also need to consider the effect of insulin action on glucose utilization of the brain. Insulin receptors have been localized to the olfactory bulb, hypothalamus, hippocampus, cerebellum, cerebral cortex, and hindbrain. At the cellular level, insulin modulates expression of hypothalamic neuropeptides, inhibits reuptake of norepinephrine, and enhances endogenous β-adrenergic activity. Central administration of insulin decreases food intake and body weight.

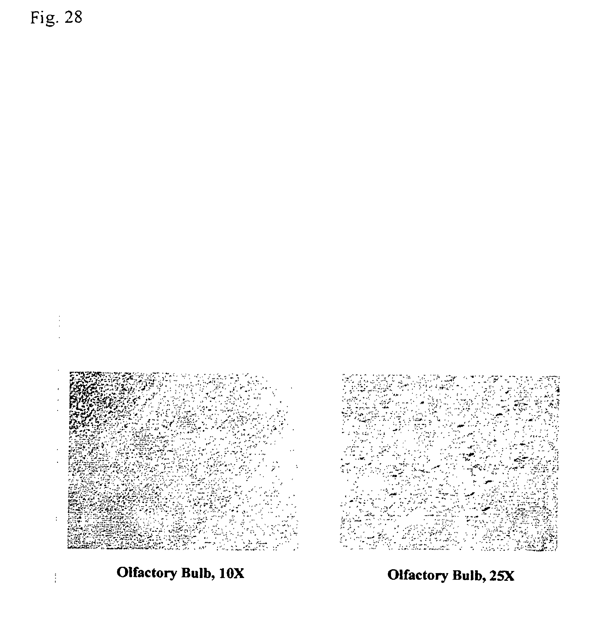

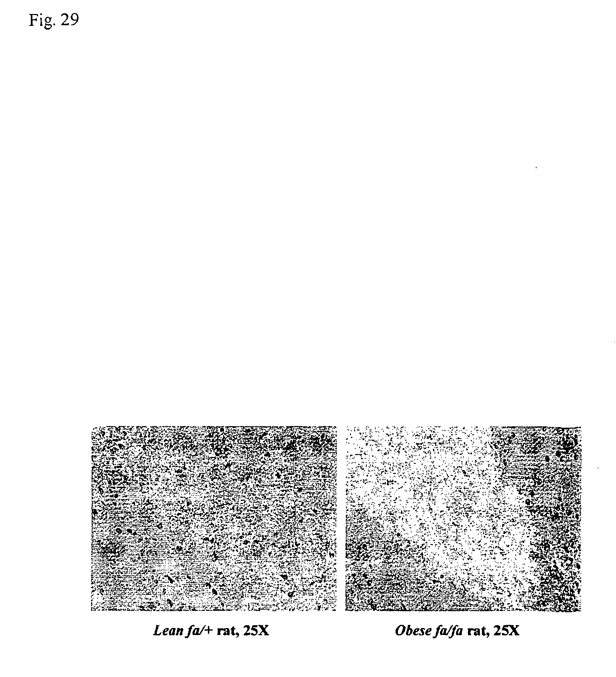

The results showed that IRSBP-1 mRNA is highly expressed in the olfactory bulb and amygdala of normal rats (Figs. 28 and 29), and is also expressed in the cerebral cortex, cerebellum and corpus callosum. Lesions of the posterodorsal aspects of the amygdala have been associated with hyperinsulinemia, hyperphagia, and obesity without the preference for particular food that characterized other brain lesion-induced obesity. The involvement of the olfactory system with high levels of expressed IRSBP-1 (Figs. 28 and 29) in the primary sense of smell, and the affective component associated with eating under the control of the limbic system, including the amygdala and corpus callosum. IRSBP-1 therefore may affect multiple aspects of brain function associated with feeding and satiety.

### Example 13: IRSBP-1 is targeted to pancreatic beta cells of the islets of Langerhans

IRSBP-1 appears to mediate a significant repertoire of insulin's cellular effects. Glucose is the principal regulator of insulin secretion from pancreatic beta cells, and the kinetic response of insulin to glucose is biphasic in nature. A rapid secretory burst begins within 1 min and decreases over the next 3 to 5 mins. The second phase is characterized by a gradual increase in insulin levels over 5-10 mins, which continues for the next hour. Many type II diabetics have a marked reduction in first phase insulin secretion.

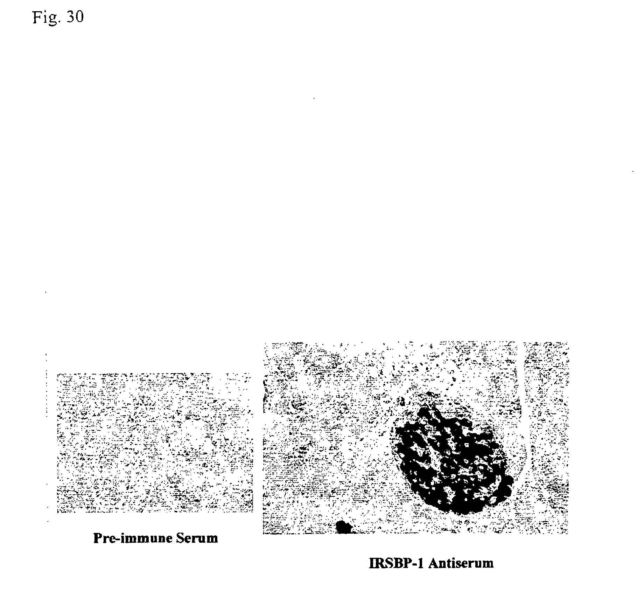

A polyclonal antibody raised against the epitopic region (SEQ ID NO: 13) of the rat IRSBP-1 polypeptide SEQ ID NO: 11 and capable of detecting the presence of the IRSBP-1 protein of at least rat or human, was used in immunohistochemical staining of the pancreas to detect the cellular location of IRSBP-1. As shown in Fig. 30, IRSBP-1 expression is strongest in the beta cells of the islets of Langerhans.

Insulin acts on beta cells to regulate insulin secretion, insulin synthesis, and glucose sensing/utilization. Functional insulin receptor and IRS-1 have been identified in beta cells. IRSBP-1 is also present in the beta cells of the islets of Langerhans (Fig. 30). Insulin secretion in isolated beta cells is mediated via the insulin receptor. Mice with knockout of the beta cell insulin receptor lose their first-phase insulin response to glucose, but not to other secretagogue like arginine, similar to type II diabetes. It has been shown that glucose stimulation of beta cells activates the insulin receptor in a similar fashion as insulin itself. Thus, the presence of insulin receptors and normal insulin signaling are necessary for the physiological response of beta cell secretion to occur.

A defect in insulin action may be the common molecular defect that links impaired insulin secretion in beta cells and insulin resistance at the peripheral tissues. Since IRSBP-1 is an effector molecule by which the major signal transduction pathways of insulin converge, and IRSBP-1 appears to mediate a significant repertoire of insulin's cellular effects. IRSBP-1 may increase insulin synthesis (through stimulation of protein synthesis), and increase glucose sensing and utilization in beta cells (through increased uptake), leading to restoration of physiological insulin response to glucose in diabetes and insulin resistance.

### Example 14: Immunodetection of IRSBP-1 expresson in pancreatic renal, vascular and neural tissues

Since glucose is the principal regulator of insulin secretion from pancreatic beta cells, and clone 52 mimics insulin action on glucose transport and metabolism, we also determined the expression of IRSBP-1 in the pancreas.

Fixed and paraffin embedded tissue was deparaffinized, rehydrated, treated with proteinase K at 50 µg/ml for 10 min at room temperature, washed with PBS, and blocked with a 1 % gelatin/PBS mixture for 20 mins. The primary antibody, anti-rat IRSBP-1 peptide antibody was added at 1:200 dilution in 1% BSA / lx PBS, and the sample was incubated in a humid chamber for 1 hour at room temperature. After washing, a biotinylated secondary antibody was added at 1:400 dilution, and incubated with the sample for 30 min. Color development was performed with the ABC-vector Red complex from an alkaline phosphatase standard kit. The slide was subsequently counterstained with Gill's hematoxylin, dehydrated and mounted.

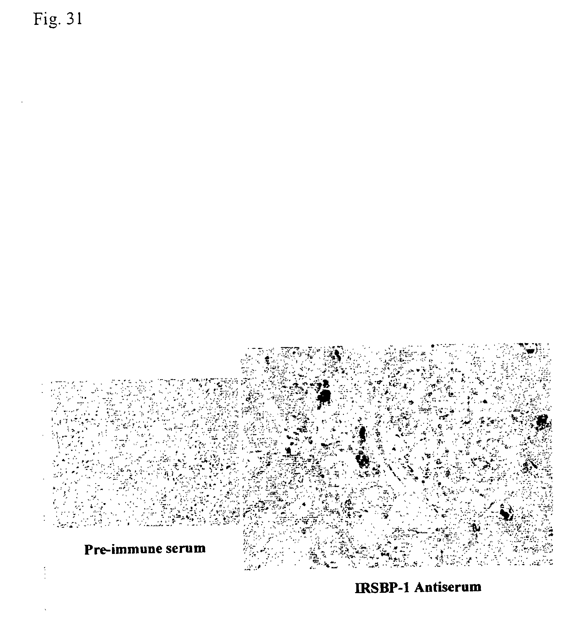

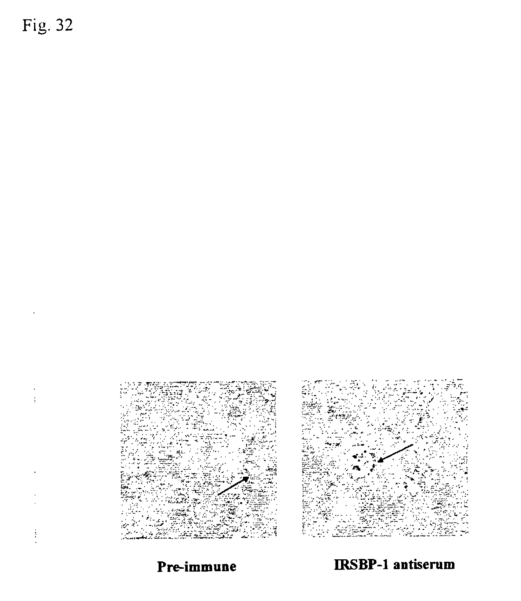

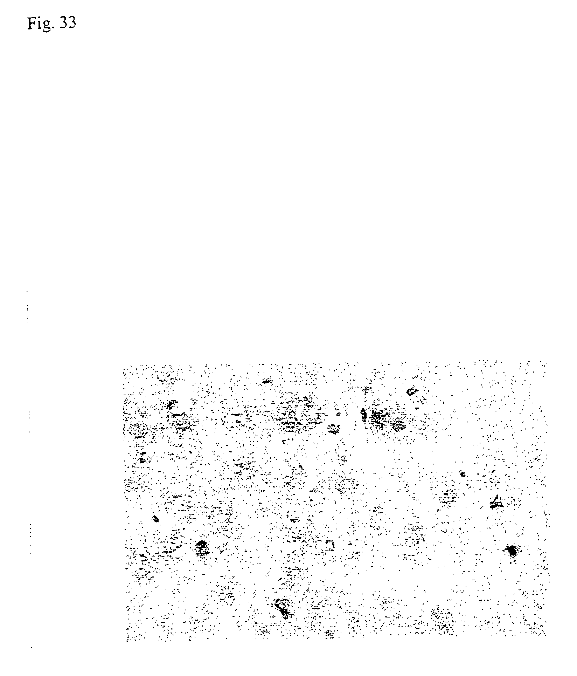

As shown in Fig. 30, immunostain of pancreas showed intense accumulation of IRSBP-1 is the cytoplasm of the islet of Langerhans. In particular, the insulin-secreting β cells of the pancreas, which comprised 74% of the islet mass and is central in location, expressed abundant IRSBP-1, as described in Example 13 above. IRSBP-1 expression was also detected by immunodetection in the mesangium of the glomerulus of the kidney (Fig. 31), in vascular endothelial cells (Fig. 32) and in neuronal cells (Fig. 33).

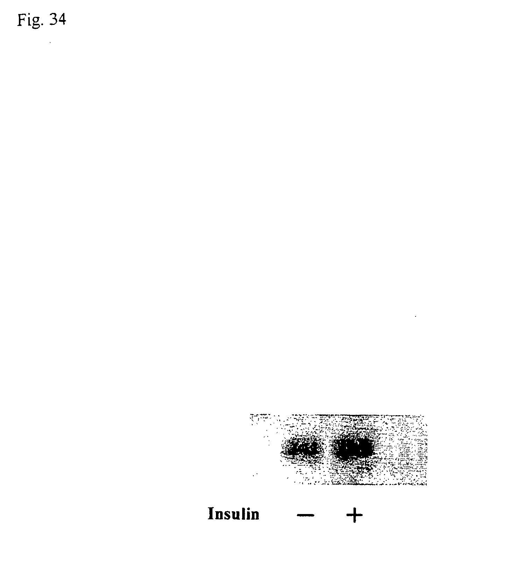

A Western blot analysis of the expressed proteins from cultured human vascular endothelial cells, treated or untreated with insulin, probed with a polyclonal anti-rat IRSBP-1 antibody shows that insulin induces the formation of IRSBP-1 in such cells (Fig. 34). This experiment further shows that a rabbit anti-rat IRSBP-1 antibody will cross-react with an IRSBP-1 of a different species. The localization of IRSBP-1 to the endothelium, mesangium and neurons has implications on the development of vascular, renal and neuropathic complications of diabetes. Insulin causes endothelium-derived nitric oxide-dependent vasodilation and modulates vascular tone. Mesangial cell proliferation and expansion is the initial event in the development of diabetic nephropathy. Thus, the action of IRSBP-1 in mediating insulin action in the endothelium will increase the vasodilatory capacity of the blood vessels, and decrease blood pressure and the subsequent onset of atherosclerosis. The antiproliferative action of IRSBP-1 will also decrease the capacity of mesangium to expand and delay the development of diabetic nephropathy.

### Example 15: Determining the position of IRSBP-1 in the insulin signaling pathway

Agarose conjugates of both anti-Akt1 (PkB) and anti-Erk2 (MAP kinase) antibodies (Santa Cruz Biotechnology, Santa Cruz, CA) were incubated with nuclear extracts from COS7 cells, then precipitated with the agarose, the protein eluted and western blotted with anti-IRSBP-1 peptide polyclonal antibody. As shown in Fig. 33 (left panel), the 98 kDa IRSBP-1 protein co-immunoprecipitated with both Aktl and Erk2, indicating that *in vivo,* the two major signaling pathways of insulin action probably converge on IRSBP-1. To test for the specificity of the immunoprecipitation, the blot was reprobed with an anti-Erk polyclonal antibody. The results showed that the lanes that precipitated with agarose conjugates of Erk2 antibody and the COS7 extracts contained the 40 kDa Erk2 protein, but not the lanes precipitated with Akt antibodies.

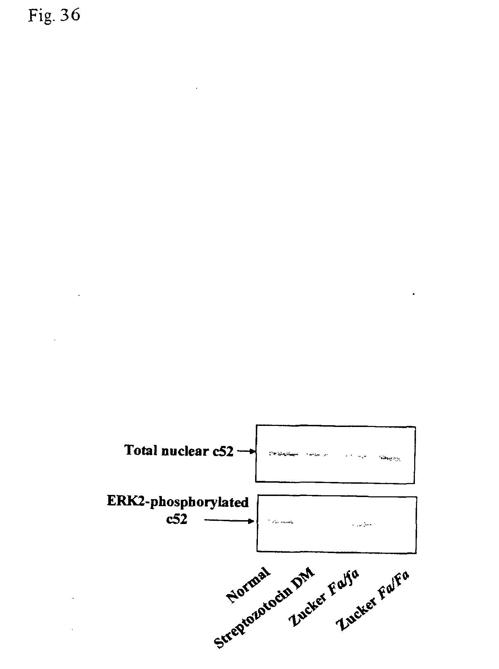

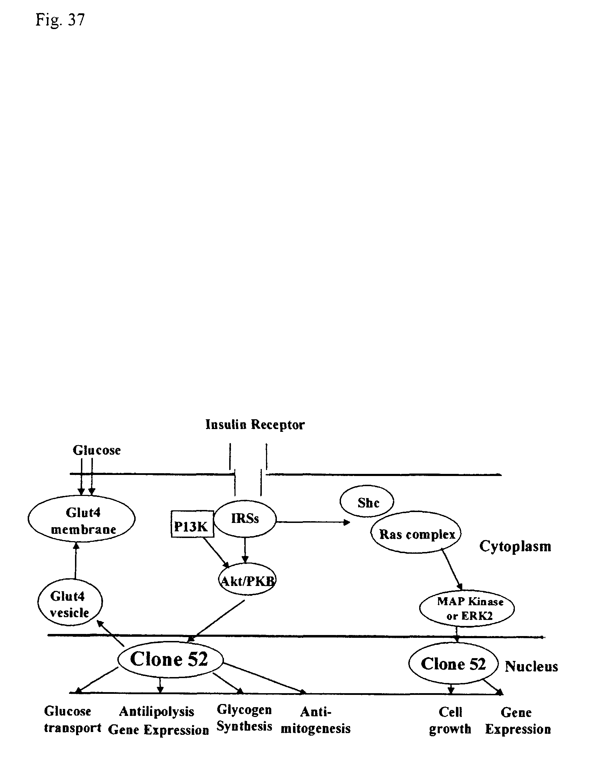

To confirm further the specificity of the immunoprecipitation, the blot was stripped and reprobed with anti-Sp1 antibody. Sp1 is a ubiquitous transcription factor in mammalian cells, and as shown in the right panel of Fig. 33, Sp1 protein is detected in COS7 cell extracts, but not in lanes precipitated with either Aktl or Erk2 antibodies. Decreased Erk-mediated phosphorylation of IRSBP-1 in diabetic and obese rats was also seen (Fig. 36). The locations of the IRSBP-1 protein in the signal transduction pathway of insulin signaling are shown in Fig. 37.

### Example 16: Administration of IRSBP-1 sense and antisense oligonucleotides to an animal

*Methods & Materials.* The methodology was adapted from that of *Apostolakis et al*, J. Neurosci. 16: 4823-4834). Briefly, ovariectomized (OVX) female rats (200-250 gm) were housed individually and maintained on a 12:12 h light:dark cycle (lights on at 0700 CST) with rat chow and water in excess *ad libitum.* After acclimation (7 days), females underwent stereotaxic implantation of third ventricle cannula guides (26 gauge, Plastics One, Roanoke VA). The experiment started 7 days after surgery. Females were randomly assigned identification numbers and weighed daily between 0900 and 1000 CST for 5 days immediately before experimental treatment, allowing each animal to serve as its own control. Each animal (n = 5) received a single intraventricular (icv) injection of antisense (AS, sequence 5'-CTAACTCACAGGTGATGATGTAGAG-3', SEQ ID NO: 42) oligos (4 nM in 1 µl vol. over 2 min) after weighing on the fifth day. Another group of animals (n = 4) served as positive controls and received sense (S, sequence 5'-CTCTACATCATCACCTGTGAGTTAG-3', SEQ ID NO: 43) oligos (4 nM in 1 µl vol. over 2 min). Animals were euthanized under deep anesthesia 8 days after oligonucleotide treatment.

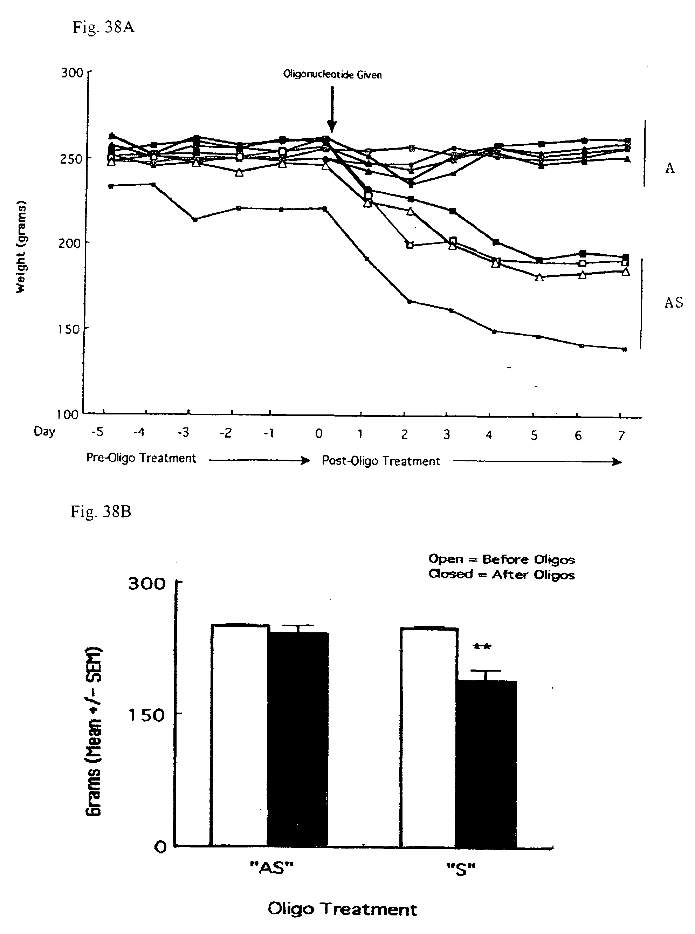

There was a difference in mean weight loss between those animals following treatment with antisense DNA and sense DNA (Fig. 38A). For the five days prior to oligonucleotide treatment, the animals demonstrated stable weight. Within 24 hours after treatment, animals that received sense DNA began to lose weight with the greatest loss (54±6.4 gm) being attained at 72-96 hours after treatment as shown in Fig. 38A. Antisense DNA had no significant effect on weight (251±2 gm) while sense DNA-treated animals lost weight (54.3±6.2 gm). Individually, the females receiving sense DNA lost 27% of their body weight as compared with either their initial weight, their mean weight during the pretreatment time or mean weight of antisense-treated animals as shown in Fig. 38B.

### Example 17: Inhibitory effect of IRSBP-1 on cell proliferation

### Transfected L6 cells are growth arrested when transfected with IRSBP-1 expressing nucleic acid.

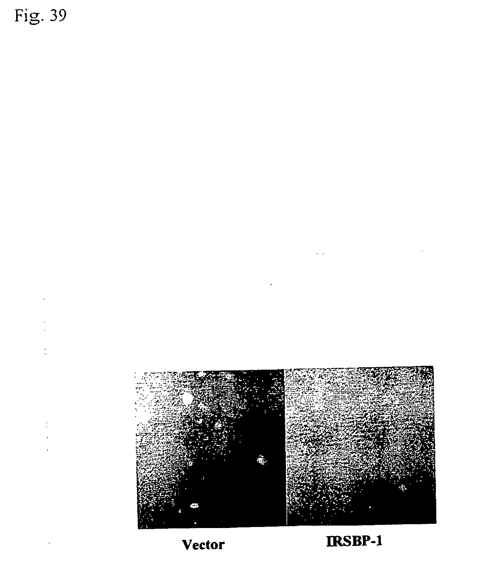

L6 cells stably transfected with the IRSBP-1-encoding cDNA (SEQ ID NO: 14) exhibited significantly reduced proliferation rates when cultured in soft agar, compared to transfection with the vector alone, as shown in Fig. 39. *Flow cytometry analysis of the cell cycle compartments of L6 cells with or without over-expression of the IRSBP-1 nucleic acid sequence.*

Flow cytometry was conducted to analyze DNA content and progression through the cell cycle. Stable transfectants were fixed in ice cold 100% ethanol and debris was removed by centrifugation through a cushion of fetal bovine serum. Cell pellets were treated with RNAse solution (500 units/ml of 1.12% (w/v) sodium citrate) at 37° C for 15 mins, and DNA was stained with propidium iodide (5 mg/100 ml of 1.12% sodium citrate) for 30 minutes at room temperature before analyzing on the flow cytometer.

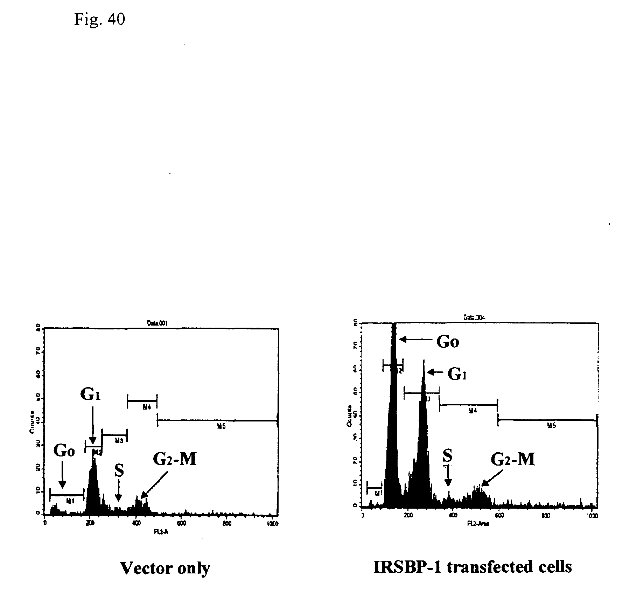

Stable cell lines transfected with the expression vector pCMV-Tag with and without the IRSBP-1 nucleic acid sequence (SEQ ID NO: 14) were grown to confluence, fixed with ethanol, and stained with propidium iodide. Cell cycle compartment analysis was done by flow cytometry, measuring excitation at 488 nm with argon lasers. The histograms illustrated in Fig. 40 show that cells transfected with the vector alone, 58% were in the G0/GI phase, 12% in the S phase and 20% in the G2/M phases. With cells transfected with the plasmid comprising the IRSBP-1 sequence (SEQ ID NO: 14), 86% of the cells were in the GO/G 1 phase, and only 11% of the cells were in S or G2/M phases.

### Example 18: Generation of IRSBP-1 knockout mice using the IRSBP-1 cDNA clones of the present invention

To investigate the function of IRSBP-1, transgenic mice will be generated in which the *IRSBP-1* gene is replaced by the neo^{r} gene. A DNA construct will first be generated that contains neo^{r} linked to a constitutive promoter. This gene will be flanked on either side by at least 1kb of genomic IRSBP-1 sequence, which will allow for homologous recombination and integration of neo^{r} into the endogenous *IRSBP-1* gene. Finally, the Herpes Simplex Virus thymidine kinase (HSV-tk) gene will be incorporated into each end of the DNA construct, adjacent to the IRSBP-1 sequences, to allow for selection of homologous recombinants.

Following linearization, the DNA construct described above will be transfected into embryonic stem (ES) cells by electroporation, and these cells will be transferred to culture on gelatin-coated dishes. The addition of G418 will permit selection for cells that contain integration of neo^{r}, while the nucleoside analog gancyclovir will allow for selection of cells in which homologous recombination has occurred; homologous but not heterologous recombination results in removal of the HSV-tk genes from the transfected construct, thus preventing gancyclovir cytotoxicity. ES cell colonies that are resistant to both G418 and gancyclovir will be screened by PCR or Southern analysis for presence and copy number of the *neo*^{*r*} gene, and positive colonies will be subcultured and amplified.

ES cells that have successfully integrated neo^{r} in place of the *IRSBP-1* gene will be used for morula aggregation with 8-cell embryos; aggregates will subsequently be implanted into pseudopregnant female mice. Chimeric mice will be identifiable by their coat color, since the aggregation of ES cells from the 129 strain of agouti mice with 8-cell embryos from an albino strand such as CDI will result in chimeric mice exhibiting white coats with brown splotches. Chimeras will be bred in order to look for germline transmission of the transgene; transgenic offspring will have completely brown coats and will be heterozygous for the transgene. These heterozygous mice will be crossed, resulting in a homozygous line if mutation of the IRSBP-1 is not lethal.

If inactivation of IRSBP-1 proves to be lethal, we will produce tissue-specific knockouts. A transgenic line will be created encoding Cre recombinase in selected tissues using the strategy of targeted transgene expression. A responder "knock-in" mouse will also be created containing a targeting vector that is nearly an exact copy of a segment of genomic IRSBP-1 DNA, except that a critical exon will be flanked by the sites binding the recombinanse (*lox-P* sites). The mice will be intercrossed, and compound transgenic knockout mice will be produced in which the target gene is excised by Cre recombinase only in the desired cell type (Cre^{+/+} x IRSBP-1^{-/-} or Cre^{+/-} x IRSBP-1^{-/-}).

Once IRSBP-1 knockout mice (IRSBP-1^{-/-}) are obtained that survive widespread tissue inactivation of IRSBP-1, we will look for tissue-specific, developmental and metabolic changes. Although it is possible that other gene products might be capable of functionally replacing IRSBP-1, our preliminary experiments with stably transfected L6 myoblasts suggest that IRSBP-1 acts at the distal end of the insulin activation pathway and is thus unlikely to be genetically redundant. With tissue-specific knockouts (muscle Cre^{+/+} x floxed IRSBP-1^{-/-}, adipose Cre^{+/+} x floxed IRSBP-1^{-/-}, and liver Cre^{+/+} x floxed IRSBP-1^{-/-}), serial crosses of mice will be conducted to allow concurrent inactivation of IRSBP-1 in multiple organs.

Once IRSBP-1 knockout mice are obtained , they will used to test insulin action, and investigate their susceptibility to diabetes and diabetes complications. Growth curves, including intrauterine growth, size and weight of the viscera, and necropsy at various ages to determine for gross histological differences will be determined. We will define the phenotype of the animals in terms of blood glucose, insulin, glucagon, cortisol and leptin measurements at fasted and fed levels. Immunohistochemical analysis of the pancreas, kidneys, intestine and organs targeted for knockout studies will be done to confirm the effectiveness of gene ablation and detect presence of changes associated with diabetes. Glucose tolerance tests will be performed by intraperitoneal glucose injection (2 mg/gm body weight), and tail bleed will be at 0, 30, 60 and 120 mins, after injection to check for glucose and insulin levels. Finally, wild-type, IRSBP-1-deficient and heterozygote littermates will be subjected to a diet high in fat (50% of calories from fat) and monitored for parameters of obesity-induced insulin resistance.

## Claims

1. An isolated nucleic acid encoding a region of Insulin-Responsive Sequence DNA Binding Protein-1 (IRSBP-1) comprising the nucleotide sequence selected from SEQ ID NOS: 2, 5 to 10, and 14.

2. The isolated nucleic acid of Claim 1 further selected from SEQ ID NOS: 5 and 7.

3. The isolated nucleic acid of Claims 1 or 2 comprising a nucleotide sequence that encodes a polypeptide identical to SEQ ID NOS: 11 or 12.

4. The isolated nucleic acid of Claim 3, wherein the polypeptide is one of SEQ ID NOS: 11 or 12.

5. The isolated nucleic acid of Claim 3, wherein said polypeptide is capable of regulating the expression of an insulin responsive gene.

6. The isolated nucleic acid of Claims 1 or 2 comprising a sequence 75-100% identical to SEQ ID NOS: 5 or 7.

7. The isolated nucleic acid of Claims 1 or 2 comprising a sequence 90-100% identical to SEQ ID NOS: 5 or 7.

8. A vector comprising the nucleic acid according to one of the Claims 1 to 7.

9. The vector of Claim 8, wherein the vector is an expression vector.

10. The vector of Claim 8, wherein the vector is an expression vector comprising the nucleic acid of any one of Claims 1 to 7 operably linked to an expression control sequence, wherein the expression control sequence directs production of a transcript that hybridizes under physiological conditions to SEQ ID NOS: 5 or 7 or the complement thereof.

11. An expression system comprising the isolated nucleic acid of any one of Claims 1 to 7.

12. An isolated nucleic acid probe comprising a sequence that hybridizes under conditions that permit hybridization of the isolated nucleic acid probe with the nucleic acid of any one of Claims 1 to 7.

13. The isolated nucleic acid of any one of Claims 1 to 7 operably linked to a heterologous coding sequence.

14. The isolated nucleic acid of Claim 13, wherein the heterologous coding sequence is selected from the group consisting of promoters, enhancers, transcription, activators and the like.

15. A cultured cell comprising the vector of any one of Claims 8 to 10.

16. The cultured cell of Claim 15, wherein the cell is transfected with the vector of one of Claims 8 to 10, or a progeny of said cell, wherein said cell expresses a polypeptide.

17. The cultured cell of Claim 16, wherein said polypeptide comprises amino acid sequence SEQ ID NOS: 11 or 12.

18. A method of producing a protein, comprising culturing the cell of Claim 16 or 17 under conditions permitting expression under the control of the expression control sequence, and purifying the polypeptide from the cell or the medium of the cell.

19. A method of hybridization comprising providing a single-stranded oligonucleotide at least eight nucleotides in length, the oligonucleotide being complementary to a nucleic acid sequence selected from SEQ ID NOS: 2, 5 to 10, 14, or the complement thereof, said nucleic acid sequence encoding a region of Insulin-Responsive Sequence DNA Binding Protein-1 (IRSBP-1); and contacting the oligonucleotide with a nucleic acid comprising the sequence of SEQ ID NOS: 2, 5 to 10, 14, or the complement thereof under conditions that permit hybridization of the oligonucleotide with the nucleic acid.

20. A method for identifying a nucleic acid homolog comprising the steps of:
a) obtaining a nucleic acid sequence probe molecule comprising a nucleotide sequence selected from SEQ ID NO: 2, 5 to 10, 14, or the complement thereof, and encoding a region of Insulin-Responsive Sequence DNA Binding Protein-1 (IRSBP-1);
b) accessing a sequence database comprising a target nucleotide sequence;
c) aligning said nucleic acid sequence probe molecule with said target nucleotide sequence;
d) determining a percent homology between a nucleotide sequence of said nucleic acid sequence probe molecule and said target nucleotide sequence; and
e) identifying said target nucleotide sequence in said sequence database wherein the percent homology between the nucleotide sequence of said nucleic acid sequence probe molecule and said target nucleotide sequence is 80-100%.

21. The method as in Claim 20, wherein the percent homology between the nucleotide sequence of said nucleic acid sequence probe molecule and said target nucleotide sequence is 90-100%, and wherein the nucleic acid sequence probe molecule comprises a nucleotide sequence selected from SEQ ID NOS: 5 or 7.

22. An antibody that binds specifically to a polypeptide encoded by the nucleic acids as defined in any one of Claims 1 to 7.

23. The antibody of Claim 22, wherein the polypeptide encoded by the nucleic acids has the sequence SEQ ID NO: 14.

24. A method of determining whether a biological sample contains an IRSBP-1 protein, comprising the steps of:
a) obtaining a biological sample from a human or animal,
b) extracting the biological sample with the antibody of Claims 22 or 23; and
c) determining whether the antibody specifically binds to the sample, the binding indicating that the biological sample contains IRSBP-1.

25. The method of Claim 24, wherein the biological sample comprises cells and the binding of the antibody indicates the cells as having IRSBP-1.

26. A method of detecting a disease comprising the steps of providing a biological sample from a human or animal; and selectively detecting a nucleic acid encoding a region of Insulin-Responsive Sequence DNA Binding Protein-1 (IRSBP-1) according to one of Claims 1 to 7, wherein the detection of a nucleic acid according to one of Claims 1 to 7 indicates a disease.

27. Insulin-Responsive Sequence DNA Binding Protein-1 (IRSBP-1), capable of binding to nucleic acid regions associated with genes that respond when cells are exposed to insulin or insulin-like factors and having a sequence which is 75-100% identical to SEQ ID NOS: 11 or 12.

28. The Insulin- Responsive Sequence DNA Binding Protein-1 (IRSBP-1) of claim 27, having a sequence which is 90-100% identical to SEQ ID NOS: 11 or 12.

## Patentansprüche

1. Eine isolierte Nukleinsäure, die für eine Region des Insulin-Responsive DNA-Sequenz Bindenden Protein-1 (IRSBP-1) codiert, welche die Nukleotidsequenz, ausgewählt aus den SEQ ID NOS: 2, 5 bis 10 und 14, umfasst.

2. Isolierte Nukleinsäure nach Anspruch 1, die ausserdem aus den SEQ ID NOS: 5 und 7 ausgewählt ist.

3. Isolierte Nukleinsäure nach Anspruch 1 oder 2, die eine Nukleotidsequenz umfasst, welche für ein mit den SEQ ID NOS: 11 oder 12 identisches Polypeptid codiert.

4. Isolierte Nukleinsäure nach Anspruch 3, wobei das Polypeptid eines aus den SEQ ID NOS: 11 oder 12 ist.

5. Isolierte Nukleinsäure nach Anspruch 3, wobei das Polypeptid im Stande ist, die Expression eines Insulin responsiven Gens zu regulieren.

6. Isolierte Nukleinsäure nach Anspruch 1 oder 2, die eine Sequenz umfasst, welche zu 75-100% mit den SEQ ID NOS: 5 oder 7 identisch ist.

7. Isolierte Nukleinsäure nach Anspruch 1 oder 2, die eine Sequenz umfasst, welche zu 90-100% mit den SEQ ID NOS: 5 oder 7 identisch ist.

8. Ein Vektor, der die Nukleinsäure nach einem der Ansprüche 1 bis 7 umfasst.

9. Vektor nach Anspruch 8, wobei der Vektor ein Expressionsvektor ist.

10. Vektor nach Anspruch 8, wobei der Vektor ein Expressionsvektor ist, der die Nukleinsäure nach irgendeinem der Ansprüche 1 bis 7 in funktioneller Verbindung mit einer Expressionskontrollsequenz umfasst, wobei die Expressionskontrollsequenz die Erzeugung eines Transkripts steuert, das unter physiologischen Bedingungen an die SEQ ID NOS: 5 oder 7 oder deren Komplement hybridisiert.

11. Expressionssystem, das die isolierte Nukleinsäure nach irgendeinem der Ansprüche 1 bis 7 umfasst.

12. Eine isolierte Nukleinsäuresonde, die eine Sequenz umfasst, welche unter Bedingungen, die eine Hybridisierung der isolierten Nukleinsäuresonde gestatten, mit der Nukleinsäure nach irgendeinem der Ansprüche 1 bis 7 hybridisiert.

13. Isolierte Nukleinsäure nach irgendeinem der Ansprüche 1 bis 7, die funktionell mit einer heterologen codierenden Sequenz verbunden ist.

14. Isolierte Nukleinsäure nach Anspruch 13, wobei die heterologe codierende Sequenz aus der Gruppe bestehend aus Promotoren, Enhancern, Transkriptionsaktivatoren und dergleichen ausgewählt ist.

15. Eine kultivierte Zelle, die den Vektor nach irgendeinem der Ansprüche 8 bis 10 enthält.

16. Kultivierte Zelle nach Anspruch 15, wobei die Zelle mit dem Vektor nach einem der Ansprüche 8 bis 10 transfektiert ist, oder ein Nachkomme dieser Zelle, wobei die Zelle ein Polypeptid exprimiert.

17. Kultivierte Zelle nach Anspruch 16, wobei das Polypeptid die Aminosäuresequenz der SEQ ID NOS: 11 oder 12 umfasst.

18. Verfahren zur Herstellung eines Proteins, welches das Kultivieren der Zelle nach Anspruch 16 oder 17 unter Bedingungen, welche die Expression unter der Kontrolle der Expressionskontrollsequenz gestatten, und die Aufreinigung des Polypeptids aus der Zelle oder dem Medium der Zelle umfasst.

19. Verfahren zur Hybridisierung, das die Bereitstellung eines einsträngigen Oligonukleotids von wenigstens acht Nukleotiden Länge, wobei das Oligonukleotid komplementär ist zu einer Nukleinsäuresequenz, ausgewählt aus den SEQ ID NOS: 2, 5 bis 10, 14 oder deren Komplement, wobei diese Nukleinsäuresequenz für eine Region vom Insulin-Responsive DNA-Sequenz Bindenden Protein-1 (IRSBP-1) codiert; und das Inkontaktbringen des Oligonukleotids mit einer Nukleinsäure, welche die Sequenz der SEQ ID NOS: 2, 5 bis 10, 14 oder deren Komplement umfasst, unter Bedingungen, welche eine Hybridisierung des Oligonukleotids mit der Nukleinsäure gestatten, umfasst.

20. Verfahren zur Identifizierung eines Nukleinsäure-Homologons, das die folgenden Schritte umfasst:
a) Gewinnung eines Nukleinsäuresequenz-Sondenmoleküls, das eine Nukleotidsequenz, ausgewählt aus den SEQ ID NOS: 2, 5 bis 10, 14 oder deren Komplement, umfasst und für eine Region vom Insulin-Responsive DNA-Sequenz Bindenden Protein-1 (IRSBP-1) codiert;
b) Zugriff auf eine Sequenzdatenbank, die eine Zielnukleotidsequenz umfasst;
c) Alignment des Nukleinsäuresequenz-Sondenmoleküls mit der Zielnukleotidsequenz;
d) Bestimmung einer Prozenthomologie zwischen einer Nukleotidsequenz des Nukleinsäuresequenz-Sondenmoleküls und der Zielnukleotidsequenz; und
e) Identifizierung der Zielnukleotidsequenz in der Sequenzdatenbank, wobei die Prozenthomologie zwischen der Nukleotidsequenz des Nukleinsäuresequenz-Sondenmoleküls und der Zielnukleotidsequenz 80-100% beträgt.

21. Verfahren nach Anspruch 20, wobei die Prozenthomologie zwischen der Nukleotidsequenz des Nukleinsäuresequenz-Sondenmoleküls und der Zielnukleotidsequenz 90-100% beträgt und wobei das Nukleinsäuresequenz-Sondenmolekül eine Nukleotidsequenz umfasst, die aus den SEQ ID NOS: 5 oder 7 ausgewählt ist.

22. Ein Antikörper, der spezifisch an ein Polypeptid bindet, das von den Nukleinsäuren, wie sie in irgendeinem der Ansprüche 1 bis 7 definiert sind, codiert wird.

23. Antikörper nach Anspruch 22, wobei das von den Nukleinsäuren codierte Polypeptid die Sequenz SEQ ID NO: 14 aufweist.

24. Verfahren zur Bestimmung, ob eine biologische Probe ein IRSBP-1-Protein enthält, das die folgenden Schritte umfasst:
a) Gewinnung einer biologischen Probe von einem Menschen oder Tier;
b) Extraktion der biologischen Probe mit dem Antikörper nach Anspruch 22 oder 23; und
c) Bestimmung, ob der Antikörper spezifisch an die Probe bindet, wobei die Bindung anzeigt, dass die biologische Probe IRSBP-1 enthält.

25. Verfahren nach Anspruch 24, wobei die biologische Probe Zellen umfasst und die Bindung des Antikörpers anzeigt, dass die Zellen IRSBP-1 aufweisen.

26. Verfahren zum Nachweis einer Krankheit, das die Bereitstellung einer biologischen Probe von einem Menschen oder Tier und den selektiven Nachweis einer Nukleinsäure nach einem der Ansprüche 1 bis 7, die für eine Region vom Insulin-Responsive DNA-Sequenz Bindenden Protein-1 (IRSBP-1) codiert, umfasst, wobei der Nachweis einer Nukleinsäure nach einem der Ansprüche 1 bis 7 eine Krankheit anzeigt.

27. Ein Insulin-Responsive DNA-Sequenz Bindendes Protein-1 (IRSBP-1), das im Stande ist, an Nukleinsäureregionen zu binden, die mit Genen assoziiert sind, welche reagieren, wenn Zellen Insulin oder insulinähnlichen Faktoren ausgesetzt sind, und das eine Sequenz aufweist, die zu 75-100% mit den SEQ ID NOS: 11 oder 12 identisch ist.

28. Insulin-Responsive DNA-Sequenz Bindendes Protein-1 (IRSBP-1) nach Anspruch 27, das eine Sequenz aufweist, die zu 90-100% mit den SEQ ID NOS: 11 oder 12 identisch ist.

## Revendications

1. Acide nucléique isolé codant pour une région de la protéine liant l'ADN de la séquence répondant à l'insuline (IRSBP-1) comprenant la séquence de nucléotides sélectionnée parmi les séquences ayant le numéro d'identification 2, 5 à 10 et 14.

2. Acide nucléique isolé selon la revendication 1, sélectionné en outre parmi les séquences ayant le numéro d'identification 5 et 7.

3. Acide nucléique isolé selon les revendications 1 ou 2 comprenant une séquence de nucléotides codant pour un polypeptide identique aux séquences ayant le numéro d'identification 11 ou 12.

4. Acide nucléique isolé selon la revendication 3, dans lequel le polypeptide est soit la séquence ayant le numéro d'identification 11 ou 12.

5. Acide nucléique isolé selon la revendication 3, dans lequel ledit polypeptide peut réguler l'expression d'un gène répondant à l'insuline.

6. Acide nucléique isolé selon les revendications 1 ou 2 comportant une séquence identique à 75-100 % à la séquence ayant le numéro d'identification 5 ou 7.

7. Acide nucléique isolé selon les revendications 1 ou 2 comportant une séquence identique à 90-100 % à la séquence ayant le numéro d'identification 5 ou 7.

8. Vecteur comprenant l'acide nucléique selon l'une quelconque des revendications 1 à 7.

9. Vecteur selon la revendication 8, dans lequel le vecteur est un vecteur d'expression.

10. Vecteur selon la revendication 8, dans lequel le vecteur est un vecteur d'expression comprenant l'acide nucléique selon l'une quelconque des revendications 1 à 7 en liaison fonctionnelle avec une séquence de régulation de l'expression, dans lequel la séquence de régulation de l'expression contrôle la production d'un transcript qui s'hybride dans les conditions physiologiques avec les séquences ayant le numéro d'identification 5 ou 7 ou leur complément.

11. Système d'expression comprenant l'acide nucléique isolé selon l'une quelconque des revendications 1 à 7.

12. Sonde d'acide nucléique isolé comprenant une séquence qui s'hybride dans des conditions permettant l'hybridation de la sonde d'acide nucléique isolé avec l'acide nucléique selon l'une quelconque des revendications 1 à 7.

13. Acide nucléique isolé selon l'une quelconque des revendications 1 à 7 en liaison fonctionnelle avec une séquence de codage hétérologue.

14. Acide nucléique isolé selon la revendication 13, dans lequel la séquence de codage hétérologue est sélectionnée dans le groupe comprenant des promoteurs, des relevants, des activateurs de transcription et similaires.

15. Cellule de culture contenant le vecteur selon l'une quelconque des revendications 8 à 10.

16. Cellule de culture selon la revendication 15, dans laquelle la cellule est transfectée avec le vecteur selon l'une quelconque des revendications 8 à 10, ou un descendant de ladite cellule, ladite cellule exprimant un polypeptide.

17. Cellule de culture selon la revendication 16, dans laquelle ledit polypeptide comprend la séquence d'acides aminés de la séquence ayant le numéro d'identification 11 ou 12.

18. Procédé pour la production d'une protéine, comprenant la mise en culture de la cellule selon la revendication 16 ou 17 dans des conditions permettant l'expression, sous le contrôle de la séquence de régulation de l'expression, et la purification du polypeptide à partir de la cellule ou du milieu de culture de la cellule.

19. Procédé d'hybridation comprenant la production d'un oligonucléotide à un seul brin ayant au moins huit nucléotides de longueur, lequel oligonucléotide est complémentaire d'une séquence d'acides nucléiques sélectionnée parmi les séquences ayant le numéro d'identification 2, 5 à 10, 14, ou leur complément, laquelle séquence d'acides nucléiques code pour une région de la protéine liant l'ADN de la séquence répondant à l'insuline 1 (IRSBP-1) ; et la mise en contact de l'oligonucléotide avec un acide nucléique contenant la séquence comprise dans les séquences ayant le numéro d'identification 2, 5 à 10, 14, ou leur complément dans des conditions permettant l'hybridation de l'oligonucléotide avec l'acide nucléique.

20. Procédé pour identifier un homologue d'acide nucléique, comprenant les étapes suivantes :
a) obtention d'une molécule sonde de séquence d'acides nucléiques contenant une séquence de nucléotides sélectionnée parmi les séquences ayant le numéro d'identification 2, 5 à 10, 14, ou leur complément, et codant pour une région de la protéine liant l'ADN de la séquence répondant à l'insuline 1 (IRSBP-1) ;
b) accès à une base de données de séquences contenant une séquence de nucléotides cible ;
c) alignement de ladite molécule sonde de séquence d'acides nucléiques avec ladite séquence de nucléotides cible ;
d) détermination d'un pourcentage d'homologie entre une séquence de nucléotides de ladite molécule sonde de séquence d'acides nucléiques et ladite séquence de nucléotides cible ; et
e) identification de ladite séquence de nucléotides cible dans ladite base de données de séquences, le pourcentage d'homologie entre la séquence de nucléotides de ladite molécule sonde de séquence d'acides nucléiques et ladite séquence de nucléotides cible étant de 80 à 100 %.

21. Procédé selon la revendication 20, dans lequel le pourcentage d'homologie entre la séquence de nucléotides de ladite molécule sonde de séquence d'acides nucléiques et ladite séquence de nucléotides cible est de 90 à 100 %, et dans lequel la molécule sonde de séquence d'acides nucléides comprend une séquence de nucléotides sélectionnée parmi les séquences ayant le numéro d'identification 5 ou 7.

22. Anticorps qui se lie spécifiquement à un polypeptide codé par les acides nucléiques définies selon l'une quelconque des revendications 1 à 7.

23. Anticorps selon la revendication 22, dans lequel le polypeptide codé par les acides nucléiques est la séquence ayant le numéro d'identification 14.

24. Procédé pour déterminer si un échantillon biologique contient une protéine IRSBP-1, comprenant les étapes suivantes :
a) prélèvement d'un échantillon biologique d'un sujet humain ou d'un animal ;
b) extraction de l'échantillon biologique avec l'anticorps selon les revendications 22 ou 23 ; et
c) détermination du fait que l'anticorps se lie ou non spécifiquement à l'échantillon, la liaison indiquant que l'échantillon biologique contient de l'IRSBP-1.

25. Procédé selon la revendication 24, dans lequel l'échantillon biologique contient des cellules et la liaison de l'anticorps indique que les cellules contiennent de l'IRSBP-1.

26. Procédé pour la détection d'une maladie, comprenant les étapes de présentation d'un échantillon biologique obtenu d'un sujet humain ou d'un animal et de détection sélective d'un acide nucléique codant pour une région de la protéine liant l'ADN de la séquence répondant à l'insuline 1 (IRSBP-1) selon l'une quelconque des revendications 1 à 7, dans lequel la détection d'un acide nucléique selon l'une quelconque des revendications 1 à 7 indique une maladie.

27. Protéine liant l'ADN de la séquence répondant à l'insuline 1 (IRSBP-1) pouvant se lier à des régions d'acide nucléique associées à des gènes réagissant lorsque les cellules sont exposées à l'insuline ou à des facteurs similaires de l'insuline et ayant une séquence identique à 75-100 % aux séquences ayant le numéro d'identification 11 ou 12.

28. Protéine liant l'ADN de la séquence répondant à l'insuline 1 (IRSBP-1) selon la revendication 27, possédant une séquence identique à 90-100 % aux séquences ayant le numéro d'identification 11 ou 12.
